# EUROPEAN PATENT APPLICATION

(11) **EP 4 620 974 A1**
(43) Date of publication of application: **24.09.2025**
(21) Application number: 22965443.9
(22) Date of filing: 15.11.2022
(51) Int. Cl.: C07K 16/28, C07K 19/00, C12N 15/62, C12N 5/10, C12N 15/867, A61K 39/00, A61P 35/00

(54) **ANTI-AFP/HLA02 TCR-LIKE ANTIBODY AND USE THEREOF**

(71) Applicant: Oricell Therapeutics Co., Ltd., Shanghai 201203 (CN)
(72) Inventor: WANG, Huajing, Shanghai 201203 (CN); CHENG, Chao, Shanghai 201203 (CN); HE, Xiaowen, Shanghai 201203 (CN); XIE, Ermin, Shanghai 201203 (CN); LI, Xiaopei, Shanghai 201203 (CN); CHEN, Xiaorui, Shanghai 201203 (CN)
(74) Representative: Weickmann & Weickmann PartmbB
(86) International application number: PCT/CN2022/131945
(87) International publication number: WO 2024/103251

(57) **Abstract**

The present application relates to an anti-AFP/HLA02TCR-like antibody and a use thereof. The antibody can specifically bind to a human AFP₁₅₈₋₁₆₆/HLA-A02*01* complex with a *K_{D}* value of about 3.1E-09M or less. The present application further relates to a chimeric antigen receptor and an immunoconjugate comprising the antibody, and a cell comprising the chimeric antigen receptor.

## Description

### Field of the Invention

The present application relates to the field of biotechnology, and particularly to an anti-AFP/HLA02 TCR-like antibody and its use.

### Background of the Invention

Alpha-fetoprotein (AFP), a glycoprotein, can be elevated in the serum of approximately 80% of adult patients with liver cancer. The positive rate of AFP in germ cell tumors is 50%. Elevated AFP levels can also be observed to varying degrees in patients with pancreatic cancer or lung cancer, and liver cirrhosis. Therefore, AFP is expected to be a target for treating a variety of solid tumors.

Intracellular tumor-specific antigens can be processed into peptides and presented to the surface of tumor cells via class I major histocompatibility complexes (MHC), and TCR-like antibodies can bind to polypeptide/MHC complexes, thereby inducing tumor cell death. TCR-like antibodies can be converted into CAR structures that mediate specific tumor lysis via T cells.

Currently, CART technology has achieved great success in hematologic tumors, but there have been no significant breakthroughs in its application yield of treating solid tumors. Problems faced by CART therapy include: 1) unlike leukemia, which is distributed throughout the body, solid tumors require CART cells to reach the lesions of solid tumors and infiltrate into the tumor to exert their effects, thus creating a spatial barrier to CART therapy by the tissue structure of solid tumors; 2) the suppressive effect of the immune microenvironment inside the tumor can lead to the failure of CART cells to function properly, and will produce T-cell depletion; 3) the hypoxia and nutrient deficiencies in the internal microenvironment of solid tumors are also not conducive to the massive proliferation of CART cells and the production of targeted cytotoxicity.

Therefore, it is urgent to develop a new antibody and CAR structure to treat solid tumors.

### Summary of the Invention

The present application provides an isolated antigen-binding protein, having one or more of the following properties: 1) specifically binding to a human AFP₁₅₈₋₁₆₆/HLA-A02*01* complex with a *K_{D}* value of about 3.1E-09M or less; and 2) capable of binding to a mouse AFP₁₅₈//HLA-A02*01* complex.

In one aspect, the present application provides an isolated antigen-binding protein, the antigen-binding protein includes an HCDR3, and the HCDR3 includes an amino acid sequence as set forth in any one of SEQ ID NO: 19, SEQ ID NO: 25, SEQ ID NO: 30, SEQ ID NO: 35, SEQ ID NO: 46, SEQ ID NO: 52, and SEQ ID NO: 56.

In some embodiments, the antigen-binding protein includes an HCDR2, and the HCDR2 includes an amino acid sequence as set forth in any one of SEQ ID NO: 18, SEQ ID NO: 24, SEQ ID NO: 34, SEQ ID NO: 45, SEQ ID NO: 51, and SEQ ID NO: 55.

In some embodiments, the antigen-binding protein includes an HCDR1, and the HCDR1 includes an amino acid sequence as set forth in any one of SEQ ID NO: 17, SEQ ID NO: 23, SEQ ID NO: 29, SEQ ID NO: 37, SEQ ID NO: 39, SEQ ID NO: 44, and SEQ ID NO: 50.

In some embodiments, the antigen-binding protein includes an HCDR1, an HCDR2, and an HCDR3 in a heavy chain variable region as set forth in any one of SEQ ID NO: 72 to SEQ ID NO: 84.

In some embodiments, the antigen-binding protein includes an HCDR1, an HCDR2, and an HCDR3, the HCDR3 includes an amino acid sequence as set forth in any one of SEQ ID NO: 19, SEQ ID NO: 25, SEQ ID NO: 30, SEQ ID NO: 35, SEQ ID NO: 46, SEQ ID NO: 52, and SEQ ID NO: 56; the HCDR2 includes an amino acid sequence as set forth in any one of SEQ ID NO: 18, SEQ ID NO: 24, SEQ ID NO: 34, SEQ ID NO: 45, SEQ ID NO: 51, and SEQ ID NO: 55; and the HCDR1 includes an amino acid sequence as set forth in any one of SEQ ID NO: 17, SEQ ID NO: 23, SEQ ID NO: 29, SEQ ID NO: 37, SEQ ID NO: 39, SEQ ID NO: 44, and SEQ ID NO: 50.

In some embodiments, the HCDR1, HCDR2, and HCDR3 include any one group of amino acid sequences selected from:
a) HCDR1: SEQ ID NO: 17, HCDR2: SEQ ID NO: 18, and HCDR3: SEQ ID NO: 19;
b) HCDR1: SEQ ID NO: 23, HCDR2: SEQ ID NO: 24, and HCDR3: SEQ ID NO: 25;
c) HCDR1: SEQ ID NO: 29, HCDR2: SEQ ID NO: 24, and HCDR3: SEQ ID NO: 30;
d) HCDR1: SEQ ID NO: 23, HCDR2: SEQ ID NO: 34, and HCDR3: SEQ ID NO: 35;
e) HCDR1: SEQ ID NO: 37, HCDR2: SEQ ID NO: 24, and HCDR3: SEQ ID NO: 25;
f) HCDR1: SEQ ID NO: 39, HCDR2: SEQ ID NO: 24, and HCDR3: SEQ ID NO: 25;
g) HCDR1: SEQ ID NO: 23, HCDR2: SEQ ID NO: 24, and HCDR3: SEQ ID NO: 30;
h) HCDR1: SEQ ID NO: 44, HCDR2: SEQ ID NO: 45, and HCDR3: SEQ ID NO: 46;
i) HCDR1: SEQ ID NO: 50, HCDR2: SEQ ID NO: 51, and HCDR3: SEQ ID NO: 52;
j) HCDR1: SEQ ID NO: 23, HCDR2: SEQ ID NO: 55, and HCDR3: SEQ ID NO: 56;
   and
k) HCDR1: SEQ ID NO: 37, HCDR2: SEQ ID NO: 24, and HCDR3: SEQ ID NO: 30.

In some embodiments, the antigen-binding protein includes an H-FR1, the C-terminal of the H-FR1 is linked to the N-terminal of the HCDR1 directly or indirectly, and the H-FR1 includes an amino acid sequence as set forth in any one of SEQ ID NO: 58 to SEQ ID NO: 65.

In some embodiments, the antigen-binding protein includes an H-FR2, the H-FR2 is located between the HCDR1 and the HCDR2, and the H-FR2 includes an amino acid sequence as set forth in SEQ ID NO: 66.

In some embodiments, the antigen-binding protein includes an H-FR3, the H-FR3 is located between the HCDR2 and the HCDR3, and the H-FR3 includes an amino acid sequence as set forth in any one of SEQ ID NO: 67 to SEQ ID NO: 69.

In some embodiments, the antigen-binding protein includes an H-FR4, the N-terminal of the H-FR4 is linked to the C-terminal of the HCDR3 directly or indirectly, and the H-FR4 includes an amino acid sequence as set forth in any one of SEQ ID NO: 70 to SEQ ID NO: 71.

In some embodiments, the antigen-binding protein includes an H-FR1, an H-FR2, an H-FR3, and an H-FR4, the H-FR1 includes an amino acid sequence as set forth in any one of SEQ ID NO: 58 to SEQ ID NO: 65; the H-FR2 includes an amino acid sequence as set forth in SEQ ID NO: 66; the H-FR3 includes an amino acid sequence as set forth in any one of SEQ ID NO: 67 to SEQ ID NO: 69; and the H-FR4 includes an amino acid sequence as set forth in any one of SEQ ID NO: 70 to SEQ ID NO: 71.

In some embodiments, the H-FR1, H-FR2, H-FR3, and H-FR4 in the antigen-binding protein include any one group of amino acid sequences selected from:
a) H-FR1: SEQ ID NO: 58, H-FR2: SEQ ID NO: 66, H-FR3: SEQ ID NO: 67, and H-FR4: SEQ ID NO: 70;
b) H-FR1: SEQ ID NO: 59, H-FR2: SEQ ID NO: 66, H-FR3: SEQ ID NO: 67, and H-FR4: SEQ ID NO: 70;
c) H-FR1: SEQ ID NO: 60, H-FR2: SEQ ID NO: 66, H-FR3: SEQ ID NO: 67, and H-FR4: SEQ ID NO: 70;
d) H-FR1: SEQ ID NO: 61, H-FR2: SEQ ID NO: 66, H-FR3: SEQ ID NO: 67, and H-FR4: SEQ ID NO: 70;
e) H-FR1: SEQ ID NO: 62, H-FR2: SEQ ID NO: 66, H-FR3: SEQ ID NO: 67, and H-FR4: SEQ ID NO: 70;
f) H-FR1: SEQ ID NO: 63, H-FR2: SEQ ID NO: 66, H-FR3: SEQ ID NO: 67, and H-FR4: SEQ ID NO: 70;
g) H-FR1: SEQ ID NO: 64, H-FR2: SEQ ID NO: 66, H-FR3: SEQ ID NO: 67, and H-FR4: SEQ ID NO: 70;
h) H-FR1: SEQ ID NO: 64, H-FR2: SEQ ID NO: 66, H-FR3: SEQ ID NO: 68, and H-FR4: SEQ ID NO: 71; and
i) H-FR1: SEQ ID NO: 65, H-FR2: SEQ ID NO: 66, H-FR3: SEQ ID NO: 69, and H-FR4: SEQ ID NO: 70.

In some embodiments, the antigen-binding protein includes a heavy chain variable region VH, and the VH includes an amino acid sequence as set forth in any one of SEQ ID NO: 72 to SEQ ID NO: 84.

In some embodiments, the antigen-binding protein includes an antibody or an antigen binding fragment thereof.

In some embodiments, the antigen binding fragment is selected from a group consisting of: Fab, Fab', F(ab)2, an Fv fragment, F(ab')2, scFv, di-scFv, VHH, and dAb.

In some embodiments, the antigen-binding protein includes a VHH or an antigen binding fragment thereof.

In some embodiments, the antibody is selected from a group consisting of: a monoclonal antibody, a humanized antibody, a chimeric antibody, a bispecific antibody, a multispecific antibody, and a fully human antibody.

In some embodiments, the antigen-binding protein includes an amino acid sequence as set forth in any one of SEQ ID NO: 72 to SEQ ID NO: 84.

In another aspect, the present application provides a chimeric antigen receptor including a targeting moiety, the targeting moiety includes an HCDR3, and the HCDR3 includes an amino acid sequence as set forth in any one of SEQ ID NO: 19, SEQ ID NO: 25, SEQ ID NO: 30, SEQ ID NO: 35, SEQ ID NO: 46, SEQ ID NO: 52, and SEQ ID NO: 56.

In some embodiments, the targeting moiety in the chimeric antigen receptor includes an HCDR2, and the HCDR2 includes an amino acid sequence as set forth in any one of SEQ ID NO: 18, SEQ ID NO: 24, SEQ ID NO: 34, SEQ ID NO: 45, SEQ ID NO: 51, and SEQ ID NO: 55.

In some embodiments, the targeting moiety in the chimeric antigen receptor includes an HCDR1, and the HCDR1 includes an amino acid sequence as set forth in any one of SEQ ID NO: 17, SEQ ID NO: 23, SEQ ID NO: 29, SEQ ID NO: 37, SEQ ID NO: 39, SEQ ID NO: 44, and SEQ ID NO: 50.

In some embodiments, the targeting moiety in the chimeric antigen receptor includes an HCDR1, an HCDR2, and an HCDR3 in a heavy chain variable region as set forth in any one of SEQ ID NO: 72 to SEQ ID NO: 84.

In some embodiments, the targeting moiety in the chimeric antigen receptor includes an HCDR1, an HCDR2, and an HCDR3, the HCDR3 includes an amino acid sequence as set forth in any one of SEQ ID NO: 19, SEQ ID NO: 25, SEQ ID NO: 30, SEQ ID NO: 35, SEQ ID NO: 46, SEQ ID NO: 52, and SEQ ID NO: 56; the HCDR2 includes an amino acid sequence as set forth in any one of SEQ ID NO: 18, SEQ ID NO: 24, SEQ ID NO: 34, SEQ ID NO: 45, SEQ ID NO: 51, and SEQ ID NO: 55; and the HCDR1 includes an amino acid sequence as set forth in any one of SEQ ID NO: 17, SEQ ID NO: 23, SEQ ID NO: 29, SEQ ID NO: 37, SEQ ID NO: 39, SEQ ID NO: 44, and SEQ ID NO: 50.

In some embodiments, the HCDR1, HCDR2, and HCDR3 in the chimeric antigen receptor include any one group of amino acid sequences selected from:
a) HCDR1: SEQ ID NO: 17, HCDR2: SEQ ID NO: 18, and HCDR3: SEQ ID NO: 19;
b) HCDR1: SEQ ID NO: 23, HCDR2: SEQ ID NO: 24, and HCDR3: SEQ ID NO: 25;
c) HCDR1: SEQ ID NO: 29, HCDR2: SEQ ID NO: 24, and HCDR3: SEQ ID NO: 30;
d) HCDR1: SEQ ID NO: 23, HCDR2: SEQ ID NO: 34, and HCDR3: SEQ ID NO: 35;
e) HCDR1: SEQ ID NO: 37, HCDR2: SEQ ID NO: 24, and HCDR3: SEQ ID NO: 25;
f) HCDR1: SEQ ID NO: 39, HCDR2: SEQ ID NO: 24, and HCDR3: SEQ ID NO: 25;
g) HCDR1: SEQ ID NO: 23, HCDR2: SEQ ID NO: 24, and HCDR3: SEQ ID NO: 30;
h) HCDR1: SEQ ID NO: 44, HCDR2: SEQ ID NO: 45, and HCDR3: SEQ ID NO: 46;
i) HCDR1: SEQ ID NO: 50, HCDR2: SEQ ID NO: 51, and HCDR3: SEQ ID NO: 52;
j) HCDR1: SEQ ID NO: 23, HCDR2: SEQ ID NO: 55, and HCDR3: SEQ ID NO: 56;
   and
k) HCDR1: SEQ ID NO: 37, HCDR2: SEQ ID NO: 24, and HCDR3: SEQ ID NO: 30.

In some embodiments, the targeting moiety in the chimeric antigen receptor includes an H-FR1, the C-terminal of the H-FR1 is linked to the N-terminal of the HCDR1 directly or indirectly, and the H-FR1 includes an amino acid sequence as set forth in any one of SEQ ID NO: 58 to SEQ ID NO: 65.

In some embodiments, the targeting moiety in the chimeric antigen receptor includes an H-FR2, the H-FR2 is located between the HCDR1 and the HCDR2, and the H-FR2 includes an amino acid sequence as set forth in SEQ ID NO: 66.

In some embodiments, the targeting moiety in the chimeric antigen receptor includes an H-FR3, the H-FR3 is located between the HCDR2 and the HCDR3, and the H-FR3 includes an amino acid sequence as set forth in any one of SEQ ID NO: 67 to SEQ ID NO: 69.

In some embodiments, the targeting moiety in the chimeric antigen receptor includes an H-FR4, the N-terminal of the H-FR4 is linked to the C-terminal of the HCDR3 directly or indirectly, and the H-FR4 includes an amino acid sequence as set forth in any one of SEQ ID NO: 70 to SEQ ID NO: 71.

In some embodiments, the targeting moiety in the chimeric antigen receptor includes an H-FR1, an H-FR2, an H-FR3, and an H-FR4, the H-FR1 includes an amino acid sequence as set forth in any one of SEQ ID NO: 58 to SEQ ID NO: 65; the H-FR2 includes an amino acid sequence as set forth in SEQ ID NO: 66; the H-FR3 includes an amino acid sequence as set forth in any one of SEQ ID NO: 67 to SEQ ID NO: 69; and the H-FR4 includes an amino acid sequence as set forth in any one of SEQ ID NO: 70 to SEQ ID NO: 71.

In some embodiments, the H-FR1, H-FR2, H-FR3, and H-FR4 in the chimeric antigen receptor include any one group of amino acid sequences selected from:
a) H-FR1: SEQ ID NO: 58, H-FR2: SEQ ID NO: 66, H-FR3: SEQ ID NO: 67, and H-FR4: SEQ ID NO: 70;
b) H-FR1: SEQ ID NO: 59, H-FR2: SEQ ID NO: 66, H-FR3: SEQ ID NO: 67, and H-FR4: SEQ ID NO: 70;
c) H-FR1: SEQ ID NO: 60, H-FR2: SEQ ID NO: 66, H-FR3: SEQ ID NO: 67, and H-FR4: SEQ ID NO: 70;
d) H-FR1: SEQ ID NO: 61, H-FR2: SEQ ID NO: 66, H-FR3: SEQ ID NO: 67, and H-FR4: SEQ ID NO: 70;
e) H-FR1: SEQ ID NO: 62, H-FR2: SEQ ID NO: 66, H-FR3: SEQ ID NO: 67, and H-FR4: SEQ ID NO: 70;
f) H-FR1: SEQ ID NO: 63, H-FR2: SEQ ID NO: 66, H-FR3: SEQ ID NO: 67, and H-FR4: SEQ ID NO: 70;
g) H-FR1: SEQ ID NO: 64, H-FR2: SEQ ID NO: 66, H-FR3: SEQ ID NO: 67, and H-FR4: SEQ ID NO: 70;
h) H-FR1: SEQ ID NO: 64, H-FR2: SEQ ID NO: 66, H-FR3: SEQ ID NO: 68, and H-FR4: SEQ ID NO: 71; and
i) H-FR1: SEQ ID NO: 65, H-FR2: SEQ ID NO: 66, H-FR3: SEQ ID NO: 69, and H-FR4: SEQ ID NO: 70.

In some embodiments, the targeting moiety in the chimeric antigen receptor includes an antibody or an antigen binding fragment.

In some embodiments, the antigen binding fragment in the chimeric antigen receptor is selected from a group consisting of: Fab, Fab', F(ab)2, an Fv fragment, F(ab')2, scFv, di-scFv, VHH and/or dAb.

In some embodiments, the targeting moiety in the chimeric antigen receptor includes a VHH.

In some embodiments, the VHH in the chimeric antigen receptor targets a human AFP₁₅₈₋₁₆₆/HLA-A02*01* complex.

In some embodiments, the targeting moiety in the chimeric antigen receptor includes an amino acid sequence as set forth in any one of SEQ ID NO: 72 to SEQ ID NO: 84.

In some embodiments, the chimeric antigen receptor includes a hinge region.

In some embodiments, the hinge region in the chimeric antigen receptor includes a hinge region derived from a protein selected from a group consisting of: IgG4, IgG1, and CD8.

In some embodiments, the hinge region in the chimeric antigen receptor includes an amino acid sequence as set forth in SEQ ID NO: 147.

In some embodiments, the chimeric antigen receptor includes a transmembrane domain.

In some embodiments, the transmembrane domain in the chimeric antigen receptor includes a transmembrane domain derived from a protein selected from a group consisting of: CD8, CD28, CD24, 4-1BB, CD4, CD27, CD7, PD-1, TRAC, TRBC, CD3ε, CD5, ICOS, OX40, NKG2D, 2B4, CD244, FcεRIγ, BTLA, CD30, GITR, HVEM, DAP10, CD2, NKG2C, LIGHT, DAP12, CD40L, TIM1, CD226, DR3, CD45, CD80, CD86, CD9, CD16, CD22, CD33, CD37, CD64, CD134, CD154, and SLAM, or a combination thereof.

In some embodiments, the transmembrane domain in the chimeric antigen receptor includes a transmembrane domain derived from CD8.

In some embodiments, the transmembrane domain in the chimeric antigen receptor includes an amino acid sequence as set forth in SEQ ID NO: 149.

In some embodiments, the N-terminal of the transmembrane domain in the chimeric antigen receptor is linked to the C-terminal of the hinge region.

In some embodiments, the chimeric antigen receptor includes a costimulatory signaling domain.

In some embodiments, the costimulatory signaling domain in the chimeric antigen receptor includes a costimulatory signaling domain derived from a protein selected from a group consisting of: CD28, 4-1BB, CD27, CD2, CD7, CD8, OX40, CD226, DR3, SLAM, CDS, ICAM-1, NKG2D, NKG2C, B7-H3, 2B4, FcεRIγ, BTLA, GITR, HVEM, DAP10, DAP12, CD30, CD40, CD40L, TIM1, PD-1, LFA-1, LIGHT, JAML, CD244, CD100, ICOS, a ligand of CD83, CD40, and MyD88, or a combination thereof.

In some embodiments, the costimulatory signaling domain in the chimeric antigen receptor includes a costimulatory signaling domain derived from 4-1BB.

In some embodiments, the costimulatory signaling domain in the chimeric antigen receptor includes a costimulatory signaling domain derived from CD28.

In some embodiments, the costimulatory signaling domain in the chimeric antigen receptor includes an amino acid sequence as set forth in SEQ ID NO: 132 or SEQ ID NO: 130.

In some embodiments, the N-terminal of the costimulatory signaling domain in the chimeric antigen receptor is linked to the C-terminal of the transmembrane domian.

In some embodiments, the chimeric antigen receptor includes an intracellular signaling domain.

In some embodiments, the intracellular signaling domain in the chimeric antigen receptor includes an intracellular signaling domain derived from a protein selected from a group consisting of: CD3ζ, CD3δ, CD3γ, CD3ε, CD79a, CD79b, FceRIγ, FceRIβ, FcγRIIa, bovine leukemia virus gp30, Epstein-Barr virus (EBV) LMP2A, simian immunodeficiency virus PBj14 Nef, Kaposi's sarcoma-associated herpes virus (HSKV), DAP10, and DAP-12, or a combination thereof.

In some embodiments, the intracellular signaling domain in the chimeric antigen receptor includes an intracellular signaling domain derived from CD3ζ.

In some embodiments, the intracellular signaling domain in the chimeric antigen receptor includes an amino acid sequence as set forth in SEQ ID NO: 134.

In some embodiments, the N-terminal of the intracellular signaling domain in the chimeric antigen receptor is linked to the C-terminal of the costimulatory signaling domain.

In some embodiments, the chimeric antigen receptor includes an amino acid sequence as set forth in any one of SEQ ID NO: 112 to SEQ ID NO: 124.

In another aspect, the present application provides a polypeptide, which includes the isolated antigen-binding protein and/or the chimeric antigen receptor.

In another aspect, the present application provides an immunoconjugate, which includes the isolated antigen-binding protein.

In another aspect, the present application provides one or more isolated nucleic acid molecules, which encode(s) the isolated antigen-binding protein, the chimeric antigen receptor or the polypeptide.

In another aspect, the present application provides one or more vectors, which include(s) the isolated nucleic acid molecule.

In another aspect, the present application provides one or more modified cells, which include(s) the isolated antigen-binding protein, the chimeric antigen receptor, the polypeptide, the immunoconjugate, the isolated nucleic acid molecule and/or the vector.

In some embodiments, the modification includes upregulating the expression level of the low-density lipoprotein receptor-related protein or the fragment thereof in the cell.

In some embodiments, the low-density lipoprotein receptor-related protein or the fragment thereof includes one or more selected from a group consisting of: low-density lipoprotein receptor-related proteins 1-12, and functional fragments thereof.

In some embodiments, the low-density lipoprotein receptor-related protein or the fragment thereof is derived from human.

In some embodiments, the functional fragment includes a fragment or a truncated body of the low-density lipoprotein receptor-related protein having the activity of the low-density lipoprotein receptor-related protein.

In some embodiments, the low-density lipoprotein receptor-related protein includes a low-density lipoprotein receptor-related protein 6 and a truncated body thereof, and/or a low-density lipoprotein receptor-related protein 5 and a truncated body thereof.

In some embodiments, the truncated body of the low-density lipoprotein receptor-related protein 6 includes an intracellular region of the low-density lipoprotein receptor-related protein 6; and/or, the truncated body of the low-density lipoprotein receptor-related protein 5 includes an intracellular region of the low-density lipoprotein receptor-related protein 5.

In some embodiments, the truncated body of the low-density lipoprotein receptor-related protein 6 includes a transmembrane region of the low-density lipoprotein receptor-related protein 6 and an LDLR region of the low-density lipoprotein receptor-related protein 6; and/or, the truncated body of the low-density lipoprotein receptor-related protein 5 includes a transmembrane region of the low-density lipoprotein receptor-related protein 5 and an LDLR region of the low-density lipoprotein receptor-related protein 5.

In some embodiments, the low-density lipoprotein receptor-related protein or the fragment thereof includes an amino acid sequence as set forth in any one of SEQ ID NO: 138, SEQ ID NO: 140, SEQ ID NO: 142, and SEQ ID NO: 144.

In some embodiments, the modification includes introducing into the modified cell a vector that upregulates the expression level of the low-density lipoprotein receptor-related protein or the fragment thereof.

In some embodiments, the modified cell includes an immune cell.

In some embodiments, the immune cell is selected from a group consisting of: T cell, B cell, natural killer cell (NK cell), macrophage, NKT cell, monocyte, dendritic cell, granulocyte, lymphocyte, leukocyte and/or peripheral blood mononuclear cell.

In some embodiments, the modified cell includes the T cell.

In another aspect, the present application provides a method for preparing the isolated antigen-binding protein, the chimeric antigen receptor, and/or the polypeptide, which includes culturing the modified cell under a condition enabling the expression of the isolated antigen-binding protein and/or the polypeptide.

In another aspect, the present application provides one or more pharmaceutical composition(s), which include(s) the isolated antigen-binding protein, the chimeric antigen receptor, the polypeptide, the immunoconjugate, the isolated nucleic acid molecule, the vector, the modified cell, and/or a pharmaceutically acceptable adjuvant and/or excipient.

In another aspect, the present application provides a method for detecting AFP protein, which includes:
administering the isolated antigen-binding protein, the polypeptide or the immunoconjugate.

In another aspect, the present application provides a kit for detecting AFP protein, which includes the isolated antigen-binding protein, the polypeptide or the immunoconjugate.

In another aspect, the present application provides a use of the isolated antigen-binding protein, the polypeptide or the immunoconjugate in the preparation of a kit for detecting the presence and/or content of AFP protein.

In another aspect, the present application provides a use of the isolated antigen-binding protein, the chimeric antigen receptor, the polypeptide, the immunoconjugate, the isolated nucleic acid molecule, the vector, and/or the modified cell in the preparation of a drug for preventing and/or treating a tumor.

In some embodiments, the tumor in the use includes a solid tumor.

In some embodiments, the tumor in the use includes a non-solid tumor.

In some embodiments, the tumor in the use includes an AFP expression-associated tumor.

In some embodiments, the tumor in the use includes liver cancer.

In another aspect, the present application provides the isolated antigen-binding protein, the chimeric antigen receptor, the polypeptide, the immunoconjugate, the isolated nucleic acid molecule, the vector, and/or the modified cell, which are used for preventing and/or treating a tumor.

In some embodiments, the tumor in the use includes a solid tumor.

In some embodiments, the tumor in the use includes a non-solid tumor.

In some embodiments, the tumor in the use includes an AFP expression-associated tumor.

In some embodiments, the tumor in the use includes liver cancer.

In another aspect, the present application provides a method for preventing and/or treating a disease or disorder, which includes administering to a subject in need thereof an effective amount of the isolated antigen-binding protein, the chimeric antigen receptor, the polypeptide, the immunoconjugate, the isolated nucleic acid molecule, the vector, and/or the modified cell.

In some embodiments, the tumor in the method includes a solid tumor.

In some embodiments, the tumor in the method includes a non-solid tumor.

In some embodiments, the tumor in the method includes an AFP expression-associated tumor.

In some embodiments, the tumor in the method includes liver cancer.

Those skilled in the art can easily perceive other aspects and advantages of the present application from the detailed description below. In the following detailed description, only exemplary embodiments of the present application are shown and described. As those skilled in the art will recognize, the content of the present application enables those skilled in the art to make changes to the disclosed specific embodiments without departing from the spirit and scope of the invention involved in the present application. Correspondingly, the drawings and descriptions in the specification of the present application are merely exemplary, rather than restrictive.

### Brief Description of the Drawings

The specific features of the invention involved in the present application are shown in the appended claims. The characteristics and advantages of the invention involved in the present application can be better understood by referring to the exemplary embodiments and the accompanying drawings described in detail below. A brief description of the drawings is as follows:
FIG. 1 shows the identification results of ELISA after 5 rounds of panning as described in the present application.
FIG. 2 shows the sequence repeatability of single clones picked after panning as described in the present application.
FIG. 3 shows the PCR amplification results of the candidate VHH antibody nucleotide sequence as described in the present application.
FIG. 4 shows the SDS-PAGE results of the expression of purified VHH antibody as described in the present application.
FIG. 5 shows the eukaryotic expression vector of the candidate VHH-Fc antibody as described in the present application (take 1C11 for example).
FIG. 6 shows the affinity of the candidate VHH-Fc antibody as described in the present application.
FIG. 7A shows the flow cytometry results of the candidate VHH-Fc antibody with human TERT540/T2 cells as described in the present application.
FIG. 7B shows the flow cytometry results of the candidate VHH-Fc antibody with polypeptide/T2 cells as described in the present application.
FIG. 7C shows the flow cytometry results of the candidate VHH-Fc antibody with mouse AFP158/T2 cells as described in the present application.
FIG. 8 shows the core plasmid vector map as described in the present application.
FIG. 9 shows the CAR core plasmid structure diagram as described in the present application.
FIG. 10A shows the PCR amplification results of the human AFP158-166 nucleotide sequence as described in the present application.
FIG. 10B shows the enzyme digestion results of the vector as described in the present application.
FIG. 11 shows the screening results of single clones detected by flow cytometry as described in the present application.
FIG. 12 shows the positive rate results of HEPG2-MiniG, and SK-HEP-1-MiniG detected by flow cytometry screening as described in the present application.
FIGs. 13A-13B show the results of repeated stimulation and expansion experiments of CAR-T cells as described in the present application.
FIG. 13C and FIG. 13F show the *in vitro* cell killing experiment results of CAR-T cells at an effector-target ratio of 1:1 as described in the present application.
FIG. 13D and FIG. 13G show the IFN-γ cytokine secretion results at an effector-target ratio of 1:1 as described in the present application.
FIG. 13E and FIG. 13H shows the IL-2 cytokine secretion results at an effector-target ratio of 1:1 as described in the present application.
FIG. 14 shows the flow chart of animal experiment as described in the present application.
FIG. 15A shows the tumor growth curve as described in the present application.
FIG. 15B shows the animal body weight curve as described in the present application.

### Detailed Description of the Embodiments

The implementation of the present application will be illustrated in the following specific examples, and other advantages and effects of the present application will be easily known by those skilled in the art from the content disclosed in the specification.

### Definition of Terms

In the present application, the term "isolated antigen-binding protein" generally refers to a polypeptide polymer capable of specifically recognizing and/or neutralizing a particular antigen. For example, the isolated antigen-binding protein may include a part of a heavy chain. For example, the isolated antigen-binding protein may include a heavy chain variable region. The term "isolated antigen-binding protein" may include a single-domain antibody. For example, the isolated antigen-binding protein may include, but is not limited to, a human single-domain antibody.

In the present application, the term "single-domain antibody" or "sdAb" or "VHH" generally refers to a class of antibodies that lack the antibody light chain but have only the heavy chain variable region. In some cases, the single-domain antibody may be derived from Camelus bactrianus, Camelus dromedarius, alpaca, llama, nurse shark, starspotted smooth-hound or ray (e.g., see Kang Xiaozhen et al, Chinese Journal of Biotechnology, 2018, 34(12): 1974-1984). For example, the single-domain antibody may be derived from alpaca. The single-domain antibody may be composed of a heavy chain variable region (VH). The term "heavy chain variable region" generally refers to the amino-terminal domain of the heavy chain of an antigen binding fragment. The heavy chain variable region can be further differentiated into hypervariable regions called complementary determining regions (CDRs), which are interspersed with more conserved regions called framework regions (FRs). Each heavy chain variable region can be composed of three CDRs and four FRs, arranged in the following order from the amino terminus to the carboxyl terminus: FR1, CDR1, FR2, CDR2, FR3, CDR3, and FR4. The heavy chain variable region contains a binding domain interacting with the antigen (e.g., AFP).

In the present application, the term "TCR-like antibody" generally refers to an antibody capable of recognizing the peptide/MHC complex on the surface of tumor cells. These TCR-like antibodies share functional identity with TCR in terms of target recognition. Technically, TCR-like antibodies can be generated by conventional hybridoma techniques or by *in vitro* antibody library techniques known to those skilled in the art. In the present application, the "TCR-like antibody" can recognize AFP/HLA02. For example, the TCR-like antibody can recognize a human AFP₁₅₈₋₁₆₆/HLA-A02*01* complex.

In the present application, the term "transmembrane domain" generally refers to a sequence in a cell surface protein that spans the cell membrane and may contain a hydrophobic alpha helix. The transmembrane domain may be linked to the intracellular signaling domain to play a role in transmitting signals. In the present application, the transmembrane domain may be derived from any type I, type II or type III transmembrane protein. In the present application, the transmembrane domain may include a transmembrane domain derived from a protein selected from a group consisting of: CD8, CD28, CD24, 4-1BB, CD4, CD27, CD7, PD-1, TRAC, TRBC, CD3ε, CD5, ICOS, OX40, NKG2D, 2B4, CD244, FcεRIγ, BTLA, CD30, GITR, HVEM, DAP10, CD2, NKG2C, LIGHT, DAP12, CD40L, TIM1, CD226, DR3, CD45, CD80, CD86, CD9, CD16, CD22, CD33, CD37, CD64, CD134, CD154, and SLAM, or a combination thereof. For example, the transmembrane domain may include a transmembrane domain derived from the CD8.

In the present application, the term "chimeric antigen receptor (CAR)" generally refers to a fusion protein containing a targeting moiety capable of binding to the antigen and at least one intracellular domain. CAR is the core component of a chimeric antigen receptor T cell (CAR-T), which may include a targeting moiety (e.g., targeting the tumor-specific antigen and/or tumor-related antigen), a signal peptide, a transmembrane domain, a costimulatory signaling domain, and an intracellular signaling domain. In the present application, the CAR can be combined with the T cell receptor activation intracellular domain based on the specificity of the antibody for the antigen (e.g., AFP₁₅₈₋₁₆₆/HLA-A02*01* complex). T cells with CAR expressed by genetic modification can specifically recognize and eliminate malignant cells expressing target antigens. For description of CARs and CAR-T cells, see, for example, Sadelain M, Brentjens R, Rivi 'ere I. The basic principles of chimeric antigen receptor design. Cancer Discov. 2013; 3(4): 388-398; Turtle CJ, Hudecek M, Jensen MC, Riddell SR. Engineered T cells for anti-cancer therapy. Curr Opin Immunol. 2012; 24(5): 633-639; Dotti G, Gottschalk S, Savoldo B, Brenner MK. Design and development of therapies using chimeric antigen receptor-expressing T cells. Immunol Rev. 2014;257(1): 107-126; and WO2013154760, WO2016014789.

In the present application, the term "costimulatory signaling domain" generally refers to an intracellular domain capable of providing immunocostimulatory molecules, where the costimulatory molecules are cell surface molecules required for the effective response of lymphocytes to the antigen. In some cases, the costimulatory signaling domain may include a costimulatory signaling domain derived from a protein selected from a group consisting of: CD28, 4-1BB, CD27, CD2, CD7, CD8, OX40, CD226, DR3, SLAM, CDS, ICAM-1, NKG2D, NKG2C, B7-H3, 2B4, FcεRIγ, BTLA, GITR, HVEM, DAP10, DAP12, CD30, CD40, CD40L, TIM1, PD-1, LFA-1, LIGHT, JAML, CD244, CD100, ICOS, a ligand of CD83, CD40, and MyD88, or a combination thereof. For example, the costimulatory signaling domain may include a costimulatory signaling domain derived from 4-1BB.

In the present application, the term "intracellular signaling domain" generally refers to a domain capable of transducing signals that is located inside the cell. In the present application, the intracellular signaling domain can transmit signals into the cell. Generally, the intracellular signaling domain is any contiguous amino acid sequence used to direct protein targeting. In some cases, the intracellular signaling domain may include an intracellular signaling domain derived from a protein selected from a group consisting of: CD3ζ, CD3δ, CD3γ, CD3ε, CD79a, CD79b, FceRIγ, FceRIβ, FcγRIIa, bovine leukemia virus gp30, Epstein-Barr virus (EBV) LMP2A, simian immunodeficiency virus PBj14 Nef, Kaposi's sarcoma-associated herpes virus (HSKV), DAP10, DAP-12, and a domain containing at least one ITAM, or a combination thereof. For example, the intracellular signaling domain may include an intracellular signaling domain derived from CD3ζ.

In the present application, the term "antibody" generally refers to a polypeptide molecule capable of specifically recognizing and/or neutralizing a particular antigen. For example, the antibody may include an immunoglobulin composed of at least two heavy (H) chains and two light (L) chains, which are linked to each other through disulfide bonds, and include any molecules containing antigen-binding moieties thereof. The term "antibody" includes a monoclonal antibody, an antibody fragment or an antibody derivative, including but not limited to a human antibody (a fully human antibody), a humanized antibody, a chimeric antibody, a single-chain antibody (e.g., scFv), and an antibody fragment binding to an antigen (e.g., Fab, Fab', and a (Fab)2 fragment). The term "antibody" also includes any recombinant forms of the antibody, such as antibodies expressed in prokaryotic cells, unglycosylated antibodies, as well as any antibody fragments binding to an antigen and derivatives thereof. Each heavy chain may be composed of a heavy chain variable region (VH) and a heavy chain constant region. Each light chain may be composed of a light chain variable region (VL) and a light chain constant region. VH and VL regions can be further differentiated into hypervariable regions called complementarity determining regions (CDRs) interspersed with more conservative regions called framework regions (FRs). Each VH and VL can be composed of three CDRs and four FRs, arranged in the following order from the amino terminus to the carboxyl terminus: FR1, CDR1, FR2, CDR2, FR3, CDR3, and FR4. The variable regions of heavy chain and light chain contain binding domains interacting with antigens. The constant region of an antibody can mediate the binding of the immunoglobulin to the host tissue or factor, where the host tissue or factor includes a variety of cells of the immune system (e.g., effector cells) and the first component of the classical complement system (Clq).

In the present application, the term "antigen binding fragment" generally refers to one or more fragments of an antibody that function to specifically bind antigens. The antigen binding function of an antibody can be achieved through a full-length fragment of the antibody. The antigen binding function of the antibody can also be achieved by: a heavy chain including a fragment of Fv, ScFv, dsFv, Fab, Fab' or F (ab')2, or a light chain including a fragment of Fv, ScFv, dsFv, Fab, Fab' or F (ab')2. (1) a Fab fragment, a monovalent fragment generally composed of VL, VH, CL and CH domains; (2) a F(ab')2 fragment, a divalent fragment including two Fab fragments linked through a disulfide bond at the hinge region; (3) a Fd fragment composed of VH and CH domains; (4) a Fv fragment composed of the VL and VH domains of one arm of an antibody; (5) a dAb fragment composed of VH domains (Ward et al., (1989) Nature 341: 544-546); (6) an isolated complementary determining region (CDR), and (7) a combination of two or more isolated CDRs which may be optionally linked through linkers. In addition, it may also include a monovalent single-chain molecule Fv (scFv) formed by the pairing of VL and VH (see Bird et al. (1988) Science 242: 423-426; as well as Huston et al. (1988) Proc. Natl. Acad. Sci. 85: 5879-5883). The "antigen binding fragment" may also include an immunoglobulin fusion protein, where the fusion protein includes a binding domain selected from a group consisting of: (1) a binding domain polypeptide fused with the immunoglobulin hinge region polypeptide; (2) an immunoglobulin heavy chain CH2 constant region fused with the hinge region; and (3) an immunoglobulin heavy chain CH3 constant region fused with the CH2 constant region. For example, the antigen binding fragment may further include a single-domain antibody.

In the present application, the term "monoclonal antibody" generally refers to a population of substantially homogeneous antibodies, that is, various antibodies contained in the population are the same except potential naturally occurring mutations present in a trace amount. The monoclonal antibody is highly specific, and directly targets a single antigenic site. For example, the monoclonal antibody can be prepared by a hybridoma technology or produced in bacteria, eukaryotic animal or plant cells by using recombinant DNA methods. The monoclonal antibody can also be obtained from a phage antibody library, by using a technology as described in, e.g., Clackson et al., Nature, 352:624-628 (1991) and Marks et al., Mol. Biol., 222:581-597 (1991).

In the present application, the term "chimeric antibody" generally refers to such an antibody in which a part of the amino acid sequence of each heavy chain or light chain is homogeneous to the corresponding amino acid sequence in an antibody derived from a specific species or belongs to a particular class, while the rest part of the chain is homogeneous to the corresponding sequence in another species. For example, the variable regions of the light chain and the heavy chain are derived from the variable region of the antibody of an animal species (e.g., mouse, rats, and the like), while the constant part is homogeneous to the sequence of an antibody derived from another species (e.g., human). For example, to obtain a chimeric antibody, the variable region can be produced by using non-human B cells or hybridoma cells, while the constant region combined therewith is derived from human. The variable region has the advantage of being easy to prepare, and its specificity is not affected by the origin of the constant region combined therewith. Meanwhile, since the constant region of the chimeric antibody can be derived from human, the chimeric antibody is less likely to elicit an immune response when being injected than using an antibody of which the constant region is not derived from human.

In the present application, the term "humanized antibody" generally refers to a chimeric antibody that includes fewer sequences derived from non-human immunoglobulin, thus reducing the immunogenicity when a heterogeneous antibody is introduced into human, while retaining the full antigen-binding affinity and specificity of the antibody. For example, it is feasible to use CDR transplant (Jones et al., Nature 321:522 (1986)) and variants thereof; including "reshaping", (Verhoeyen, et al., 1988 Science 239:1534-1536; Riechmann, et al., 1988 Nature 332:323-337; Tempest, et al., Bio/Technol 1991 9:266-271), "hyperchimerization", (Queen, et al., 1989 Proc Natl Acad Sci USA 86:10029-10033; Co, et al., 1991 Proc Natl Acad Sci USA 88:2869-2873; Co, et al., 1992 J Immunol 148:1149-1154), and "veneering", (Mark, et al., "Derivation of therapeutically active humanized and veneered anti-CD18 antibodies." In: Metcalf B W, Dalton B J, eds. Cellular adhesion: molecular definition to therapeutic potential. New York: Plenum Press, 1994: 291-312), surface remodeling (U.S. Patent US5639641) and other technical means to humanize the binding domain of non-human sources. If other regions, such as the hinge region and the constant region domain, are also derived from non-human sources, these regions can also be humanized.

In the present application, the term "ribosome skipping site", also known as internal ribosome entry site (IRES), generally refers to a nucleotide sequence located in the middle of the mRNA sequence that is reserved for the initiation of transcription. The ribosome skipping site may allow the initiation of translation in a cap-independent manner. IRES is generally located in 5'UTR. In the present application, the ribosome skipping site may include a sequence of positions from 1 to 578 in SEQ ID NO: 145.

In the present application, the term "tumor" generally refers to a neoplasm formed by the proliferation of local tissue cells. For example, the tumor may include a solid tumor. For example, the tumor may include an AFP expression-associated tumor. The term "AFP expression-associated tumor" generally refers to altered expression of AFP in the tumor microenvironment or in the tumor compared to that in normal cells. For example, the "AFP expression-associated tumor" may be a tumor in which the expression level of AFP in the tumor microenvironment or in the tumor is upregulated compared to that in normal cells. The AFP protein expression-associated tumor may be an AFP-positive tumor. In an AFP-positive tumor, the expression level of AFP protein in the tumor cells or in the tumor microenvironment is about 1%, 5%, 10%, 15%, 20%, 25%, 30%, 35%, 40%, 50%, 60%, 70%, 80% or more higher than that in normal cells.

In the present application, the term "immunoconjugate" generally refers to a conjugate formed by conjugating (e.g. covalently linked by a linker molecule) the additional therapeutic agent to the isolated antigen-binding protein, which can deliver the additional therapeutic agent to the target cell (e.g., a tumor cell) by specific binding of the isolated antigen-binding protein to the antigen on the target cell. In addition, the antigen may also be secreted by the target cell and located in the interstitial space outside the target cell.

In the present application, the term "*K_{D}*" (likewise, "*K_{D}*" or "*K_{D}*") generally refers to "affinity constant" or "equilibrium dissociation constant", and refers to the value obtained at equilibrium in a titration measurement, or by dividing the dissociation rate constant (kd) by the association rate constant (ka). The association rate constant (ka), dissociation rate constant (kd) and equilibrium dissociation constant (*K_{D}*) are used to represent the binding affinity of the binding protein (e.g., the isolated antigen-binding protein of the present application) to the antigen (e.g., AFP protein). Methods for determining the association and dissociation rate constants are well known in the art. The use of a fluorescence-based technology provides high sensitivity and the ability to detect samples in physiological buffers at equilibrium. For example, the *K_{D}* value can be determined by Biacore (biomolecular interaction analysis) (e.g., instrument available from BIAcore International AB, a GE Healthcare company, Uppsala, Sweden), or can be detected through other experimental routes or instrument, such as Octet. In addition, the *K_{D}* value can also be determined by KinExA (Kinetic Exclusion Assay) available from Sapidyne Instruments (Boise, Idaho), or can be determined by surface plasmon resonance (SPR).

In the present application, the term "nucleic acid molecule" generally refers to an isolated nucleotide, deoxyribonucleotide or ribonucleotide of any length, or an analogue thereof isolated from its natural environment or synthesized artificially.

In the present application, the term "vector" generally refers to a nucleic acid molecule capable of self-replication in a suitable host. The vector can transfer the inserted nucleic acid molecule into and/or between cells. The vector may include a vector mainly used for inserting DNA or RNA into cells, a vector mainly used for replicating DNA or RNA, and a vector mainly used for expression of DNA or RNA transcription and/or translation. The vector may be a polynucleotide that can be transcribed and translated into a polypeptide when introduced into a suitable cell. Generally, the vector can produce the desired expression product by culturing suitable cells containing the vector. In the present application, the vector may include a lentiviral vector.

In the present application, the term "cell" generally refers to an individual cell, cell line, or cell culture that can include or has included a plasmid or vector comprising the nucleic acid molecule of the present application, or that can express the chimeric antigen receptor of the present application or the antigen-binding protein of the present application. The cell may include the progeny of a single cell. Due to natural, accidental, or intentional mutations, the progeny cells may not necessarily be exactly the same as the original parent cells in terms of morphology or genome, as long as they can express the chimeric antigen receptor or the antigen-binding protein of the present application. The cell can be obtained by transfecting cells with the vector of the present application *in vitro.* The cell may be a prokaryotic cell (e.g., *E. coli)* or an eukaryotic cell (e.g., yeast cells e.g., COS cells, Chinese Hamster Ovary (CHO) cells, HeLa cells, HEK293 cells, COS-1 cells, NS0 cells, or myeloma cells). In some embodiments, the cell may be an immune cell. For example, the immune cell may be selected from a group consisting of: T cells, B cells, natural killer cells (NK cells), macrophages, NKT cells, monocytes, dendritic cells, granulocytes, lymphocytes, leukocytes and/or peripheral blood mononuclear cells. For example, the immune cell may be a T cell.

In the present application, the term "treating" generally refers to: (i) preventing the occurrence of a disease, disorder or condition in a patient who may be susceptible to the disease, disorder and/or condition but has not yet been diagnosed with it; (ii) suppressing the disease, disorder or condition, i.e., arresting its progression; and (iii) alleviating the disease, disorder or condition, i.e., regressing the disease, disorder and/or condition and/or the symptoms associated with the disease, , disorder and/or condition.

In the present application, the term "polypeptide", "peptide", and "protein" can be used interchangeably and generally refer to a polymer of amino acids of any length. The polymer may be linear or branched, it may contain modified amino acids, and may be interrupted by non-amino acids. These terms also encompass amino acid polymers that have been modified. These modifications may include: disulfide bond formation, glycosylation, lipidation, acetylation, phosphorylation, or any other manipulation (e.g., binding to labeled components). The term "amino acid" includes natural and/or unnatural or synthetic amino acids, including glycine and D and L optical isomers, as well as amino acid analogues and peptide mimetics.

In the present application, the term "polynucleotide", "nucleotide", "nucleotide sequence", "nucleic acid", and "oligonucleotide" can be used interchangeably and generally refer to a polymeric form of nucleotides of any length, such as a deoxyribonucleotide or ribonucleotide, or analogues thereof. A polynucleotide may have any three-dimensional structure and may perform any known or unknown function. The following are non-limiting examples of polynucleotides: coding or noncoding regions of genes or gene fragments, multiple loci (one locus) defined according to linkage analysis, exons, introns, messenger RNA (mRNA), transporter RNA, ribosomal RNA, short interfering RNA (siRNA), short hairpin RNA (shRNA), micro-RNA (miRNA), ribozymes, cDNA, recombinant polynucleotides, branched polynucleotides, plasmids, vectors, isolated DNA of any sequence, isolated RNA of any sequence, nucleic acid probes, and primers. Polynucleotides may include one or more modified nucleotides, such as methylated nucleotides and nucleotide analogues. If present, modification of the nucleotide structure can be performed before or after polymer assembly. The sequence of nucleotides can be interrupted by non-nucleotide components. The polynucleotide can be further modified after polymerization, for example by conjugating with a labeled component.

In addition to particular proteins and nucleotides mentioned herein, the present application may also include their functional variants, derivatives, analogues, homologues and fragments thereof.

The term "functional variant" refers to a polypeptide having substantially the same amino acid sequence or encoded by substantially the same nucleotide sequence as the naturally occurring sequence and capable of having one or more activities of the naturally occurring sequence. In the context of the present application, the variant of any given sequence refers to a sequence in which a particular sequence of residues (either amino acid or nucleotide residues) has been modified so that the polypeptide or polynucleotide remains substantially at least one endogenous function. The variant sequence can be obtained through the addition, deletion, substitution, modification, replacement and/or variation of at least one amino acid residue and/or nucleotide residue present in a naturally occurring protein and/or polynucleotide, as long as the original functional activity is maintained.

In the present application, the term "derivative" generally refers to a polypeptide or polynucleotide of the present application including any substitution, variation, modification, replacement, deletion and/or addition from/to one (or more) amino acid residues of the sequence, provided that the resulting polypeptide or polynucleotide substantially maintains at least one endogenous function.

In the present application, the term "analogue" generally, with respect to a polypeptide or polynucleotide, includes any mimetics of the polypeptide or polynucleotide, that is, a chemical compound having at least one endogenous function of the polypeptide or polynucleotide that the mimetic mimics.

In general, amino acids can be substituted, for example, at least 1 (e.g., 1, 2, 3, 4, 5, 6, 7, 8, 9, 10 or 20 or more) amino acids can be substituted, provided that the modified sequence substantially maintains the required activity or capability. Amino acid substitution may include the use of non-naturally occurring analogues.

The protein or polypeptide used in the present application may also have deletion, insertion or substitution of amino acid residues, where the amino acid residues undergo silent changes and result in functionally equivalent proteins. Intentional amino acid substitutions can be made based on the similarity of the polarity, charge, solubility, hydrophobicity, hydrophilicity, and/or amphoteric properties of the residues, as long as the endogenous function is maintained. For example, negatively charged amino acids include aspartic acid and glutamic acid; positively charged amino acids include lysine and arginine; and amino acids containing uncharged polar head-groups with a similar hydrophilic value include asparagine, glutamine, serine, threonine and tyrosine.

In the present application, the term "AFP antigen " generally refers to a glycoprotein belonging to the family of albumin. The amino acid sequence of human AFP can be found in UniProt/Swiss-Prot Accession No. P02771.

In the present application, the term "T cell", also known as T lymphocyte, is a subtype of white blood cell that plays a central role in cell-mediated immunity. T cells can be distinguished from other lymphocytes such as B cells and natural killer cells by the presence of T cell receptors on the cell surface. In the present application, the T cell may include stem cell-like memory T cells (TSCMs) and/or central memory T cells (TCMs).

In the present application, the term "T cell receptor", generally also known as "TCR", generally refers to a molecular structure of the T cell that specifically recognizes and binds to an antigenic peptide-MHC molecule. The T cell receptor may exist in the form of a complex with a CD3 molecule on the surface of the T cell. The TCR may be a heterodimer immobilized on the cell membrane, mostly consisting of highly variable α and β subunits linked by disulfide bonds; a few consisting of γ and δ peptide chains. The TCR may include a variable region and a constant region, where the constant region may be close to the cell membrane, linking the transmembrane region and the intracellular end, while the variable region is responsible for recognizing the polypeptide/MHC complex.

In the present application, the term "low-density lipoprotein receptor-related protein" (LRP) generally refers to a mosaic protein containing 839 amino acids (after removing 21 amino acid signal peptides). The mosaic protein is embedded in the outer phospholipid layer of LDL (low density lipoprotein) particles, which is an endocytic receptor that can mediate the endocytosis of cholesterol-rich LDL and is a member of the low density lipoprotein receptor (LDLR) gene family. The LRP is most prominently expressed in bronchial epithelial cells and adrenal and cortical tissues. In the present application, the low-density lipoprotein receptor-related protein may include one or more selected from a group consisting of: low-density lipoprotein receptor-related proteins 1-12 or truncated bodies thereof.

In the present application, the term "low-density lipoprotein receptor-related protein 6" (LRP-6) and "low-density lipoprotein receptor-related protein 5" (LRP-5) generally refers to distinct subgroups of the low-density lipoprotein receptor (LDLR) family. The Accession Number of human LRP-6 in UniProt is O75581. The Accession Number of human LRP-5 in UniProt is O75197.

In the present application, the term "truncated body" generally refers to a truncated protein. The truncated body can be obtained by proteolysis or by manipulating the structural gene to eliminate the N- or C-terminal portion of the protein. Alternatively, the truncated body can be obtained by a nonsense mutation that introduces a stop codon into the structural gene, thus leading to the premature termination of translation.

In the present application, the term "and/or" should be understood as meaning either or both of the alternatives.

In the present application, the term "include" generally means the inclusion of the features expressly designated, but not excluding other elements.

In the present application, the term "about" generally refers to varying in a range of 0.5%-10% above or below a specified value, for example, varying in a range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5% or 10% above or below a specified value.

In the present application, the term "include" generally refers to the meaning of comprise, encompass, contain or embrace. In some cases, it also indicates the meaning of "is", or "composed of ......".

### Detailed Description

### Isolated antigen-binding protein of the present application

In one aspect, the present application provides an isolated antigen-binding protein, which, as determined by ForteBio AHC sensor, can specifically bind to a human AFP₁₅₈₋₁₆₆/HLA-A02*01* complex with a *K_{D}* value of 3.1E-09M or less (e.g., the *K_{D}* is not higher than 3.1E-09M, not higher than 3.0E-09M, not higher than 2.9E-09M, not higher than 2.8E-09M, not higher than 2.7E-09M, not higher than 2.6E-09M, not higher than 2.7E-09M, not higher than 2.6E-09M, not higher than 2.3E-09M, not higher than 2.0E-09M, not higher than 1.5E-09M, not higher than 1E-09M or not higher than 5E-10M or less).

In one aspect, the present application provides an isolated antigen-binding protein, which may include at least one CDR in an antibody heavy chain variable region VH, and the VH may include amino acid sequences as set forth in SEQ ID NO: 72 to SEQ ID NO: 84.

In the present application, the HCDRs of the isolated antigen-binding protein can be divided in any form, and HCDRs obtained by division in any form can fall within the scope of protection of the present application as long as the VH is identical to the amino acid sequences as set forth in SEQ ID NO: 72 to SEQ ID NO: 84.

The CDR of an antibody, also known as a complementary determining region, is a part of a variable region. Amino acid residues in this region can be in contact with antigens or antigen epitopes. The CDRs of an antibody can be identified by a variety of numbering systems, such as CCG, Kabat, Chothia, IMGT, AbM, North's, and a combination of Kabat/Chothia, etc. These numbering systems are known in the art, particularly see, e.g., http://www.bioinf.org.uk/abs/index.html#kabatnum. Those skilled in the art can determine the CDR regions using different numbering systems based on the sequence and structure of the antibody. There may be differences among the CDR regions when using different numbering systems. In the present application, the CDR encompasses CDR sequences divided according to any CDR division method; it also encompasses variants thereof, where the variants include substitution, deletion and/or addition of one or more amino acids in the amino acid sequence of the CDR, for example, substitution, deletion and/or insertion of 1-30, 1-20 or 1-10, further for example, 1, 2, 3, 4, 5, 6, 7, 8 or 9 amino acids; and it also encompasses homologs thereof, where the homologs may be amino acid sequences having at least about 85% (e.g., at least about 85%, about 90%, about 91%, about 92%, about 93%, about 94%, about 95%, about 96%, about 97%, about 98%, about 99% or higher) sequence homology with the amino acid sequence of the CDR.

In the present application, the antigen-binding protein may include a heavy chain variable region VH, and the VH may include at least one, two or three of an HCDR1, an HCDR2, and an HCDR3.

In the present application, the HCDR3 of the antigen-binding protein may include an amino acid sequence as set forth in any one of SEQ ID NO: 16, SEQ ID NO: 22, SEQ ID NO: 28, SEQ ID NO: 33, SEQ ID NO: 43, SEQ ID NO: 49, and SEQ ID NO: 54. For example, the HCDR3 sequence of the antigen-binding protein can be defined according to the IMGT numbering system.

In the present application, the HCDR3 of the antigen-binding protein may include an amino acid sequence as set forth in any one of SEQ ID NO: 19, SEQ ID NO: 25, SEQ ID NO: 30, SEQ ID NO: 35, SEQ ID NO: 46, SEQ ID NO: 52, and SEQ ID NO: 56. For example, the HCDR3 sequence of the antigen-binding protein can be defined according to the Kabat numbering system.

In the present application, the HCDR2 of the antigen-binding protein may include an amino acid sequence as set forth in any one of SEQ ID NO: 15, SEQ ID NO: 21, SEQ ID NO: 32, SEQ ID NO: 42, SEQ ID NO: 48, and SEQ ID NO: 53. For example, the HCDR2 sequence of the antigen-binding protein can be defined according to the IMGT numbering system.

In the present application, the HCDR2 of the antigen-binding protein may include an amino acid sequence as set forth in any one of SEQ ID NO: 18, SEQ ID NO: 24, SEQ ID NO: 34, SEQ ID NO: 45, SEQ ID NO: 51, and SEQ ID NO: 55. For example, the HCDR2 sequence of the antigen-binding protein can be defined according to the Kabat numbering system.

In the present application, the HCDR1 of the antigen-binding protein may include an amino acid sequence as set forth in any one of SEQ ID NO: 14, SEQ ID NO: 20, SEQ ID NO: 26, SEQ ID NO: 27, SEQ ID NO: 31, SEQ ID NO: 36, SEQ ID NO: 38, SEQ ID NO: 40, SEQ ID NO: 41, SEQ ID NO: 47, and SEQ ID NO: 57. For example, the HCDR1 sequence of the antigen-binding protein can be defined according to the IMGT numbering system.

In the present application, the HCDR1 of the antigen-binding protein may include an amino acid sequence as set forth in any one of SEQ ID NO: 17, SEQ ID NO: 23, SEQ ID NO: 29, SEQ ID NO: 37, SEQ ID NO: 39, SEQ ID NO: 44, and SEQ ID NO: 50. For example, the HCDR1 sequence of the antigen-binding protein can be defined according to the Kabat numbering system.

For example, the HCDR1 of the antigen-binding protein may include an amino acid sequence as set forth in any one of SEQ ID NO: 14, SEQ ID NO: 20, SEQ ID NO: 26, SEQ ID NO: 27, SEQ ID NO: 31, SEQ ID NO: 36, SEQ ID NO: 38, SEQ ID NO: 40, SEQ ID NO: 41, SEQ ID NO: 47, and SEQ ID NO: 57 ; the HCDR2 may include an amino acid sequence as set forth in any one of SEQ ID NO: 15, SEQ ID NO: 21, SEQ ID NO: 32, SEQ ID NO: 42, SEQ ID NO: 48, and SEQ ID NO: 53; and the HCDR3 may include an amino acid sequence as set forth in any one of SEQ ID NO: 16, SEQ ID NO: 22, SEQ ID NO: 28, SEQ ID NO: 33, SEQ ID NO: 43, SEQ ID NO: 49, and SEQ ID NO: 54. For example, the HCDR1, HCDR2, HCDR3 sequences of the antigen-binding protein can be defined according to the IMGT numbering system.

In the present application, the antigen-binding protein includes an HCDR1, an HCDR2, and an HCDR3, the HCDR3 includes an amino acid sequence as set forth in any one of SEQ ID NO: 19, SEQ ID NO: 25, SEQ ID NO: 30, SEQ ID NO: 35, SEQ ID NO: 46, SEQ ID NO: 52, and SEQ ID NO: 56; the HCDR2 includes an amino acid sequence as set forth in any one of SEQ ID NO: 18, SEQ ID NO: 24, SEQ ID NO: 34, SEQ ID NO: 45, SEQ ID NO: 51, and SEQ ID NO: 55; and the HCDR1 includes an amino acid sequence as set forth in any one of SEQ ID NO: 17, SEQ ID NO: 23, SEQ ID NO: 29, SEQ ID NO: 37, SEQ ID NO: 39, SEQ ID NO: 44, and SEQ ID NO: 50. For example, the HCDR1, HCDR2, and HCDR3 sequences of the antigen-binding protein can be defined according to the Kabat numbering system.

For example, the HCDR1 of the antigen-binding protein may include an amino acid sequence as set forth in SEQ ID NO: 17; the HCDR2 may include an amino acid sequence as set forth in SEQ ID NO: 18; and the HCDR3 may include an amino acid sequence as set forth in SEQ ID NO: 19. For example, the antigen-binding protein may include the antibody 1B3 or an antigen binding fragment having the same HCDR3 (e.g., having the same HCDRs 1-3) therewith.

For example, the HCDR1 of the antigen-binding protein may include an amino acid sequence as set forth in SEQ ID NO: 23; the HCDR2 may include an amino acid sequence as set forth in SEQ ID NO: 24; and the HCDR3 may include an amino acid sequence as set forth in SEQ ID NO: 25. For example, the antigen-binding protein may include the antibody 1C4 or an antigen binding fragment having the same HCDR3 (e.g., having the same HCDRs 1-3) therewith.

For example, the HCDR1 of the antigen-binding protein may include an amino acid sequence as set forth in SEQ ID NO: 23; the HCDR2 may include an amino acid sequence as set forth in SEQ ID NO: 24; and the HCDR3 may include an amino acid sequence as set forth in SEQ ID NO: 25. For example, the antigen-binding protein may include the antibody 1C11 or an antigen binding fragment having the same HCDR3 (e.g., having the same HCDRs 1-3) therewith.

For example, the HCDR1 of the antigen-binding protein may include an amino acid sequence as set forth in SEQ ID NO: 29; the HCDR2 may include an amino acid sequence as set forth in SEQ ID NO: 24; and the HCDR3 may include an amino acid sequence as set forth in SEQ ID NO: 30. For example, the antigen-binding protein may include the antibody 1D12 or an antigen binding fragment having the same HCDR3 (e.g., having the same HCDRs 1-3) therewith.

For example, the HCDR1 of the antigen-binding protein may include an amino acid sequence as set forth in SEQ ID NO: 23; the HCDR2 may include an amino acid sequence as set forth in SEQ ID NO: 34; and the HCDR3 may include an amino acid sequence as set forth in SEQ ID NO: 35. For example, the antigen-binding protein may include the antibody 2F9 or an antigen binding fragment having the same HCDR3 (e.g., having the same HCDRs 1-3) therewith.

For example, the HCDR1 of the antigen-binding protein may include an amino acid sequence as set forth in SEQ ID NO: 37; the HCDR2 may include an amino acid sequence as set forth in SEQ ID NO: 24; and the HCDR3 may include an amino acid sequence as set forth in SEQ ID NO: 25. For example, the antigen-binding protein may include the antibody No. 3 or an antigen binding fragment having the same HCDR3 (e.g., having the same HCDRs 1-3) therewith.

For example, the HCDR1 of the antigen-binding protein may include an amino acid sequence as set forth in SEQ ID NO: 39; the HCDR2 may include an amino acid sequence as set forth in SEQ ID NO: 24; and the HCDR3 may include an amino acid sequence as set forth in SEQ ID NO: 25. For example, the antigen-binding protein may include the antibody No. 4 or an antigen binding fragment having the same HCDR3 (e.g., having the same HCDRs 1-3) therewith.

For example, the HCDR1 of the antigen-binding protein may include an amino acid sequence as set forth in SEQ ID NO: 23; the HCDR2 may include an amino acid sequence as set forth in SEQ ID NO: 24; and the HCDR3 may include an amino acid sequence as set forth in SEQ ID NO: 30. For example, the antigen-binding protein may include the antibody No. 7 or an antigen binding fragment having the same HCDR3 (e.g., having the same HCDRs 1-3) therewith.

For example, the HCDR1 of the antigen-binding protein may include an amino acid sequence as set forth in SEQ ID NO: 23; the HCDR2 may include an amino acid sequence as set forth in SEQ ID NO: 24; and the HCDR3 may include an amino acid sequence as set forth in SEQ ID NO: 30. For example, the antigen-binding protein may include the antibody No. 8 or an antigen binding fragment having the same HCDR3 (e.g., having the same HCDRs 1-3) therewith.

For example, the HCDR1 of the antigen-binding protein may include an amino acid sequence as set forth in SEQ ID NO: 44; the HCDR2 may include an amino acid sequence as set forth in SEQ ID NO: 45; and the HCDR3 may include an amino acid sequence as set forth in SEQ ID NO: 46. For example, the antigen-binding protein may include the antibody No. 15 or an antigen binding fragment having the same HCDR3 (e.g., having the same HCDRs 1-3) therewith.

For example, the HCDR1 of the antigen-binding protein may include an amino acid sequence as set forth in SEQ ID NO: 50; the HCDR2 may include an amino acid sequence as set forth in SEQ ID NO: 51; and the HCDR3 may include an amino acid sequence as set forth in SEQ ID NO: 52. For example, the antigen-binding protein may include the antibody No. 17 or an antigen binding fragment having the same HCDR3 (e.g., having the same HCDRs 1-3) therewith.

For example, the HCDR1 of the antigen-binding protein may include an amino acid sequence as set forth in SEQ ID NO: 23; the HCDR2 may include an amino acid sequence as set forth in SEQ ID NO: 55; and the HCDR3 may include an amino acid sequence as set forth in SEQ ID NO: 56. For example, the antigen-binding protein may include the antibody No. 24 or an antigen binding fragment having the same HCDR3 (e.g., having the same HCDRs 1-3) therewith.

For example, the HCDR1 of the antigen-binding protein may include an amino acid sequence as set forth in SEQ ID NO: 37; the HCDR2 may include an amino acid sequence as set forth in SEQ ID NO: 24; and the HCDR3 may include an amino acid sequence as set forth in SEQ ID NO: 30. For example, the antigen-binding protein may include the antibody No. 29 or an antigen binding fragment having the same HCDR3 (e.g., having the same HCDRs 1-3) therewith.

For example, the VH of the antigen-binding protein may include framework regions H-FR1, H-FR2, H-FR3, and H-FR4.

In the present application, the H-FR1 of the antigen-binding protein may include an amino acid sequence as set forth in any one of SEQ ID NO: 58 to SEQ ID NO: 65.

In the present application, the H-FR2 of the antigen-binding protein may include an amino acid sequence as set forth in SEQ ID NO: 66.

In the present application, the H-FR3 of the antigen-binding protein may include an amino acid sequence as set forth in any one of SEQ ID NO: 67 to SEQ ID NO: 69.

In the present application, the H-FR4 of the antigen-binding protein may include an amino acid sequence as set forth in any one of SEQ ID NO: 70 to SEQ ID NO: 71.

In the present application, the H-FR1 of the antigen-binding protein includes an amino acid sequence as set forth in any one of SEQ ID NO: 58 to SEQ ID NO: 65; the H-FR2 includes an amino acid sequence as set forth in SEQ ID NO: 66; the H-FR3 includes an amino acid sequence as set forth in any one of SEQ ID NO: 67 to SEQ ID NO: 69; and the H-FR4 includes an amino acid sequence as set forth in any one of SEQ ID NO: 70 to SEQ ID NO: 71.

In the present application, the H-FR1 of the antigen-binding protein may include an amino acid sequence as set forth in SEQ ID NO: 58; the H-FR2 may include an amino acid sequence as set forth in SEQ ID NO: 66; the H-FR3 may include an amino acid sequence as set forth in SEQ ID NO: 67; and the H-FR4 may include an amino acid sequence as set forth in SEQ ID NO: 70. For example, the antigen-binding protein may include the antibody 1B3 or an antigen binding fragment having the same H-FRs 1-4 therewith.

In the present application, the H-FR1 of the antigen-binding protein may include an amino acid sequence as set forth in SEQ ID NO: 59; the H-FR2 may include an amino acid sequence as set forth in SEQ ID NO: 66; the H-FR3 may include an amino acid sequence as set forth in SEQ ID NO: 67; and the H-FR4 may include an amino acid sequence as set forth in SEQ ID NO: 70. For example, the antigen-binding protein may include an antibody 1C4 or an antigen binding fragment having the same H-FRs 1-4 therewith.

In the present application, the H-FR1 of the antigen-binding protein may include an amino acid sequence as set forth in SEQ ID NO: 60; the H-FR2 may include an amino acid sequence as set forth in SEQ ID NO: 66; the H-FR3 may include an amino acid sequence as set forth in SEQ ID NO: 67; and the H-FR4 may include an amino acid sequence as set forth in SEQ ID NO: 70. For example, the antigen-binding protein may include an antibody 1C11 or an antigen binding fragment having the same H-FRs 1-4 therewith.

In the present application, the H-FR1 of the antigen-binding protein may include an amino acid sequence as set forth in SEQ ID NO: 60; the H-FR2 may include an amino acid sequence as set forth in SEQ ID NO: 66; the H-FR3 may include an amino acid sequence as set forth in SEQ ID NO: 67; and the H-FR4 may include an amino acid sequence as set forth in SEQ ID NO: 70. For example, the antigen-binding protein may include an antibody 1D12 or an antigen binding fragment having the same H-FRs 1-4 therewith.

In the present application, the H-FR1 of the antigen-binding protein may include an amino acid sequence as set forth in SEQ ID NO: 61; the H-FR2 may include an amino acid sequence as set forth in SEQ ID NO: 66; the H-FR3 may include an amino acid sequence as set forth in SEQ ID NO: 67; and the H-FR4 may include an amino acid sequence as set forth in SEQ ID NO: 70. For example, the antigen-binding protein may include an antibody 2F9 or an antigen binding fragment having the same H-FRs 1-4 therewith.

In the present application, the H-FR1 of the antigen-binding protein may include an amino acid sequence as set forth in SEQ ID NO: 59; the H-FR2 may include an amino acid sequence as set forth in SEQ ID NO: 66; the H-FR3 may include an amino acid sequence as set forth in SEQ ID NO: 67; and the H-FR4 may include an amino acid sequence as set forth in SEQ ID NO: 70. For example, the antigen-binding protein may include the antibody No. 3 or an antigen binding fragment having the same H-FRs 1-4 therewith.

In the present application, the H-FR1 of the antigen-binding protein may include an amino acid sequence as set forth in SEQ ID NO: 62; the H-FR2 may include an amino acid sequence as set forth in SEQ ID NO: 66; the H-FR3 may include an amino acid sequence as set forth in SEQ ID NO: 67; and the H-FR4 may include an amino acid sequence as set forth in SEQ ID NO: 70. For example, the antigen-binding protein may include the antibody No. 4 or an antigen binding fragment having the same H-FRs 1-4 therewith.

In the present application, the H-FR1 of the antigen-binding protein may include an amino acid sequence as set forth in SEQ ID NO: 60; the H-FR2 may include an amino acid sequence as set forth in SEQ ID NO: 66; the H-FR3 may include an amino acid sequence as set forth in SEQ ID NO: 67; and the H-FR4 may include an amino acid sequence as set forth in SEQ ID NO: 70. For example, the antigen-binding protein may include the antibody No. 7 or an antigen binding fragment having the same H-FRs 1-4 therewith.

In the present application, the H-FR1 of the antigen-binding protein may include an amino acid sequence as set forth in SEQ ID NO: 63; the H-FR2 may include an amino acid sequence as set forth in SEQ ID NO: 66; the H-FR3 may include an amino acid sequence as set forth in SEQ ID NO: 67; and the H-FR4 may include an amino acid sequence as set forth in SEQ ID NO: 70. For example, the antigen-binding protein may include the antibody No. 8 or an antigen binding fragment having the same H-FRs 1-4 therewith.

In the present application, the H-FR1 of the antigen-binding protein may include an amino acid sequence as set forth in SEQ ID NO: 64; the H-FR2 may include an amino acid sequence as set forth in SEQ ID NO: 66; the H-FR3 may include an amino acid sequence as set forth in SEQ ID NO: 67; and the H-FR4 may include an amino acid sequence as set forth in SEQ ID NO: 70. For example, the antigen-binding protein may include the antibody No. 15 or an antigen binding fragment having the same H-FRs 1-4 therewith.

In the present application, the H-FR1 of the antigen-binding protein may include an amino acid sequence as set forth in SEQ ID NO: 64; the H-FR2 may include an amino acid sequence as set forth in SEQ ID NO: 66; the H-FR3 may include an amino acid sequence as set forth in SEQ ID NO: 68; and the H-FR4 may include an amino acid sequence as set forth in SEQ ID NO: 71. For example, the antigen-binding protein may include the antibody No. 17 or an antigen binding fragment having the same H-FRs 1-4 therewith.

In the present application, the H-FR1 of the antigen-binding protein may include an amino acid sequence as set forth in SEQ ID NO: 61; the H-FR2 may include an amino acid sequence as set forth in SEQ ID NO: 66; the H-FR3 may include an amino acid sequence as set forth in SEQ ID NO: 67; and the H-FR4 may include an amino acid sequence as set forth in SEQ ID NO: 70. For example, the antigen-binding protein may include the antibody No. 24 or an antigen binding fragment having the same H-FRs 1-4 therewith.

In the present application, the H-FR1 of the antigen-binding protein may include an amino acid sequence as set forth in SEQ ID NO: 65; the H-FR2 may include an amino acid sequence as set forth in SEQ ID NO: 66; the H-FR3 may include an amino acid sequence as set forth in SEQ ID NO: 69; and the H-FR4 may include an amino acid sequence as set forth in SEQ ID NO: 70. For example, the antigen-binding protein may include the antibody No. 29 or an antigen binding fragment having the same H-FRs 1-4 therewith.

In the present application, the heavy chain variable region of the antigen-binding protein may include an amino acid sequence as set forth in any one of SEQ ID NO: 72 to SEQ ID NO: 84.

In the present application, the antigen-binding protein may include a heavy chain variable region, and the heavy chain variable region may include HCDRs 1-3, and H-FRs 1-4. For example, the HCDR1 may include an amino acid sequence as set forth in SEQ ID NO: 17; the HCDR2 may include an amino acid sequence as set forth in SEQ ID NO: 18; the HCDR3 may include an amino acid sequence as set forth in SEQ ID NO: 19. For example, the H-FR1 may include an amino acid sequence as set forth in SEQ ID NO: 58; the H-FR2 may include an amino acid sequence as set forth in SEQ ID NO: 66; the H-FR3 may include an amino acid sequence as set forth in SEQ ID NO: 67; the H-FR4 may include an amino acid sequence as set forth in SEQ ID NO: 70. For example, the heavy chain variable region of the antigen-binding protein may include an amino acid sequence as set forth in SEQ ID NO: 72. For example, the antigen-binding protein may include the antibody 1B3 or an antigen-binding protein having the same heavy chain variable region therewith.

In the present application, the antigen-binding protein may include a heavy chain variable region, and the heavy chain variable region may include HCDRs 1-3, and H-FRs 1-4. For example, the HCDR1 may include an amino acid sequence as set forth in SEQ ID NO: 23; the HCDR2 may include an amino acid sequence as set forth in SEQ ID NO: 24; the HCDR3 may include an amino acid sequence as set forth in SEQ ID NO: 25. For example, the H-FR1 may include an amino acid sequence as set forth in SEQ ID NO: 59; the H-FR2 may include an amino acid sequence as set forth in SEQ ID NO: 66; the H-FR3 may include an amino acid sequence as set forth in SEQ ID NO: 67; the H-FR4 may include an amino acid sequence as set forth in SEQ ID NO: 70. For example, the heavy chain variable region of the antigen-binding protein may include an amino acid sequence as set forth in SEQ ID NO: 73. For example, the antigen-binding protein may include the antibody 1C4 or an antigen-binding protein having the same heavy chain variable region therewith.

In the present application, the antigen-binding protein may include a heavy chain variable region, and the heavy chain variable region may include HCDRs 1-3, and H-FRs 1-4. For example, the HCDR1 may include an amino acid sequence as set forth in SEQ ID NO: 23; the HCDR2 may include an amino acid sequence as set forth in SEQ ID NO: 24; the HCDR3 may include an amino acid sequence as set forth in SEQ ID NO: 25. For example, the H-FR1 may include an amino acid sequence as set forth in SEQ ID NO: 60; the H-FR2 may include an amino acid sequence as set forth in SEQ ID NO: 66; the H-FR3 may include an amino acid sequence as set forth in SEQ ID NO: 67; the H-FR4 may include an amino acid sequence as set forth in SEQ ID NO: 70. For example, the heavy chain variable region of the antigen-binding protein may include an amino acid sequence as set forth in SEQ ID NO: 74. For example, the antigen-binding protein may include the antibody 1C11 or an antigen-binding protein having the same heavy chain variable region therewith.

In the present application, the antigen-binding protein may include a heavy chain variable region, and the heavy chain variable region may include HCDRs 1-3, and H-FRs 1-4. For example, the HCDR1 may include an amino acid sequence as set forth in SEQ ID NO: 29; the HCDR2 may include an amino acid sequence as set forth in SEQ ID NO: 24; the HCDR3 may include an amino acid sequence as set forth in SEQ ID NO: 30. For example, the H-FR1 may include an amino acid sequence as set forth in SEQ ID NO: 60; the H-FR2 may include an amino acid sequence as set forth in SEQ ID NO: 66; the H-FR3 may include an amino acid sequence as set forth in SEQ ID NO: 67; the H-FR4 may include an amino acid sequence as set forth in SEQ ID NO: 70. For example, the heavy chain variable region of the antigen-binding protein may include an amino acid sequence as set forth in SEQ ID NO: 75. For example, the antigen-binding protein may include the antibody 1D12 or an antigen-binding protein having the same heavy chain variable region therewith.

In the present application, the antigen-binding protein may include a heavy chain variable region, and the heavy chain variable region may include HCDRs 1-3, and H-FRs 1-4. For example, the HCDR1 may include an amino acid sequence as set forth in SEQ ID NO: 23; the HCDR2 may include an amino acid sequence as set forth in SEQ ID NO: 34; the HCDR3 may include an amino acid sequence as set forth in SEQ ID NO: 35. For example, the H-FR1 may include an amino acid sequence as set forth in SEQ ID NO: 61; the H-FR2 may include an amino acid sequence as set forth in SEQ ID NO: 66; the H-FR3 may include an amino acid sequence as set forth in SEQ ID NO: 67; the H-FR4 may include an amino acid sequence as set forth in SEQ ID NO: 70. For example, the heavy chain variable region of the antigen-binding protein may include an amino acid sequence as set forth in SEQ ID NO: 76. For example, the antigen-binding protein may include the antibody 2F9 or an antigen-binding protein having the same heavy chain variable region therewith.

In the present application, the antigen-binding protein may include a heavy chain variable region, and the heavy chain variable region may include HCDRs 1-3, and H-FRs 1-4. For example, the HCDR1 may include an amino acid sequence as set forth in SEQ ID NO: 37; the HCDR2 may include an amino acid sequence as set forth in SEQ ID NO: 24; the HCDR3 may include an amino acid sequence as set forth in SEQ ID NO: 25. For example, the H-FR1 may include an amino acid sequence as set forth in SEQ ID NO: 59; the H-FR2 may include an amino acid sequence as set forth in SEQ ID NO: 66; the H-FR3 may include an amino acid sequence as set forth in SEQ ID NO: 67; the H-FR4 may include an amino acid sequence as set forth in SEQ ID NO: 70. For example, the heavy chain variable region of the antigen-binding protein may include an amino acid sequence as set forth in SEQ ID NO: 77. For example, the antigen-binding protein may include the antibody No. 3 or an antigen-binding protein having the same heavy chain variable region therewith.

In the present application, the antigen-binding protein may include a heavy chain variable region, and the heavy chain variable region may include HCDRs 1-3, and H-FRs 1-4. For example, the HCDR1 may include an amino acid sequence as set forth in SEQ ID NO: 39; the HCDR2 may include an amino acid sequence as set forth in SEQ ID NO: 24; the HCDR3 may include an amino acid sequence as set forth in SEQ ID NO: 25. For example, the H-FR1 may include an amino acid sequence as set forth in SEQ ID NO: 62; the H-FR2 may include an amino acid sequence as set forth in SEQ ID NO: 66; the H-FR3 may include an amino acid sequence as set forth in SEQ ID NO: 67; the H-FR4 may include an amino acid sequence as set forth in SEQ ID NO: 70. For example, the heavy chain variable region of the antigen-binding protein may include an amino acid sequence as set forth in SEQ ID NO: 78. For example, the antigen-binding protein may include the antibody No. 4 or an antigen-binding protein having the same heavy chain variable region therewith.

In the present application, the antigen-binding protein may include a heavy chain variable region, and the heavy chain variable region may include HCDRs 1-3, and H-FRs 1-4. For example, the HCDR1 may include an amino acid sequence as set forth in SEQ ID NO: 23; the HCDR2 may include an amino acid sequence as set forth in SEQ ID NO: 24; the HCDR3 may include an amino acid sequence as set forth in SEQ ID NO: 30. For example, the H-FR1 may include an amino acid sequence as set forth in SEQ ID NO: 60; the H-FR2 may include an amino acid sequence as set forth in SEQ ID NO: 66; the H-FR3 may include an amino acid sequence as set forth in SEQ ID NO: 67; the H-FR4 may include an amino acid sequence as set forth in SEQ ID NO: 70. For example, the heavy chain variable region of the antigen-binding protein may include an amino acid sequence as set forth in SEQ ID NO: 79. For example, the antigen-binding protein may include the antibody No. 7 or an antigen-binding protein having the same heavy chain variable region therewith.

In the present application, the antigen-binding protein may include a heavy chain variable region, and the heavy chain variable region may include HCDRs 1-3, and H-FRs 1-4. For example, the HCDR1 may include an amino acid sequence as set forth in SEQ ID NO: 23; the HCDR2 may include an amino acid sequence as set forth in SEQ ID NO: 24; the HCDR3 may include an amino acid sequence as set forth in SEQ ID NO: 30. For example, the H-FR1 may include an amino acid sequence as set forth in SEQ ID NO: 63; the H-FR2 may include an amino acid sequence as set forth in SEQ ID NO: 66; the H-FR3 may include an amino acid sequence as set forth in SEQ ID NO: 67; the H-FR4 may include an amino acid sequence as set forth in SEQ ID NO: 70. For example, the heavy chain variable region of the antigen-binding protein may include an amino acid sequence as set forth in SEQ ID NO: 80. For example, the antigen-binding protein may include the antibody No. 8 or an antigen-binding protein having the same heavy chain variable region therewith.

In the present application, the antigen-binding protein may include a heavy chain variable region, and the heavy chain variable region may include HCDRs 1-3, and H-FRs 1-4. For example, the HCDR1 may include an amino acid sequence as set forth in SEQ ID NO: 44; the HCDR2 may include an amino acid sequence as set forth in SEQ ID NO: 45; the HCDR3 may include an amino acid sequence as set forth in SEQ ID NO: 46. For example, the H-FR1 may include an amino acid sequence as set forth in SEQ ID NO: 64; the H-FR2 may include an amino acid sequence as set forth in SEQ ID NO: 66; the H-FR3 may include an amino acid sequence as set forth in SEQ ID NO: 67; the H-FR4 may include an amino acid sequence as set forth in SEQ ID NO: 70. For example, the heavy chain variable region of the antigen-binding protein may include an amino acid sequence as set forth in SEQ ID NO: 81. For example, the antigen-binding protein may include the antibody No. 15 or an antigen-binding protein having the same heavy chain variable region therewith.

In the present application, the antigen-binding protein may include a heavy chain variable region, and the heavy chain variable region may include HCDRs 1-3, and H-FRs 1-4. For example, the HCDR1 may include an amino acid sequence as set forth in SEQ ID NO: 50; the HCDR2 may include an amino acid sequence as set forth in SEQ ID NO: 51; the HCDR3 may include an amino acid sequence as set forth in SEQ ID NO: 52. For example, the H-FR1 may include an amino acid sequence as set forth in SEQ ID NO: 64; the H-FR2 may include an amino acid sequence as set forth in SEQ ID NO: 66; the H-FR3 may include an amino acid sequence as set forth in SEQ ID NO: 68; the H-FR4 may include an amino acid sequence as set forth in SEQ ID NO: 71. For example, the heavy chain variable region of the antigen-binding protein may include an amino acid sequence as set forth in SEQ ID NO: 82. For example, the antigen-binding protein may include the antibody No. 17 or an antigen-binding protein having the same heavy chain variable region therewith.

In the present application, the antigen-binding protein may include a heavy chain variable region, and the heavy chain variable region may include HCDRs 1-3, and H-FRs 1-4. For example, the HCDR1 may include an amino acid sequence as set forth in SEQ ID NO: 23; the HCDR2 may include an amino acid sequence as set forth in SEQ ID NO: 55; the HCDR3 may include an amino acid sequence as set forth in SEQ ID NO: 56. For example, the H-FR1 may include an amino acid sequence as set forth in SEQ ID NO: 61; the H-FR2 may include an amino acid sequence as set forth in SEQ ID NO: 66; the H-FR3 may include an amino acid sequence as set forth in SEQ ID NO: 67; the H-FR4 may include an amino acid sequence as set forth in SEQ ID NO: 70. For example, the heavy chain variable region of the antigen-binding protein may include an amino acid sequence as set forth in SEQ ID NO: 83. For example, the antigen-binding protein may include the antibody No. 24 or an antigen-binding protein having the same heavy chain variable region therewith.

In the present application, the antigen-binding protein may include a heavy chain variable region, and the heavy chain variable region may include HCDRs 1-3, and H-FRs 1-4. For example, the HCDR1 may include an amino acid sequence as set forth in SEQ ID NO: 37; the HCDR2 may include an amino acid sequence as set forth in SEQ ID NO: 24; the HCDR3 may include an amino acid sequence as set forth in SEQ ID NO: 30. For example, the H-FR1 may include an amino acid sequence as set forth in SEQ ID NO: 65; the H-FR2 may include an amino acid sequence as set forth in SEQ ID NO: 66; the H-FR3 may include an amino acid sequence as set forth in SEQ ID NO: 69; the H-FR4 may include an amino acid sequence as set forth in SEQ ID NO: 70. For example, the heavy chain variable region of the antigen-binding protein may include an amino acid sequence as set forth in SEQ ID NO: 84. For example, the antigen-binding protein may include the antibody No. 29 or an antigen-binding protein having the same heavy chain variable region therewith.

In the present application, the isolated antigen-binding protein can compete with a reference antibody for binding to a human AFP₁₅₈₋₁₆₆/HLA-A02*01* complex.

In the present application, the reference antibody may include a heavy chain variable region VH, and the VH may include at least one, two or three of an HCDR1, an HCDR2, and an HCDR3.

In the present application, the HCDR3 of the reference antibody may include an amino acid sequence as set forth in any one of SEQ ID NO: 16, SEQ ID NO: 22, SEQ ID NO: 28, SEQ ID NO: 33, SEQ ID NO: 43, SEQ ID NO: 49, and SEQ ID NO: 54. For example, the HCDR3 sequence of the reference antibody can be defined according to the IMGT numbering system.

In the present application, the HCDR3 of the reference antibody may include an amino acid sequence as set forth in any one of SEQ ID NO: 19, SEQ ID NO: 25, SEQ ID NO: 30, SEQ ID NO: 35, SEQ ID NO: 46, SEQ ID NO: 52, and SEQ ID NO: 56. For example, the HCDR3 sequence of the reference antibody can be defined according to the Kabat numbering system.

In the present application, the HCDR2 of the reference antibody may include an amino acid sequence as set forth in any one of SEQ ID NO: 15, SEQ ID NO: 21, SEQ ID NO: 32, SEQ ID NO: 42, SEQ ID NO: 48, and SEQ ID NO: 53. For example, the HCDR2 sequence of the reference antibody can be defined according to the IMGT numbering system.

In the present application, the HCDR2 of the reference antibody may include an amino acid sequence as set forth in any one of SEQ ID NO: 18, SEQ ID NO: 24, SEQ ID NO: 34, SEQ ID NO: 45, SEQ ID NO: 51, and SEQ ID NO: 55. For example, the HCDR2 sequence of the reference antibody can be defined according to the Kabat numbering system.

In the present application, the HCDR1 of the reference antibody may include an amino acid sequence as set forth in any one of SEQ ID NO: 14, SEQ ID NO: 20, SEQ ID NO: 26, SEQ ID NO: 27, SEQ ID NO: 31, SEQ ID NO: 36, SEQ ID NO: 38, SEQ ID NO: 40, SEQ ID NO: 41, SEQ ID NO: 47, and SEQ ID NO: 57. For example, the HCDR1 sequence of the reference antibody can be defined according to the IMGT numbering system.

In the present application, the HCDR1 of the reference antibody may include an amino acid sequence as set forth in any one of SEQ ID NO: 17, SEQ ID NO: 23, SEQ ID NO: 29, SEQ ID NO: 37, SEQ ID NO: 39, SEQ ID NO: 44, and SEQ ID NO: 50. For example, the HCDR1 sequence of the reference antibody can be defined according to the Kabat numbering system.

For example, the HCDR1 of the reference antibody may include an amino acid sequence as set forth in any one of SEQ ID NO: 14, SEQ ID NO: 20, SEQ ID NO: 26, SEQ ID NO: 27, SEQ ID NO: 31, SEQ ID NO: 36, SEQ ID NO: 38, SEQ ID NO: 40, SEQ ID NO: 41, SEQ ID NO: 47, and SEQ ID NO: 57; the HCDR2 may include an amino acid sequence as set forth in any one of SEQ ID NO: 15, SEQ ID NO: 21, SEQ ID NO: 32, SEQ ID NO: 42, SEQ ID NO: 48, and SEQ ID NO: 53; and the HCDR3 may include an amino acid sequence as set forth in any one of SEQ ID NO: 16, SEQ ID NO: 22, SEQ ID NO: 28, SEQ ID NO: 33, SEQ ID NO: 43, SEQ ID NO: 49, and SEQ ID NO: 54. For example, the HCDR1, HCDR2, HCDR3 sequences of the reference antibody can be defined according to the IMGT numbering system.

In the present application, the reference antibody includes an HCDR1, an HCDR2, and an HCDR3, the HCDR3 includes an amino acid sequence as set forth in any one of SEQ ID NO: 19, SEQ ID NO: 25, SEQ ID NO: 30, SEQ ID NO: 35, SEQ ID NO: 46, SEQ ID NO: 52, and SEQ ID NO: 56; the HCDR2 includes an amino acid sequence as set forth in any one of SEQ ID NO: 18, SEQ ID NO: 24, SEQ ID NO: 34, SEQ ID NO: 45, SEQ ID NO: 51, and SEQ ID NO: 55; and the HCDR1 includes an amino acid sequence as set forth in any one of SEQ ID NO: 17, SEQ ID NO: 23, SEQ ID NO: 29, SEQ ID NO: 37, SEQ ID NO: 39, SEQ ID NO: 44, and SEQ ID NO: 50. For example, the HCDR1, HCDR2, and HCDR3 sequences of the reference antibody can be defined according to the Kabat numbering system.

For example, the HCDR1 of the reference antibody may include an amino acid sequence as set forth in SEQ ID NO: 17; the HCDR2 may include an amino acid sequence as set forth in SEQ ID NO: 18; and the HCDR3 may include an amino acid sequence as set forth in SEQ ID NO: 19. For example, the reference antibody may include the antibody 1B3 or an antigen binding fragment having the same HCDR3 (e.g., having the same HCDRs 1-3) therewith.

For example, the HCDR1 of the reference antibody may include an amino acid sequence as set forth in SEQ ID NO: 23; the HCDR2 may include an amino acid sequence as set forth in SEQ ID NO: 24; and the HCDR3 may include an amino acid sequence as set forth in SEQ ID NO: 25. For example, the reference antibody may include the antibody 1C4 or an antigen binding fragment having the same HCDR3 (e.g., having the same HCDRs 1-3) therewith.

For example, the HCDR1 of the reference antibody may include an amino acid sequence as set forth in SEQ ID NO: 23; the HCDR2 may include an amino acid sequence as set forth in SEQ ID NO: 24; and the HCDR3 may include an amino acid sequence as set forth in SEQ ID NO: 25. For example, the reference antibody may include the antibody 1C11 or an antigen binding fragment having the same HCDR3 (e.g., having the same HCDRs 1-3) therewith.

For example, the HCDR1 of the reference antibody may include an amino acid sequence as set forth in SEQ ID NO: 29; the HCDR2 may include an amino acid sequence as set forth in SEQ ID NO: 24; and the HCDR3 may include an amino acid sequence as set forth in SEQ ID NO: 30. For example, the reference antibody may include the antibody 1D12 or an antigen binding fragment having the same HCDR3 (e.g., having the same HCDRs 1-3) therewith.

For example, the HCDR1 of the reference antibody may include an amino acid sequence as set forth in SEQ ID NO: 23; the HCDR2 may include an amino acid sequence as set forth in SEQ ID NO: 34; and the HCDR3 may include an amino acid sequence as set forth in SEQ ID NO: 35. For example, the reference antibody may include the antibody 2F9 or an antigen binding fragment having the same HCDR3 (e.g., having the same HCDRs 1-3) therewith.

For example, the HCDR1 of the reference antibody may include an amino acid sequence as set forth in SEQ ID NO: 37; the HCDR2 may include an amino acid sequence as set forth in SEQ ID NO: 24; and the HCDR3 may include an amino acid sequence as set forth in SEQ ID NO: 25. For example, the reference antibody may include the antibody No. 3 or an antigen binding fragment having the same HCDR3 (e.g., having the same HCDRs 1-3) therewith.

For example, the HCDR1 of the reference antibody may include an amino acid sequence as set forth in SEQ ID NO: 39; the HCDR2 may include an amino acid sequence as set forth in SEQ ID NO: 24; and the HCDR3 may include an amino acid sequence as set forth in SEQ ID NO: 25. For example, the reference antibody may include the antibody No. 4 or an antigen binding fragment having the same HCDR3 (e.g., having the same HCDRs 1-3) therewith.

For example, the HCDR1 of the reference antibody may include an amino acid sequence as set forth in SEQ ID NO: 23; the HCDR2 may include an amino acid sequence as set forth in SEQ ID NO: 24; and the HCDR3 may include an amino acid sequence as set forth in SEQ ID NO: 30. For example, the reference antibody may include the antibody No. 7 or an antigen binding fragment having the same HCDR3 (e.g., having the same HCDRs 1-3) therewith.

For example, the HCDR1 of the reference antibody may include an amino acid sequence as set forth in SEQ ID NO: 23; the HCDR2 may include an amino acid sequence as set forth in SEQ ID NO: 24; and the HCDR3 may include an amino acid sequence as set forth in SEQ ID NO: 30. For example, the reference antibody may include the antibody No. 8 or an antigen binding fragment having the same HCDR3 (e.g., having the same HCDRs 1-3) therewith.

For example, the HCDR1 of the reference antibody may include an amino acid sequence as set forth in SEQ ID NO: 44; the HCDR2 may include an amino acid sequence as set forth in SEQ ID NO: 45; and the HCDR3 may include an amino acid sequence as set forth in SEQ ID NO: 46. For example, the reference antibody may include the antibody No. 15 or an antigen binding fragment having the same HCDR3 (e.g., having the same HCDRs 1-3) therewith.

For example, the HCDR1 of the reference antibody may include an amino acid sequence as set forth in SEQ ID NO: 50; the HCDR2 may include an amino acid sequence as set forth in SEQ ID NO: 51; and the HCDR3 may include an amino acid sequence as set forth in SEQ ID NO: 52. For example, the reference antibody may include the antibody No. 17 or an antigen binding fragment having the same HCDR3 (e.g., having the same HCDRs 1-3) therewith.

For example, the HCDR1 of the reference antibody may include an amino acid sequence as set forth in SEQ ID NO: 23; the HCDR2 may include an amino acid sequence as set forth in SEQ ID NO: 55; and the HCDR3 may include an amino acid sequence as set forth in SEQ ID NO: 56. For example, the reference antibody may include the antibody No. 24 or an antigen binding fragment having the same HCDR3 (e.g., having the same HCDRs 1-3) therewith.

For example, the HCDR1 of the reference antibody may include an amino acid sequence as set forth in SEQ ID NO: 37; the HCDR2 may include an amino acid sequence as set forth in SEQ ID NO: 24; and the HCDR3 may include an amino acid sequence as set forth in SEQ ID NO: 30. For example, the reference antibody may include the antibody No. 29 or an antigen binding fragment having the same HCDR3 (e.g., having the same HCDRs 1-3) therewith.

### Chimeric antigen receptor and modified cell

In the present application, the targeting moiety of the chimeric antigen receptor may include a heavy chain variable region VH, and the VH may include at least one, two or three of an HCDR1, an HCDR2, and an HCDR3.

In the present application, the HCDR3 of the targeting moiety of the chimeric antigen receptor may include an amino acid sequence as set forth in any one of SEQ ID NO: 16, SEQ ID NO: 22, SEQ ID NO: 28, SEQ ID NO: 33, SEQ ID NO: 43, SEQ ID NO: 49, and SEQ ID NO: 54. For example, the HCDR3 sequence of the targeting moiety of the chimeric antigen receptor can be defined according to the IMGT numbering system.

In the present application, the HCDR3 of the targeting moiety of the chimeric antigen receptor may include an amino acid sequence as set forth in any one of SEQ ID NO: 19, SEQ ID NO: 25, SEQ ID NO: 30, SEQ ID NO: 35, SEQ ID NO: 46, SEQ ID NO: 52, and SEQ ID NO: 56. For example, the HCDR3 sequence of the targeting moiety of the chimeric antigen receptor can be defined according to the Kabat numbering system.

In the present application, the HCDR2 of the targeting moiety of the chimeric antigen receptor may include an amino acid sequence as set forth in any one of SEQ ID NO: 15, SEQ ID NO: 21, SEQ ID NO: 32, SEQ ID NO: 42, SEQ ID NO: 48, and SEQ ID NO: 53. For example, the HCDR2 sequence of the targeting moiety of the chimeric antigen receptor can be defined according to the IMGT numbering system.

In the present application, the HCDR2 of the targeting moiety of the chimeric antigen receptor may include an amino acid sequence as set forth in any one of SEQ ID NO: 18, SEQ ID NO: 24, SEQ ID NO: 34, SEQ ID NO: 45, SEQ ID NO: 51, and SEQ ID NO: 55. For example, the HCDR2 sequence of the targeting moiety of the chimeric antigen receptor can be defined according to the Kabat numbering system.

In the present application, the HCDR1 of the targeting moiety of the chimeric antigen receptor may include an amino acid sequence as set forth in any one of SEQ ID NO: 14, SEQ ID NO: 20, SEQ ID NO: 26, SEQ ID NO: 27, SEQ ID NO: 31, SEQ ID NO: 36, SEQ ID NO: 38, SEQ ID NO: 40, SEQ ID NO: 41, SEQ ID NO: 47, and SEQ ID NO: 57. For example, the HCDR1 sequence of the targeting moiety of the chimeric antigen receptor can be defined according to the IMGT numbering system.

In the present application, the HCDR1 of the targeting moiety of the chimeric antigen receptor may include an amino acid sequence as set forth in any one of SEQ ID NO: 17, SEQ ID NO: 23, SEQ ID NO: 29, SEQ ID NO: 37, SEQ ID NO: 39, SEQ ID NO: 44, and SEQ ID NO: 50. For example, the HCDR1 sequence of the targeting moiety of the chimeric antigen receptor can be defined according to the Kabat numbering system.

For example, the HCDR1 of the targeting moiety of the chimeric antigen receptor may include an amino acid sequence as set forth in any one of SEQ ID NO: 14, SEQ ID NO: 20, SEQ ID NO: 26, SEQ ID NO: 27, SEQ ID NO: 31, SEQ ID NO: 36, SEQ ID NO: 38, SEQ ID NO: 40, SEQ ID NO: 41, SEQ ID NO: 47, and SEQ ID NO: 57; the HCDR2 may include an amino acid sequence as set forth in any one of SEQ ID NO: 15, SEQ ID NO: 21, SEQ ID NO: 32, SEQ ID NO: 42, SEQ ID NO: 48, and SEQ ID NO: 53; and the HCDR3 may include an amino acid sequence as set forth in any one of SEQ ID NO: 16, SEQ ID NO: 22, SEQ ID NO: 28, SEQ ID NO: 33, SEQ ID NO: 43, SEQ ID NO: 49, and SEQ ID NO: 54. For example, the HCDR1, HCDR2, HCDR3 sequences of the targeting moiety of the chimeric antigen receptor can be defined according to the IMGT numbering system.

In the present application, the targeting moiety of the chimeric antigen receptor includes an HCDR1, an HCDR2, and an HCDR3, the HCDR3 includes an amino acid sequence as set forth in any one of SEQ ID NO: 19, SEQ ID NO: 25, SEQ ID NO: 30, SEQ ID NO: 35, SEQ ID NO: 46, SEQ ID NO: 52, and SEQ ID NO: 56; the HCDR2 includes an amino acid sequence as set forth in any one of SEQ ID NO: 18, SEQ ID NO: 24, SEQ ID NO: 34, SEQ ID NO: 45, SEQ ID NO: 51, and SEQ ID NO: 55; and the HCDR1 includes an amino acid sequence as set forth in any one of SEQ ID NO: 17, SEQ ID NO: 23, SEQ ID NO: 29, SEQ ID NO: 37, SEQ ID NO: 39, SEQ ID NO: 44, and SEQ ID NO: 50. For example, the HCDR1, HCDR2, and HCDR3 sequences of the targeting moiety of the chimeric antigen receptor can be defined according to the Kabat numbering system.

For example, the HCDR1 of the targeting moiety of the chimeric antigen receptor may include an amino acid sequence as set forth in SEQ ID NO: 17; the HCDR2 may include an amino acid sequence as set forth in SEQ ID NO: 18; and the HCDR3 may include an amino acid sequence as set forth in SEQ ID NO: 19. For example, the targeting moiety of the chimeric antigen receptor may include the antibody 1B3 or an antigen binding fragment having the same HCDR3 (e.g., having the same HCDRs 1-3) therewith.

For example, the HCDR1 of the targeting moiety of the chimeric antigen receptor may include an amino acid sequence as set forth in SEQ ID NO: 23; the HCDR2 may include an amino acid sequence as set forth in SEQ ID NO: 24; and the HCDR3 may include an amino acid sequence as set forth in SEQ ID NO: 25. For example, the targeting moiety of the chimeric antigen receptor may include the antibody 1C4 or an antigen binding fragment having the same HCDR3 (e.g., having the same HCDRs 1-3) therewith.

For example, the HCDR1 of the targeting moiety of the chimeric antigen receptor may include an amino acid sequence as set forth in SEQ ID NO: 23; the HCDR2 may include an amino acid sequence as set forth in SEQ ID NO: 24; and the HCDR3 may include an amino acid sequence as set forth in SEQ ID NO: 25. For example, the targeting moiety of the chimeric antigen receptor may include the antibody 1C11 or an antigen binding fragment having the same HCDR3 (e.g., having the same HCDRs 1-3) therewith.

For example, the HCDR1 of the targeting moiety of the chimeric antigen receptor may include an amino acid sequence as set forth in SEQ ID NO: 29; the HCDR2 may include an amino acid sequence as set forth in SEQ ID NO: 24; and the HCDR3 may include an amino acid sequence as set forth in SEQ ID NO: 30. For example, the targeting moiety of the chimeric antigen receptor may include the antibody 1D12 or an antigen binding fragment having the same HCDR3 (e.g., having the same HCDRs 1-3) therewith.

For example, the HCDR1 of the targeting moiety of the chimeric antigen receptor may include an amino acid sequence as set forth in SEQ ID NO: 23; the HCDR2 may include an amino acid sequence as set forth in SEQ ID NO: 34; and the HCDR3 may include an amino acid sequence as set forth in SEQ ID NO: 35. For example, the targeting moiety of the chimeric antigen receptor may include the antibody 2F9 or an antigen binding fragment having the same HCDR3 (e.g., having the same HCDRs 1-3) therewith.

For example, the HCDR1 of the targeting moiety of the chimeric antigen receptor may include an amino acid sequence as set forth in SEQ ID NO: 37; the HCDR2 may include an amino acid sequence as set forth in SEQ ID NO: 24; and the HCDR3 may include an amino acid sequence as set forth in SEQ ID NO: 25. For example, the targeting moiety of the chimeric antigen receptor may include the antibody No. 3 or an antigen binding fragment having the same HCDR3 (e.g., having the same HCDRs 1-3) therewith.

For example, the HCDR1 of the targeting moiety of the chimeric antigen receptor may include an amino acid sequence as set forth in SEQ ID NO: 39; the HCDR2 may include an amino acid sequence as set forth in SEQ ID NO: 24; and the HCDR3 may include an amino acid sequence as set forth in SEQ ID NO: 25. For example, the targeting moiety of the chimeric antigen receptor may include the antibody No. 4 or an antigen binding fragment having the same HCDR3 (e.g., having the same HCDRs 1-3) therewith.

For example, the HCDR1 of the targeting moiety of the chimeric antigen receptor may include an amino acid sequence as set forth in SEQ ID NO: 23; the HCDR2 may include an amino acid sequence as set forth in SEQ ID NO: 24; and the HCDR3 may include an amino acid sequence as set forth in SEQ ID NO: 30. For example, the targeting moiety of the chimeric antigen receptor may include the antibody No. 7 or an antigen binding fragment having the same HCDR3 (e.g., having the same HCDRs 1-3) therewith.

For example, the HCDR1 of the targeting moiety of the chimeric antigen receptor may include an amino acid sequence as set forth in SEQ ID NO: 23; the HCDR2 may include an amino acid sequence as set forth in SEQ ID NO: 24; and the HCDR3 may include an amino acid sequence as set forth in SEQ ID NO: 30. For example, the targeting moiety of the chimeric antigen receptor may include the antibody No. 8 or an antigen binding fragment having the same HCDR3 (e.g., having the same HCDRs 1-3) therewith.

For example, the HCDR1 of the targeting moiety of the chimeric antigen receptor may include an amino acid sequence as set forth in SEQ ID NO: 44; the HCDR2 may include an amino acid sequence as set forth in SEQ ID NO: 45; and the HCDR3 may include an amino acid sequence as set forth in SEQ ID NO: 46. For example, the targeting moiety of the chimeric antigen receptor may include the antibody No. 15 or an antigen binding fragment having the same HCDR3 (e.g., having the same HCDRs 1-3) therewith.

For example, the HCDR1 of the targeting moiety of the chimeric antigen receptor may include an amino acid sequence as set forth in SEQ ID NO: 50; the HCDR2 may include an amino acid sequence as set forth in SEQ ID NO: 51; and the HCDR3 may include an amino acid sequence as set forth in SEQ ID NO: 52. For example, the targeting moiety of the chimeric antigen receptor may include the antibody No. 17 or an antigen binding fragment having the same HCDR3 (e.g., having the same HCDRs 1-3) therewith.

For example, the HCDR1 of the targeting moiety of the chimeric antigen receptor may include an amino acid sequence as set forth in SEQ ID NO: 23; the HCDR2 may include an amino acid sequence as set forth in SEQ ID NO: 55; and the HCDR3 may include an amino acid sequence as set forth in SEQ ID NO: 56. For example, the targeting moiety of the chimeric antigen receptor may include the antibody No. 24 or an antigen binding fragment having the same HCDR3 (e.g., having the same HCDRs 1-3) therewith.

For example, the HCDR1 of the targeting moiety of the chimeric antigen receptor may include an amino acid sequence as set forth in SEQ ID NO: 37; the HCDR2 may include an amino acid sequence as set forth in SEQ ID NO: 24; and the HCDR3 may include an amino acid sequence as set forth in SEQ ID NO: 30. For example, the targeting moiety of the chimeric antigen receptor may include the antibody No. 29 or an antigen binding fragment having the same HCDR3 (e.g., having the same HCDRs 1-3) therewith.

For example, the VH of the targeting moiety of the chimeric antigen receptor may include framework regions H-FR1, H-FR2, H-FR3, and H-FR4.

In the present application, the H-FR1 of the targeting moiety of the chimeric antigen receptor may include an amino acid sequence as set forth in any one of SEQ ID NO: 58 to SEQ ID NO: 65.

In the present application, the H-FR2 of the targeting moiety of the chimeric antigen receptor may include an amino acid sequence as set forth in SEQ ID NO: 66.

In the present application, the H-FR3 of the targeting moiety of the chimeric antigen receptor may include an amino acid sequence as set forth in any one of SEQ ID NO: 67 to SEQ ID NO: 69.

In the present application, the H-FR4 of the targeting moiety of the chimeric antigen receptor may include an amino acid sequence as set forth in any one of SEQ ID NO: 70 to SEQ ID NO: 71.

In the present application, the H-FR1 of the targeting moiety of the chimeric antigen receptor includes an amino acid sequence as set forth in any one of SEQ ID NO: 58 to SEQ ID NO: 65; the H-FR2 includes an amino acid sequence as set forth in SEQ ID NO: 66; the H-FR3 includes an amino acid sequence as set forth in any one of SEQ ID NO: 67 to SEQ ID NO: 69; and the H-FR4 includes an amino acid sequence as set forth in any one of SEQ ID NO: 70 to SEQ ID NO: 71.

In the present application, the H-FR1 of the targeting moiety of the chimeric antigen receptor may include an amino acid sequence as set forth in SEQ ID NO: 58; the H-FR2 may include an amino acid sequence as set forth in SEQ ID NO: 66; the H-FR3 may include an amino acid sequence as set forth in SEQ ID NO: 67; and the H-FR4 may include an amino acid sequence as set forth in SEQ ID NO: 70. For example, the targeting moiety of the chimeric antigen receptor may include the antibody 1B3 or an antigen binding fragment having the same H-FRs 1-4 therewith.

In the present application, the H-FR1 of the targeting moiety of the chimeric antigen receptor may include an amino acid sequence as set forth in SEQ ID NO: 59; the H-FR2 may include an amino acid sequence as set forth in SEQ ID NO: 66; the H-FR3 may include an amino acid sequence as set forth in SEQ ID NO: 67; and the H-FR4 may include an amino acid sequence as set forth in SEQ ID NO: 70. For example, the targeting moiety of the chimeric antigen receptor may include the antibody 1C4 or an antigen binding fragment having the same H-FRs 1-4 therewith.

In the present application, the H-FR1 of the targeting moiety of the chimeric antigen receptor may include an amino acid sequence as set forth in SEQ ID NO: 60; the H-FR2 may include an amino acid sequence as set forth in SEQ ID NO: 66; the H-FR3 may include an amino acid sequence as set forth in SEQ ID NO: 67; and the H-FR4 may include an amino acid sequence as set forth in SEQ ID NO: 70. For example, the targeting moiety of the chimeric antigen receptor may include the antibody 1C11 or an antigen binding fragment having the same H-FRs 1-4 therewith.

In the present application, the H-FR1 of the targeting moiety of the chimeric antigen receptor may include an amino acid sequence as set forth in SEQ ID NO: 60; the H-FR2 may include an amino acid sequence as set forth in SEQ ID NO: 66; the H-FR3 may include an amino acid sequence as set forth in SEQ ID NO: 67; and the H-FR4 may include an amino acid sequence as set forth in SEQ ID NO: 70. For example, the targeting moiety of the chimeric antigen receptor may include the antibody 1D12 or an antigen binding fragment having the same H-FRs 1-4 therewith.

In the present application, the H-FR1 of the targeting moiety of the chimeric antigen receptor may include an amino acid sequence as set forth in SEQ ID NO: 61; the H-FR2 may include an amino acid sequence as set forth in SEQ ID NO: 66; the H-FR3 may include an amino acid sequence as set forth in SEQ ID NO: 67; and the H-FR4 may include an amino acid sequence as set forth in SEQ ID NO: 70. For example, the targeting moiety of the chimeric antigen receptor may include the antibody 2F9 or an antigen binding fragment having the same H-FRs 1-4 therewith.

In the present application, the H-FR1 of the targeting moiety of the chimeric antigen receptor may include an amino acid sequence as set forth in SEQ ID NO: 59; the H-FR2 may include an amino acid sequence as set forth in SEQ ID NO: 66; the H-FR3 may include an amino acid sequence as set forth in SEQ ID NO: 67; and the H-FR4 may include an amino acid sequence as set forth in SEQ ID NO: 70. For example, the targeting moiety of the chimeric antigen receptor may include the antibody No. 3 or an antigen binding fragment having the same H-FRs 1-4 therewith.

In the present application, the H-FR1 of the targeting moiety of the chimeric antigen receptor may include an amino acid sequence as set forth in SEQ ID NO: 62; the H-FR2 may include an amino acid sequence as set forth in SEQ ID NO: 66; the H-FR3 may include an amino acid sequence as set forth in SEQ ID NO: 67; and the H-FR4 may include an amino acid sequence as set forth in SEQ ID NO: 70. For example, the targeting moiety of the chimeric antigen receptor may include the antibody No. 4 or an antigen binding fragment having the same H-FRs 1-4 therewith.

In the present application, the H-FR1 of the targeting moiety of the chimeric antigen receptor may include an amino acid sequence as set forth in SEQ ID NO: 60; the H-FR2 may include an amino acid sequence as set forth in SEQ ID NO: 66; the H-FR3 may include an amino acid sequence as set forth in SEQ ID NO: 67; and the H-FR4 may include an amino acid sequence as set forth in SEQ ID NO: 70. For example, the targeting moiety of the chimeric antigen receptor may include the antibody No. 7 or an antigen binding fragment having the same H-FRs 1-4 therewith.

In the present application, the H-FR1 of the targeting moiety of the chimeric antigen receptor may include an amino acid sequence as set forth in SEQ ID NO: 63; the H-FR2 may include an amino acid sequence as set forth in SEQ ID NO: 66; the H-FR3 may include an amino acid sequence as set forth in SEQ ID NO: 67; and the H-FR4 may include an amino acid sequence as set forth in SEQ ID NO: 70. For example, the targeting moiety of the chimeric antigen receptor may include the antibody No. 8 or an antigen binding fragment having the same H-FRs 1-4 therewith.

In the present application, the H-FR1 of the targeting moiety of the chimeric antigen receptor may include an amino acid sequence as set forth in SEQ ID NO: 64; the H-FR2 may include an amino acid sequence as set forth in SEQ ID NO: 66; the H-FR3 may include an amino acid sequence as set forth in SEQ ID NO: 67; and the H-FR4 may include an amino acid sequence as set forth in SEQ ID NO: 70. For example, the targeting moiety of the chimeric antigen receptor may include the antibody No. 15 or an antigen binding fragment having the same H-FRs 1-4 therewith.

In the present application, the H-FR1 of the targeting moiety of the chimeric antigen receptor may include an amino acid sequence as set forth in SEQ ID NO: 64; the H-FR2 may include an amino acid sequence as set forth in SEQ ID NO: 66; the H-FR3 may include an amino acid sequence as set forth in SEQ ID NO: 68; and the H-FR4 may include an amino acid sequence as set forth in SEQ ID NO: 71. For example, the targeting moiety of the chimeric antigen receptor may include the antibody No. 17 or an antigen binding fragment having the same H-FRs 1-4 therewith.

In the present application, the H-FR1 of the targeting moiety of the chimeric antigen receptor may include an amino acid sequence as set forth in SEQ ID NO: 61; the H-FR2 may include an amino acid sequence as set forth in SEQ ID NO: 66; the H-FR3 may include an amino acid sequence as set forth in SEQ ID NO: 67; and the H-FR4 may include an amino acid sequence as set forth in SEQ ID NO: 70. For example, the targeting moiety of the chimeric antigen receptor may include the antibody No. 24 or an antigen binding fragment having the same H-FRs 1-4 therewith.

In the present application, the H-FR1 of the targeting moiety of the chimeric antigen receptor may include an amino acid sequence as set forth in SEQ ID NO: 65; the H-FR2 may include an amino acid sequence as set forth in SEQ ID NO: 66; the H-FR3 may include an amino acid sequence as set forth in SEQ ID NO: 69; and the H-FR4 may include an amino acid sequence as set forth in SEQ ID NO: 70. For example, the targeting moiety of the chimeric antigen receptor may include the antibody No. 29 or an antigen binding fragment having the same H-FRs 1-4 therewith.

In the present application, the heavy chain variable region of the targeting moiety of the chimeric antigen receptor may include an amino acid sequence as set forth in any one of SEQ ID NO: 72 to SEQ ID NO: 84.

In the present application, the targeting moiety of the chimeric antigen receptor may include a heavy chain variable region, and the heavy chain variable region may include HCDRs 1-3 and H-FRs 1-4. For example, the HCDR1 may include an amino acid sequence as set forth in SEQ ID NO: 17; the HCDR2 may include an amino acid sequence as set forth in SEQ ID NO: 18; the HCDR3 may include an amino acid sequence as set forth in SEQ ID NO: 19. For example, the H-FR1 may include an amino acid sequence as set forth in SEQ ID NO: 58; the H-FR2 may include an amino acid sequence as set forth in SEQ ID NO: 66; the H-FR3 may include an amino acid sequence as set forth in SEQ ID NO: 67; the H-FR4 may include an amino acid sequence as set forth in SEQ ID NO: 70. For example, the heavy chain variable region of the targeting moiety of the chimeric antigen receptor may include an amino acid sequence as set forth in SEQ ID NO: 72. For example, the targeting moiety of the chimeric antigen receptor may include the antibody 1B3 or a targeting moiety of a chimeric antigen receptor having the same heavy chain variable region therewith.

In the present application, the targeting moiety of the chimeric antigen receptor may include a heavy chain variable region, and the heavy chain variable region may include HCDRs 1-3 and H-FRs 1-4. For example, the HCDR1 may include an amino acid sequence as set forth in SEQ ID NO: 23; the HCDR2 may include an amino acid sequence as set forth in SEQ ID NO: 24; the HCDR3 may include an amino acid sequence as set forth in SEQ ID NO: 25. For example, the H-FR1 may include an amino acid sequence as set forth in SEQ ID NO: 59; the H-FR2 may include an amino acid sequence as set forth in SEQ ID NO: 66; the H-FR3 may include an amino acid sequence as set forth in SEQ ID NO: 67; the H-FR4 may include an amino acid sequence as set forth in SEQ ID NO: 70. For example, the heavy chain variable region of the targeting moiety of the chimeric antigen receptor may include an amino acid sequence as set forth in SEQ ID NO: 73. For example, the targeting moiety of the chimeric antigen receptor may include the antibody 1C4 or a targeting moiety of a chimeric antigen receptor having the same heavy chain variable region therewith.

In the present application, the targeting moiety of the chimeric antigen receptor may include a heavy chain variable region, and the heavy chain variable region may include HCDRs 1-3 and H-FRs 1-4. For example, the HCDR1 may include an amino acid sequence as set forth in SEQ ID NO: 23; the HCDR2 may include an amino acid sequence as set forth in SEQ ID NO: 24; the HCDR3 may include an amino acid sequence as set forth in SEQ ID NO: 25. For example, the H-FR1 may include an amino acid sequence as set forth in SEQ ID NO: 60; the H-FR2 may include an amino acid sequence as set forth in SEQ ID NO: 66; the H-FR3 may include an amino acid sequence as set forth in SEQ ID NO: 67; the H-FR4 may include an amino acid sequence as set forth in SEQ ID NO: 70. For example, the heavy chain variable region of the targeting moiety of the chimeric antigen receptor may include an amino acid sequence as set forth in SEQ ID NO: 74. For example, the targeting moiety of the chimeric antigen receptor may include the antibody 1C11 or a targeting moiety of a chimeric antigen receptor having the same heavy chain variable region therewith.

In the present application, the targeting moiety of the chimeric antigen receptor may include a heavy chain variable region, and the heavy chain variable region may include HCDRs 1-3 and H-FRs 1-4. For example, the HCDR1 may include an amino acid sequence as set forth in SEQ ID NO: 29; the HCDR2 may include an amino acid sequence as set forth in SEQ ID NO: 24; the HCDR3 may include an amino acid sequence as set forth in SEQ ID NO: 30. For example, the H-FR1 may include an amino acid sequence as set forth in SEQ ID NO: 60; the H-FR2 may include an amino acid sequence as set forth in SEQ ID NO: 66; the H-FR3 may include an amino acid sequence as set forth in SEQ ID NO: 67; the H-FR4 may include an amino acid sequence as set forth in SEQ ID NO: 70. For example, the heavy chain variable region of the targeting moiety of the chimeric antigen receptor may include an amino acid sequence as set forth in SEQ ID NO: 75. For example, the targeting moiety of the chimeric antigen receptor may include the antibody 1D12 or a targeting moiety of a chimeric antigen receptor having the same heavy chain variable region therewith.

In the present application, the targeting moiety of the chimeric antigen receptor may include a heavy chain variable region, and the heavy chain variable region may include HCDRs 1-3 and H-FRs 1-4. For example, the HCDR1 may include an amino acid sequence as set forth in SEQ ID NO: 23; the HCDR2 may include an amino acid sequence as set forth in SEQ ID NO: 34; the HCDR3 may include an amino acid sequence as set forth in SEQ ID NO: 35. For example, the H-FR1 may include an amino acid sequence as set forth in SEQ ID NO: 61; the H-FR2 may include an amino acid sequence as set forth in SEQ ID NO: 66; the H-FR3 may include an amino acid sequence as set forth in SEQ ID NO: 67; the H-FR4 may include an amino acid sequence as set forth in SEQ ID NO: 70. For example, the heavy chain variable region of the targeting moiety of the chimeric antigen receptor may include an amino acid sequence as set forth in SEQ ID NO: 76. For example, the targeting moiety of the chimeric antigen receptor may include the antibody 2F9 or a targeting moiety of a chimeric antigen receptor having the same heavy chain variable region therewith.

In the present application, the targeting moiety of the chimeric antigen receptor may include a heavy chain variable region, and the heavy chain variable region may include HCDRs 1-3 and H-FRs 1-4. For example, the HCDR1 may include an amino acid sequence as set forth in SEQ ID NO: 37; the HCDR2 may include an amino acid sequence as set forth in SEQ ID NO: 24; the HCDR3 may include an amino acid sequence as set forth in SEQ ID NO: 25. For example, the H-FR1 may include an amino acid sequence as set forth in SEQ ID NO: 59; the H-FR2 may include an amino acid sequence as set forth in SEQ ID NO: 66; the H-FR3 may include an amino acid sequence as set forth in SEQ ID NO: 67; the H-FR4 may include an amino acid sequence as set forth in SEQ ID NO: 70. For example, the heavy chain variable region of the targeting moiety of the chimeric antigen receptor may include an amino acid sequence as set forth in SEQ ID NO: 77. For example, the targeting moiety of the chimeric antigen receptor may include the antibody No. 3 or a targeting moiety of a chimeric antigen receptor having the same heavy chain variable region therewith.

In the present application, the targeting moiety of the chimeric antigen receptor may include a heavy chain variable region, and the heavy chain variable region may include HCDRs 1-3 and H-FRs 1-4. For example, the HCDR1 may include an amino acid sequence as set forth in SEQ ID NO: 39; the HCDR2 may include an amino acid sequence as set forth in SEQ ID NO: 24; the HCDR3 may include an amino acid sequence as set forth in SEQ ID NO: 25. For example, the H-FR1 may include an amino acid sequence as set forth in SEQ ID NO: 62; the H-FR2 may include an amino acid sequence as set forth in SEQ ID NO: 66; the H-FR3 may include an amino acid sequence as set forth in SEQ ID NO: 67; the H-FR4 may include an amino acid sequence as set forth in SEQ ID NO: 70. For example, the heavy chain variable region of the targeting moiety of the chimeric antigen receptor may include an amino acid sequence as set forth in SEQ ID NO: 78. For example, the targeting moiety of the chimeric antigen receptor may include the antibody No. 4 or a targeting moiety of a chimeric antigen receptor having the same heavy chain variable region therewith.

In the present application, the targeting moiety of the chimeric antigen receptor may include a heavy chain variable region, and the heavy chain variable region may include HCDRs 1-3 and H-FRs 1-4. For example, the HCDR1 may include an amino acid sequence as set forth in SEQ ID NO: 23; the HCDR2 may include an amino acid sequence as set forth in SEQ ID NO: 24; the HCDR3 may include an amino acid sequence as set forth in SEQ ID NO: 30. For example, the H-FR1 may include an amino acid sequence as set forth in SEQ ID NO: 60; the H-FR2 may include an amino acid sequence as set forth in SEQ ID NO: 66; the H-FR3 may include an amino acid sequence as set forth in SEQ ID NO: 67; the H-FR4 may include an amino acid sequence as set forth in SEQ ID NO: 70. For example, the heavy chain variable region of the targeting moiety of the chimeric antigen receptor may include an amino acid sequence as set forth in SEQ ID NO: 79. For example, the targeting moiety of the chimeric antigen receptor may include the antibody No. 7 or a targeting moiety of a chimeric antigen receptor having the same heavy chain variable region therewith.

In the present application, the targeting moiety of the chimeric antigen receptor may include a heavy chain variable region, and the heavy chain variable region may include HCDRs 1-3 and H-FRs 1-4. For example, the HCDR1 may include an amino acid sequence as set forth in SEQ ID NO: 23; the HCDR2 may include an amino acid sequence as set forth in SEQ ID NO: 24; the HCDR3 may include an amino acid sequence as set forth in SEQ ID NO: 30. For example, the H-FR1 may include an amino acid sequence as set forth in SEQ ID NO: 63; the H-FR2 may include an amino acid sequence as set forth in SEQ ID NO: 66; the H-FR3 may include an amino acid sequence as set forth in SEQ ID NO: 67; the H-FR4 may include an amino acid sequence as set forth in SEQ ID NO: 70. For example, the heavy chain variable region of the targeting moiety of the chimeric antigen receptor may include an amino acid sequence as set forth in SEQ ID NO: 80. For example, the targeting moiety of the chimeric antigen receptor may include the antibody No. 8 or a targeting moiety of a chimeric antigen receptor having the same heavy chain variable region therewith.

In the present application, the targeting moiety of the chimeric antigen receptor may include a heavy chain variable region, and the heavy chain variable region may include HCDRs 1-3 and H-FRs 1-4. For example, the HCDR1 may include an amino acid sequence as set forth in SEQ ID NO: 44; the HCDR2 may include an amino acid sequence as set forth in SEQ ID NO: 45; the HCDR3 may include an amino acid sequence as set forth in SEQ ID NO: 46. For example, the H-FR1 may include an amino acid sequence as set forth in SEQ ID NO: 64; the H-FR2 may include an amino acid sequence as set forth in SEQ ID NO: 66; the H-FR3 may include an amino acid sequence as set forth in SEQ ID NO: 67; the H-FR4 may include an amino acid sequence as set forth in SEQ ID NO: 70. For example, the heavy chain variable region of the targeting moiety of the chimeric antigen receptor may include an amino acid sequence as set forth in SEQ ID NO: 81. For example, the targeting moiety of the chimeric antigen receptor may include the antibody No. 15 or a targeting moiety of a chimeric antigen receptor having the same heavy chain variable region therewith.

In the present application, the targeting moiety of the chimeric antigen receptor may include a heavy chain variable region, and the heavy chain variable region may include HCDRs 1-3 and H-FRs 1-4. For example, the HCDR1 may include an amino acid sequence as set forth in SEQ ID NO: 50; the HCDR2 may include an amino acid sequence as set forth in SEQ ID NO: 51; the HCDR3 may include an amino acid sequence as set forth in SEQ ID NO: 52. For example, the H-FR1 may include an amino acid sequence as set forth in SEQ ID NO: 64; the H-FR2 may include an amino acid sequence as set forth in SEQ ID NO: 66; the H-FR3 may include an amino acid sequence as set forth in SEQ ID NO: 68; the H-FR4 may include an amino acid sequence as set forth in SEQ ID NO: 71. For example, the heavy chain variable region of the targeting moiety of the chimeric antigen receptor may include an amino acid sequence as set forth in SEQ ID NO: 82. For example, the targeting moiety of the chimeric antigen receptor may include the antibody No. 17 or a targeting moiety of a chimeric antigen receptor having the same heavy chain variable region therewith.

In the present application, the targeting moiety of the chimeric antigen receptor may include a heavy chain variable region, and the heavy chain variable region may include HCDRs 1-3 and H-FRs 1-4. For example, the HCDR1 may include an amino acid sequence as set forth in SEQ ID NO: 23; the HCDR2 may include an amino acid sequence as set forth in SEQ ID NO: 55; the HCDR3 may include an amino acid sequence as set forth in SEQ ID NO: 56. For example, the H-FR1 may include an amino acid sequence as set forth in SEQ ID NO: 61; the H-FR2 may include an amino acid sequence as set forth in SEQ ID NO: 66; the H-FR3 may include an amino acid sequence as set forth in SEQ ID NO: 67; the H-FR4 may include an amino acid sequence as set forth in SEQ ID NO: 70. For example, the heavy chain variable region of the targeting moiety of the chimeric antigen receptor may include an amino acid sequence as set forth in SEQ ID NO: 83. For example, the targeting moiety of the chimeric antigen receptor may include the antibody No. 24 or a targeting moiety of a chimeric antigen receptor having the same heavy chain variable region therewith.

In the present application, the targeting moiety of the chimeric antigen receptor may include a heavy chain variable region, and the heavy chain variable region may include HCDRs 1-3 and H-FRs 1-4. For example, the HCDR1 may include an amino acid sequence as set forth in SEQ ID NO: 37; the HCDR2 may include an amino acid sequence as set forth in SEQ ID NO: 24; the HCDR3 may include an amino acid sequence as set forth in SEQ ID NO: 30. For example, the H-FR1 may include an amino acid sequence as set forth in SEQ ID NO: 65; the H-FR2 may include an amino acid sequence as set forth in SEQ ID NO: 66; the H-FR3 may include an amino acid sequence as set forth in SEQ ID NO: 69; the H-FR4 may include an amino acid sequence as set forth in SEQ ID NO: 70. For example, the heavy chain variable region of the targeting moiety of the chimeric antigen receptor may include an amino acid sequence as set forth in SEQ ID NO: 84. For example, the targeting moiety of the chimeric antigen receptor may include the antibody No. 29 or a targeting moiety of a chimeric antigen receptor having the same heavy chain variable region therewith.

In the present application, the chimeric antigen receptor may include a hinge region. For example, the hinge region may include a hinge region derived from a protein selected from a group consisting of: IgG4, IgG1, and CD8. For example, the hinge region may include an amino acid sequence as set forth in SEQ ID NO: 147. For example, the hinge region may include an amino acid sequence having at least 80% (e.g., at least 85%, 90%, 91%, 92%, 93%, 94%, 95%, 96%, 97%, 98%, 99% or higher) sequence homology to the amino acid sequence as set forth in SEQ ID NO: 147. For example, the nucleotide sequence encoding the hinge region may include a nucleotide sequence as set forth in SEQ ID NO: 148.

In the present application, the chimeric antigen receptor may include a transmembrane domain. For example, the transmembrane domain may include but not limited to a transmembrane domain derived from a protein selected from a group consisting of: CD8, CD28, CD24, 4-1BB, CD4, CD27, CD7, PD-1, TRAC, TRBC, CD3ε, CD5, ICOS, OX40, NKG2D, 2B4, CD244, FcεRIγ, BTLA, CD30, GITR, HVEM, DAP10, CD2, NKG2C, LIGHT, DAP12, CD40L, TIM1, CD226, DR3, CD45, CD80, CD86, CD9, CD16, CD22, CD33, CD37, CD64, CD134, CD154, and SLAM, or a combination thereof. For example, the transmembrane domain may include a transmembrane domain derived from CD8. For example, the transmembrane domain may include an amino acid sequence as set forth in SEQ ID NO: 149. For example, the transmembrane domain may include an amino acid sequence having at least 80% (e.g., at least 85%, 90%, 91%, 92%, 93%, 94%, 95%, 96%, 97%, 98%, 99% or higher) sequence homology to the amino acid sequence as set forth in SEQ ID NO: 149. For example, the nucleotide sequence encoding the transmembrane domain may include a nucleotide sequence as set forth in SEQ ID NO: 150.

For example, the hinge region and the transmembrane region may include an amino acid sequence as set forth in SEQ ID NO: 128. For example, the hinge region and the transmembrane region may include an amino acid sequence having at least 80% (e.g., at least 85%, 90%, 91%, 92%, 93%, 94%, 95%, 96%, 97%, 98%, 99% or higher) sequence homology to the amino acid sequence as set forth in SEQ ID NO: 128. For example, the hinge region and the transmembrane region may include a nucleotide sequence as set forth in SEQ ID NO: 127.

In the present application, the chimeric antigen receptor may include a costimulatory signaling domain. For example, the costimulatory signaling domain may include but not limited to a costimulatory signaling domain derived from a protein selected from a group consisting of: CD28, 4-1BB, CD27, CD2, CD7, CD8, OX40, CD226, DR3, SLAM, CDS, ICAM-1, NKG2D, NKG2C, B7-H3, 2B4, FcεRIγ, BTLA, GITR, HVEM, DAP10, DAP12, CD30, CD40, CD40L, TIM1, PD-1, LFA-1, LIGHT, JAML, CD244, CD100, ICOS, a ligand of CD83, CD40, and MyD88, or a combination thereof. For example, the costimulatory signaling domain may include a costimulatory signaling domain derived from 4-1BB. For example, the costimulatory signaling domain may include an amino acid sequence as set forth in SEQ ID NO: 132. For example, the costimulatory signaling domain may include an amino acid sequence having at least 80% (e.g., at least 85%, 90%, 91%, 92%, 93%, 94%, 95%, 96%, 97%, 98%, 99% or higher) sequence homology to the amino acid sequence as set forth in SEQ ID NO: 132. For example, the costimulatory signaling domain may include a nucleotide sequence as set forth in SEQ ID NO: 131.

For example, the costimulatory signaling domain may include a costimulatory signaling domain derived from CD28. For example, the costimulatory signaling domain may include an amino acid sequence as set forth in SEQ ID NO: 130. For example, the costimulatory signaling domain may include an amino acid sequence having at least 80% (e.g., at least 85%, 90%, 91%, 92%, 93%, 94%, 95%, 96%, 97%, 98%, 99% or higher) sequence homology to the amino acid sequence as set forth in SEQ ID NO: 130. For example, the costimulatory signaling domain may include a nucleotide sequence as set forth in SEQ ID NO: 129.

In the present application, the chimeric antigen receptor may include an intracellular signaling domain. For example, the intracellular signaling domain may include an intracellular signaling domain derived from a protein selected from a group consisting of: CD3ζ, CD3δ, CD3γ, CD3ε, CD79a, CD79b, FceRIγ, FceRIβ, FcyRIIa, bovine leukemia virus gp30, Epstein-Barr virus (EBV) LMP2A, simian immunodeficiency virus PBj 14 Nef, Kaposi's sarcoma-associated herpes virus (HSKV), DAP10, and DAP-12, or a combination thereof. For example, the intracellular signaling domain may include an intracellular signaling domain derived from CD3ζ. For example, the intracellular signaling domain may include an amino acid sequence as set forth in SEQ ID NO: 134. For example, the intracellular signaling domain may include an amino acid sequence having at least 80% (e.g., at least 85%, 90%, 91%, 92%, 93%, 94%, 95%, 96%, 97%, 98%, 99% or higher) sequence homology to the amino acid sequence as set forth in SEQ ID NO: 134. For example, the intracellular signaling domain may include a nucleotide sequence as set forth in SEQ ID NO: 133.

For example, the N-terminal of the transmembrane domain may be linked to the C-terminal of the targeting moiety. For example, the C-terminal of the transmembrane domain may be linked to the N-terminal of the costimulatory signaling domain. For example, the C-terminal of the costimulatory signaling domain may be linked to the N-terminal of the intracellular signaling domain.

For example, the chimeric antigen receptor may include the following domains sequentially from N-terminal to C-terminal: a targeting moiety, a transmembrane domain, a costimulatory signaling domain, and an intracellular signaling domain.

For example, the chimeric antigen receptor may include the following domains sequentially from N-terminal to C-terminal: the antigen-binding protein (e.g., VHH) of the present application, a transmembrane domain derived from CD8, a costimulatory signaling domain derived from CD137, and an intracellular signaling domain derived from CD3zeta.

In the present application, the chimeric antigen receptor may include an amino acid sequence as set forth in any one of SEQ ID NO: 112 to SEQ ID NO: 124. For example, the chimeric antigen receptor may include an amino acid sequence having at least 80% (e.g., at least 85%, 90%, 91%, 92%, 93%, 94%, 95%, 96%, 97%, 98%, 99% or higher) sequence homology to an amino acid sequence as set forth in SEQ ID NO: 112 to SEQ ID NO: 124.

In another aspect, the present application provides a modified cell, which may include the chimeric antigen receptor above. In the present application, the modification may include upregulating the expression level of the low-density lipoprotein receptor-related protein or the fragment thereof in the cell.

### Low-density lipoprotein receptor-related protein or fragment thereof

In the present application, the low-density lipoprotein receptor-related protein may include one or more selected from a group consisting of: low-density lipoprotein receptor-related proteins 1-12 and functional fragments thereof.

In the present application, the low-density lipoprotein receptor-related protein or the fragment thereof may be derived from mammal, such as human, macaque, rats, and mouse. For example, the low-density lipoprotein receptor-related protein or the fragment thereof may be derived from human.

In the present application, the functional fragment may include a fragment or a truncated body of the low-density lipoprotein receptor-related protein having the activity of the low-density lipoprotein receptor-related protein. For example, the low-density lipoprotein receptor-related protein may include a low-density lipoprotein receptor-related protein 6 and a truncated body thereof, and/or a low-density lipoprotein receptor-related protein 5 and a truncated body thereof.

In the present application, the truncated body of the low-density lipoprotein receptor-related protein 6 may include an intracellular region of the low-density lipoprotein receptor-related protein 6; and/or, the truncated body of the low-density lipoprotein receptor-related protein 5 may include an intracellular region of the low-density lipoprotein receptor-related protein 5. In the present application, the term "intracellular region" generally refers to the domain of a protein that is located within the cell membrane. In the present application, the intracellular domain may refer to the domain within the cell membrane of the low-density lipoprotein receptor-related protein. In the present application, the intracellular region may include a sequence of positions from 24 to 243 in SEQ ID NO: 140 or an amino acid sequence having at least 80% homology therewith, or, a sequence of positions from 24 to 231 in SEQ ID NO: 144 or an amino acid sequence having at least 80% homology therewith. Further for example, the truncated body of the low-density lipoprotein receptor-related protein 6 may include a transmembrane region of the low-density lipoprotein receptor-related protein 6 and an LDLR region of the low-density lipoprotein receptor-related protein 6; and/or, the truncated body of the low-density lipoprotein receptor-related protein 5 may include a transmembrane region of the low-density lipoprotein receptor-related protein 5 and an LDLR region of the low-density lipoprotein receptor-related protein 5. In the present application, the term "LDLR region" generally refers to a domain close to the N-segment outside the transmembrane region of the low-density lipoprotein receptor-related protein. The domain may have the function of enhancing Wnt signaling. In the present application, the LDLR region may include a sequence of positions from 5 to 119 in SEQ ID NO: 138 or an amino acid sequence having at least 80% homology therewith, or, a sequence of positions from 4 to 119 in SEQ ID NO: 142 or an amino acid sequence having at least 80% homology therewith.

In the present application, the low-density lipoprotein receptor-related protein or the fragment thereof includes an amino acid sequence as set forth in any one below: SEQ ID NO: 138, SEQ ID NO: 140, SEQ ID NO: 142, and SEQ ID NO: 144, or an amino acid sequence having at least 80% homology therewith.

In the present application, the nucleic acid molecule encoding the low-density lipoprotein receptor-related protein or the fragment thereof includes a nucleic acid sequence as set forth in any one below: SEQ ID NO: 137, SEQ ID NO: 139, SEQ ID NO: 141, and SEQ ID NO: 143, or a nucleic acid sequence having at least 80% homology therewith.

In the present application, the modified cell may include immune cells (e.g., lymphocytes). In the present application, the modified cell may include modified T cells. In the present application, the modified cell may include modified stem cell-like memory T cells (TSCMs) and/or genetically modified central memory T cells (TCMs). In the present application, the TSCM may include CCR7+ and/or CD62L+. In the present application, the TSCM also includes one or more selected from a group consisting of: CD45RA+ or CD45RA-, CD45RO+ or CD45RO-, CD27+, CD28+, CD127+, CD122+, CD3+, CD4+, and CD8+.

In the present application, the modified cell can express the chimeric antigen receptor (CAR). For example, the modified cell may include a vector capable of expressing the chimeric antigen receptor (CAR). The vector may include a nucleotide molecule encoding the chimeric antigen receptor (CAR). Further for example, the vector may be selected from a group consisting of: a retroviral vector, a lentiviral vector and/or a transposon plasmid.

In the present application, the vector capable of expressing the low-density lipoprotein receptor-related protein or the fragment thereof and the vector capable of expressing the chimeric antigen receptor (CAR) may be the same vector or different vectors, as long as the one or more vectors can express the low-density lipoprotein receptor-related protein or the fragment thereof and the chimeric antigen receptor (CAR), such that the genetically modified immune cell possessing both the low-density lipoprotein receptor-related protein or the fragment thereof and the chimeric antigen receptor (CAR).

For example, the vector capable of expressing the low-density lipoprotein receptor-related protein or the fragment thereof and the vector capable of expressing the chimeric antigen receptor (CAR) may be the same vector. In such a vector, the nucleotide molecule encoding the low-density lipoprotein receptor-related protein or the fragment thereof and the nucleotide molecule encoding the chimeric antigen receptor (CAR) may be located in the same expression cassette. For example, the nucleotide molecule encoding the low-density lipoprotein receptor-related protein or the fragment thereof may be located at the 3'-end of the nucleotide molecule encoding the chimeric antigen receptor (CAR).

In the present application, the nucleotide molecule encoding the low-density lipoprotein receptor-related protein or the fragment thereof may be linked to the nucleotide molecule encoding the chimeric antigen receptor (CAR) directly or indirectly. For example, the indirect linkage may be made through a linking sequence. The 5'-end of the linking sequence may be linked to the 3'-end of the nucleotide molecule encoding the low-density lipoprotein receptor-related protein or the fragment thereof, and the 3'-end of the linking sequence may be linked to the 5'-end of the nucleotide molecule encoding the chimeric antigen receptor (CAR).

In the present application, the low-density lipoprotein receptor-related protein or the fragment thereof and the chimeric antigen receptor (CAR), both obtained by expression of the modified immune cell, may be two separate proteins. That is, the two do not have any interconnections to form any form of dimer (polymer) or protein complex. However, the low-density lipoprotein receptor-related protein or the fragment thereof and the chimeric antigen receptor (CAR), both obtained by expression of the modified immune cell, may also be linked to each other. For example, in some cases, the two proteins formed by translation are not cleaved completely, resulting in a complex is formed between the low-density lipoprotein receptor-related protein or the fragment thereof and the chimeric antigen receptor.

The chimeric antigen receptor may include a 2A sequence. The term "2A sequence" generally refers to a protease-independent, self-cleavage amino acid sequence. The 2A sequence may facilitate transcription so as to produce two proteins. In the present application, the 2A sequence may include a sequence of positions from 1 to 54 in SEQ ID NO: 136. For example, the nucleotide sequence encoding the 2A sequence may include a nucleotide sequence of SEQ ID NO: 135.

In the present application, the chimeric antigen receptor may include a leader sequence. For example, the nucleotide sequence encoding the leader sequence may include a nucleotide sequence of SEQ ID NO: 146.

### Polypeptide and Immunoconjugate

In another aspect, the present application provides one or more polypeptides which may include the isolated antigen-binding protein of the present application. For example, the polypeptide may include a fusion protein. For example, the polypeptide may include multispecific antibodies (e.g., bispecific antibodies).

In another aspect, the present application provides one or more immunoconjugates, which may include the isolated antigen-binding protein of the present application. In some embodiments, the immunoconjugate may further include a pharmaceutically acceptable therapeutic agent, a marker and/or a detecting agent.

### Nucleic acid, Vector, and Cell

In another aspect, the present application further provides one or more isolated nucleic acid molecules, which can encode the isolated antigen-binding protein or the polypeptide of the present application. For example, each of the one or more nucleic acid molecules can encode an intact antigen-binding protein or a part thereof (e.g., one or more of HCDRs 1-3 and heavy chain variable regions).

For example, when the nucleic acid molecules encode a part of the antigen-binding protein or polypeptide, respectively, the products encoded by the nucleic acid molecules can be combined together to form the isolated antigen-binding protein of the present application having a function (e.g., binding to AFP).

The nucleic acid molecules of the present application may be isolated. For example, they can be produced or synthesized by the following methods: (i) amplification *in vitro,* e.g., being produced by amplification through polymerase chain reaction (PCR), (ii) being produced by cloning and recombination, (iii) being purified, for example fractionation by enzymatic digestion and gel electrophoresis, or, (iv) being synthesized, for example through chemical synthesis. For example, the isolated nucleic acids may be nucleic acid molecules prepared by recombinant DNA technology.

In the present application, the nucleic acid encoding the isolated antigen-binding protein can be prepared by a variety of methods known in the art, including but not limited to, using reverse transcription-PCR and PCR to obtain nucleic acid molecules encoding the isolated antigen-binding protein or polypeptide of the present application.

In another aspect, the present application provides one or more vectors, which include one or more nucleic acid molecules of the present application. Each vector may include one or more of the nucleic acid molecules. In addition, the vector may further include other gene(s), e.g., a marker gene that allows the selection of the vector in an appropriate host cell and under appropriate conditions. In addition, the vector may further include an expression control element that allows the coding region to be properly expressed in an appropriate host. Such a control element is well known by persons skilled in the art. For example, it may include a promoter, a ribosome binding site, an enhancer and other control elements regulating the gene transcription or mRNA translation, and the like. In some embodiments, the expression control sequence may be an adjustable element. The specific structure of the expression control sequence may vary depending on the function of the species or cell type, but typically includes 5' non-transcriptional sequences and 5' and 3' non-translational sequences involved in the initiation of transcription and translation, respectively, such as TATA cassettes, capped sequences, CAAT sequences and the like. For example, 5' non-transcriptional expression control sequences may include a promoter region which may contain a promoter sequence for transcriptional control and functionally linked to a nucleic acid. The expression control sequence may further include an enhancer sequence or an upstream activator sequence. In the present application, suitable promoters may include, for example, promoters for SP6, T3, and T7 polymerases, human U6RNA promoters, CMV promoters, and artificial hybrid promoters thereof (e.g., CMV), where a portion of the promoter may be fused to a portion of a gene promoter of other cellular proteins (e.g., human GAPDH, glyceraldehyde-3-phosphate dehydrogenase), which may or may not contain an additional intron. One or more nucleic acid molecules of the present application may be operatively linked to the expression control element.

The vector may include, e.g., plasmid, cosmid, virus, phage, or other vectors commonly used in, e.g., genetic engineering. For example, the vector may be an expression vector. For example, the vector may be a viral vector. The viral vector can be directly given to the patient (*in vivo*) or can be given to the patient in an indirect form, e.g., by treating cells with the virus *in vitro* and then giving the treated cells to the patient (*ex vivo*). Viral vector technologies are well known in the art, and are described in, for example, Sambrook et al. (2001, Molecular Cloning: A Laboratory Manual, Cold Spring Harbor Laboratory, New York), and other virology and molecular biology manuals. Conventional virus-based systems may include retroviral vectors, lentiviral vectors, adenoviral vectors, adeno-associated viral vectors, and herpes simplex viral vectors for gene transfer. In some cases, gene transfer can be integrated into the host genome using retrovirus, lentivirus, and adeno-associated virus methods to allow long-term expression of the inserted gene. Lentiviral vectors are retroviral vectors that are capable of transducing or infecting non-dividing cells and typically producing high viral titers. Lentiviral vectors may include a long terminal repeat 5' LTR and a truncated 3'LTR, RRE, rev response element (cPPT), a central termination sequence (CTS) and/or a post-translational regulatory element (WPRE). The vector of the present application can be introduced into the cell.

In another aspect, the present application provides a cell. The cell may include the isolated antigen-binding protein of the present application, the polypeptide, the immunoconjugate, one or more nucleic acid molecules and/or the one or more vectors of the present application. For example, each kind of or each of the cells may include one or one kind of the nucleic acid molecule or the vector of the present application. For example, each kind of or each of the cells may include multiple (e.g., two or more) or multiple kinds of (e.g., two or more kinds of) the nucleic acid molecule or the vector of the present application. For example, the vector of the present application can be introduced into the host cell, e.g., prokaryotic cells (e.g., bacterial cells), CHO cells, NS/0 cells, HEK293T cells, 293F cells or HEK293A cells, or other eukaryotic cells, such as plant-derived cells, fungi or yeast cells, and the like. The vector of the present application can be introduced into the host cell by methods known in the art, such as electroporation, lipofectine transfection, lipofectamin transfection, and the like. For example, the cell may include yeast cells. For example, the cell may include *E. coli* cells. For example, the cell may include mammalian cells. For example, the cell may include immune cells.

The cell may include immune cells. In some cases, the cell may include immune cells. For example, the cell may include T cells, B cells, natural killer (NK) cells, macrophages, NKT cells, monocytes, dendritic cells, granulocytes, lymphocytes, leukocytes and/or peripheral blood mononuclear cells. For example, the cell may include T cells.

### Pharmaceutical composition

In another aspect, the present application provides a pharmaceutical composition. The pharmaceutical composition may include the isolated antigen-binding protein, the polypeptide, the immunoconjugate, the isolated nucleic acid molecule, the vector, the cell of the present application, and/or a pharmaceutically acceptable adjuvant and/or excipient. In the present application, the pharmaceutically acceptable adjuvant may include a buffer, an antioxidant, a preservative, a low molecular weight polypeptide, a protein, a hydrophilic polymer, an amino acid, a sugar, a chelating agent, a counter ion, a metal complex and/or a nonionic surfactant. Unless incompatible with the cell of the present application, any conventional media or reagents may be considered for use in the pharmaceutical composition of the present application. In the present application, the pharmaceutically acceptable excipient may include additives other than the main drug in pharmaceutical preparations, also referred to be accessories. For example, the excipient may include binder, filler, disintegrant, and lubricant in tablets. For example, the excipient may include wine, vinegar, drug juice, etc. in Chinese medicine pills. For example, the excipient may include a matrix part in a semi-solid preparation ointment or cream. For example, the excipient may include preservative, antioxidant, flavoring agent, fragrance, cosolvent, emulsifier, solubilizer, osmotic pressure regulator, and colouring agent in liquid preparations.

### Kit, use, and method

In another aspect, the present application provides a method for detecting or determining AFP, which may include using the isolated antigen-binding protein or the polypeptide.

In the present application, the method may include an in vitro method, an ex vivo method, and methods for non-diagnostic or non-therapeutic purposes.

For example, the method may include a method for detecting the presence and/or content of AFP for non-diagnostic purposes, which may include the following steps:
1) contacting a sample with the antigen-binding protein of the present application; and
2) detecting the presence and/or content of the antigen-binding protein bound by the sample to determine the presence and/or expression level of AFP in the sample obtained from the subject.

In another aspect, the present application provides a kit for detecting AFP, which may include using the isolated antigen-binding protein or the polypeptide.

In the present application, the kit may further include an instruction for illustrating the method for detecting the presence and/or content of AFP. For example, the method may include an in vitro method, an ex vivo method, and methods for non-diagnostic or non-therapeutic purposes.

In another aspect, the present application provides a use of the isolated antigen-binding protein or the polypeptide in the preparation of a kit which can be used in the method for detecting the presence and/or content of AFP. For example, the method may include an in vitro method, an ex vivo method, and methods for non-diagnostic or non-therapeutic purposes.

In another aspect, the present application provides the isolated antigen-binding protein, the polypeptide, the immunoconjugate, the isolated nucleic acid molecule, the vector, and the pharmaceutical composition, which are used for preventing, alleviating and/or treating a disease or disorder.

In another aspect, in the present application, the kit and/or the pharmaceutical composition are used for preventing, alleviating and/or treating a disease or disorder.

For example, the disease or disorder may include a tumor. For example, the tumor may include an AFP expression-associated tumor. The term "AFP expression-associated tumor" generally refers to altered expression of AFP in the tumor microenvironment or in the tumor cells compared to that in normal cells. For example, the "AFP expression-associated tumor" may be a tumor in which the expression level of AFP in the tumor microenvironment or in the tumor cells is upregulated compared to that in normal cells. The AFP protein expression-associated tumor may be an AFP-positive tumor. In an AFP-positive tumor, the expression level of AFP protein in the tumor cells or in the tumor microenvironment is about 1%, 5%, 10%, 15%, 20%, 25%, 30%, 35%, 40%, 50%, 60%, 70%, 80% or more higher than that in normal cells. For example, the tumor may include a solid tumor. For example, the tumor may include liver cancer.

In another aspect, the present application provides a use of the isolated antigen-binding protein, the polypeptide, the immunoconjugate, the isolated nucleic acid molecule, the vector, the cell and/or the pharmaceutical composition in the preparation of a drug for preventing, alleviating and/or treating a disease or disorder.

In another aspect, the present application provides a use of the pharmaceutical composition in the preparation of a drug for preventing, alleviating and/or treating a disease or disorder.

For example, the disease or disorder may include a tumor. For example, the tumor may include an AFP expression-associated tumor. The term "AFP expression-associated tumor" generally refers to altered expression of AFP in the tumor microenvironment or in tumor cells compared to that in normal cells. For example, the "AFP expression-associated tumor" may be a tumor in which the expression of AFP in the tumor microenvironment or in tumor cells is upregulated compared to that in normal cells. The AFP protein expression-associated tumor may be an AFP-positive tumor. In an AFP-positive tumor, the expression level of AFP protein in tumor cells or in the tumor microenvironment is about 1%, 5%, 10%, 15%, 20%, 25%, 30%, 35%, 40%, 50%, 60%, 70%, 80% or more higher than that in normal cells.

For example, the tumor may include a solid tumor. For example, the tumor may include liver cancer.

In another aspect, the present application provides a method for preventing and/or treating a disease or disorder, which includes administering to a subject in need thereof the isolated antigen-binding protein, the isolated nucleic acid molecule, the vector, the cell, the pharmaceutical composition.

For example, the disease or disorder may include a tumor. For example, the tumor may include an AFP expression-associated tumor. The term "AFP expression-associated tumor" generally refers to altered expression of AFP in the tumor microenvironment or in tumor cells compared to that in normal cells. For example, the "AFP expression-associated tumor" may be a tumor in which the expression of AFP in the tumor microenvironment or in tumor cells is upregulated compared to that in normal cells. The AFP protein expression-associated tumor may be an AFP-positive tumor. In an AFP-positive tumor, the expression level of AFP protein in tumor cells or in the tumor microenvironment is about 1%, 5%, 10%, 15%, 20%, 25%, 30%, 35%, 40%, 50%, 60%, 70%, 80% or more higher than that in normal cells.

For example, the tumor may include a solid tumor. For example, the tumor may include liver cancer.

The pharmaceutical composition and the method described in the present application may be used in combination with other types of cancer therapies, such as chemotherapy, surgery, radiation, gene therapy, etc. The pharmaceutical composition and the method described in the present application may be used for other disease conditions that depend on an immune response, such as inflammation, immune disorders and infectious diseases.

In the present application, the subject may include human or non-human animals. For example, the non-human animals may be selected from a group consisting of: monkey, chicken, goose, cat, dog, mouse, and rat. In addition, non-human animals may also include any animal species other than human, such as domestic animals, or rodents, or primates, or domesticated animals, or poultry animals. The human may be Caucasian, African, Asian, Semitic, or other races, or a hybrid of various races. Further for example, the human may be an older person, an adult, a teenager, a child or an infant.

Effective amounts in humans can be inferred from the effective amounts in experimental animals. For example, Freireich et al. have described the correlation of doses in animals and humans (based on milligrams per square metre of body surface) (Freireich et al., Cancer Chemother. Rep. 50, 219 (1966)). Body surface area can be approximately determined from the patient's height and weight. See, for example, Scientific Tables, Geigy Pharmaceuticals, Ardsley, N.Y., 537 (1970).

Without intending to be limited by any theory, the following examples are only to illustrate the antigen-binding protein, the chimeric antigen receptor, the preparation method, and the use of the present application, and not intended to limit the scope of the present application.

### Examples

### Example 1 Construction of Artificially Synthesized Nanobody (VHH Antibody) Library and Screening of Anti-AFP/HLA02 TCR-Like Antibodies

### 1.1 Construction of Artificially Synthesized Nanobody (VHH Antibody) Library

By aligning 296 unrepeated nucleotide sequences of nanobodies (VHH antibodies) and protein structures thereof from the PDB (Protein Data Bank) database, the mutation strategy for the CDRs of the artificially synthesized nanobody library was determined, in which CDR3 has three kinds of lengths: 14, 17 and 21 amino acids. Selecting the marketed nanobody Caplacizumab (Publication Number: NL300966I2) as a framework, the full nucleotide sequence of the nanobody Caplacizumab was synthesized. After the artificially synthesized full nucleotide sequence of Caplacizumab was cloned into the HP153 phage vector, the single-stranded DNA of the HP153 phage vector was extracted. Using the extracted single-stranded DNA as a template, the nucleotides in the CDRs of the nanobody Caplacizumab were mutated using the Kunkel Mutagenesis method, and a library of the double-stranded DNAs of the mutated nanobodies was obtained. The double-stranded DNA was electroporated into M13KO7-preinfected competent *Escherichia coli* SS320. After overnight culture, the phage supernatant was collected to finally construct an artificially synthesized nanobody library NanoOri_1.0 ( Oricell Therapeutics (Shanghai) Co., Ltd.) with a diversity of 1.44×10¹⁰, which served as a seed library for antibody sequence screening.

### 1.2 Liquid-Phase Panning of Artificially Synthesized Nanobody Library

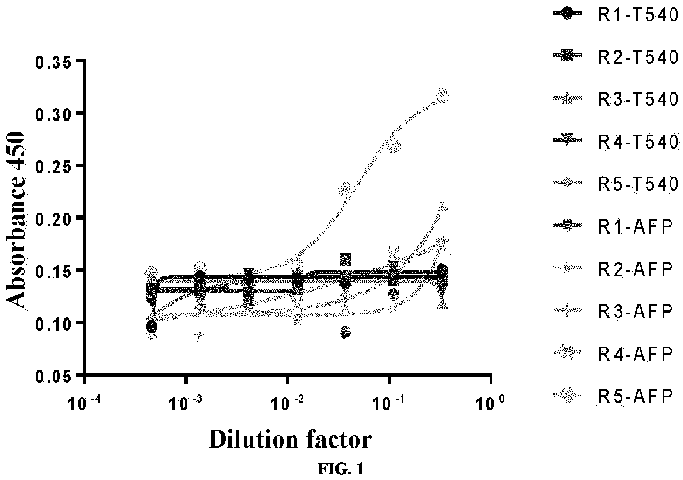

The competent *E. coli* TG1 cells were coated on an antibiotic-free bacterial culture plate. The next day, a single TG1 colony was inoculated into 15 ml of antibiotic-free 2×YT medium and cultured overnight at 37°C. 200 µl of the overnight-cultured TG1 bacterial liquid was inoculated into a new antibiotic-free 2×YT medium, and cultured at 37°C under a rotation speed of 250 rpm until OD600 reached approximately 0.8 for subsequent use. 900 µl of magnetic beads with streptavidin were evenly added into three 1.5 ml EP tubes, with 300 µl in each tube, placed on a magnetic rack and washed three times with PBS; wherein two of the EP tubes with 30 µg of a biotin-labeled human TERT540 polypeptide added respectively were designated as 540-1 and 540-2, and the third tube with 30 µg of a biotin-labeled human AFP polypeptide added was designated as hAFP, and the three 1.5 ml EP tubes were incubated at room temperature for 1 h, placed on the magnetic rack and washed three times with PBS; 700 µl of the phage library was added to the tube 540-1, incubated at room temperature for 1 h, placed on the magnetic rack and the supernatant was collected; the supernatant was added to the tube hAFP, incubated at room temperature for 1 h, placed on the magnetic rack and washed ten times with PBST (0.05% Tween); the phages were eluted with 500 µl of Gly-HCl (pH 2.2), left at room temperature for 15 min, placed on the magnetic rack and the supernatant was collected, into which was then added 250 µl of Tris-HCl (pH 8.0) for neutralization; the neutralized supernatant was added to the tube 540-2, incubated at room temperature for 1 h, placed on the magnetic rack and the supernatant was collected; and a secondary library was obtained; 20 ml of the pre-prepared TG1 bacterial liquid above (OD600=0.8) was added to the secondary library, left at 37°C for 30 min, and then 1 ml was collected for counting; 40 µl of helper phage M13K07 was added and left at 37°C for 30 min; antibiotics ampicillin and kanamycin were added, 20 ml of 2×YT medium was supplemented, and the mixture were cultured overnight at 37°C under a rotation speed of 220 rpm; the bacterial liquid grown overnight was centrifuged at 8000 rpm, the supernatant was collected, add PEG-NaCl precipitation at 1/5 of the supermatant, placed on an ice bath for 1 h, centrifuged at 8000 rpm for 30 min, and the supernatant was removed, and the precipitate was resuspended in 1 ml of PBS to obtain the first round of panning results. This phage was then used for the second round of panning. In each subsequent round of panning, the expected goal could be achieved by adjusting the screening pressure. See Table 1 for specific conditions, and five rounds of panning were completed in total. The panning results are shown in Table 1, with obvious enrichment observed starting from the third round. The ELISA results of five rounds of panning are shown in Fig. 1. The secondary antibody used was an anti-M13 phage antibody (HRP) (Manufacturer: Sino Biological).

**Table 1 Panning Conditions and Results of Nanobodies**

| | Antigen binding amount (µg) | Binding time (min) | Washing times | Counts |
|---|---|---|---|---|
| Nano-library R1 | 30 | 60 | 20 | 1.6×10⁷ |
| Nano-library R2 | 20 | 60 | 20 | 6×10⁷ |
| Nano-library R3 | 15 | 30 | 20 | 6×10⁹ |
| Nano-library R4 | 10 | 10 | 30 | 1.2×10¹⁰ |
| Nano-library R5 | 5 | 5 | 30 | 4×10⁹ |

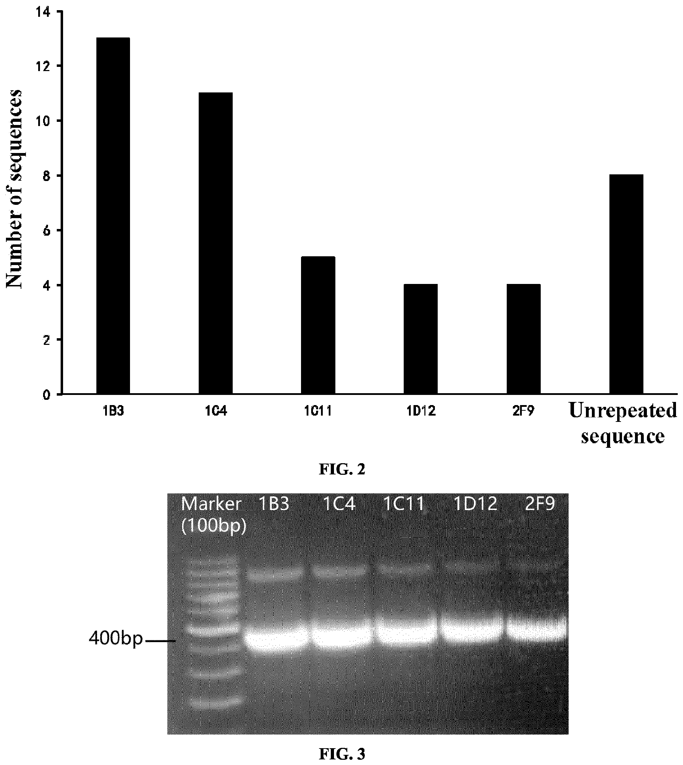

Single clones were selected for sequencing identification and analysis; sequence alignment analysis was performed using the software BioEdit, and the statistical results are shown in Fig. 2. Among the picked single clones, there are a total of 13 repeated nucleotide sequences of the VHH antibody 1B3 (with the nucleotide sequence as set forth in SEQ ID NO: 1), 11 repeated nucleotide sequences of the VHH antibody 1C4 (with the nucleotide sequence as set forth in SEQ ID NO: 2), 5 repeated nucleotide sequences of the VHH antibody 1C11 (with the nucleotide sequence as set forth in SEQ ID NO: 3), 4 repeated nucleotide sequences of the VHH antibody 1D12 (with the nucleotide sequence as set forth in SEQ ID NO: 4), and 4 repeated nucleotide sequences of the VHH antibody 2F9 (with the nucleotide sequence as set forth in SEQ ID NO: 5). There are 8 unrepeated single nucleotide sequences of VHH antibodies.

### Example 2 Expression and Characterization of Candidate VHH-Fc Antibodies

### 2.1 Construction of Eukaryotic Expression Vectors for Candidate VHH-Fc Antibodies and Purification of Antibodies

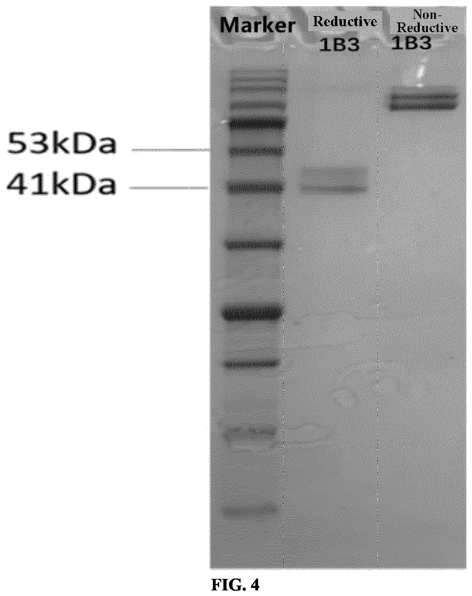

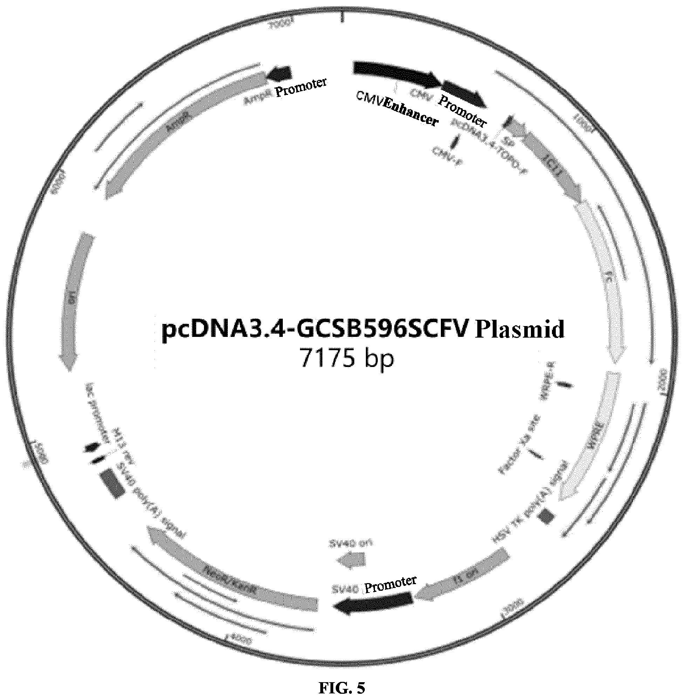

Primers were designed to perform PCR amplification on the nucleotide sequences of the candidate VHH antibodies obtained in Example 1 (with the nucleotide sequences as set forth in any one of SEQ ID NO: 1 to SEQ ID NO: 13). The products were cloned respectively through homologous recombination into an ampicillin-resistant pcDNA3.4 vector that had been double-digested with the restriction endonucleases SfiI and NotI, with the 3' end of the nucleotide sequence of the VHH antibody linked to the 5' end of the Fc portion of the antibody (SEQ ID NO: 125), thereby constructing a eukaryotic expression vector for the candidate VHH-Fc antibodies. The map of the constructed vector is shown in Fig. 5. The correctly sequenced recombinant plasmids were used to transfect Expi293F cells for transient expression, and purified via a Protein A column to obtain different candidate VHH-Fc antibodies. The PCR results are shown in Fig. 3, displaying from left to right successively: the bands of 100 bp marker, 1B3 (the nucleotide of the VHH antibody 1B3 ), 1C4 (the nucleotide of the VHH antibody 1C4), 1C11 (the nucleotide of the VHH antibody 1C11), 1D12 (the nucleotide of the VHH antibody 1D12), and 2F9 (the nucleotide of the VHH antibody 2F9) (about 400 bp). The electrophoretogram of the purified proteins is shown in Fig. 4, displaying from left to right successively: Marker, reductive 1B3 (a VHH-Fc antibody containing the nucleotide sequence of the VHH antibody 1B3), and non-reductive 1B3, with the purified 1B3 having a correct molecular weight (approximately 41kDa after reduction).

### 2.2 Identification of Affinity of Candidate VHH-Fc Antibodies

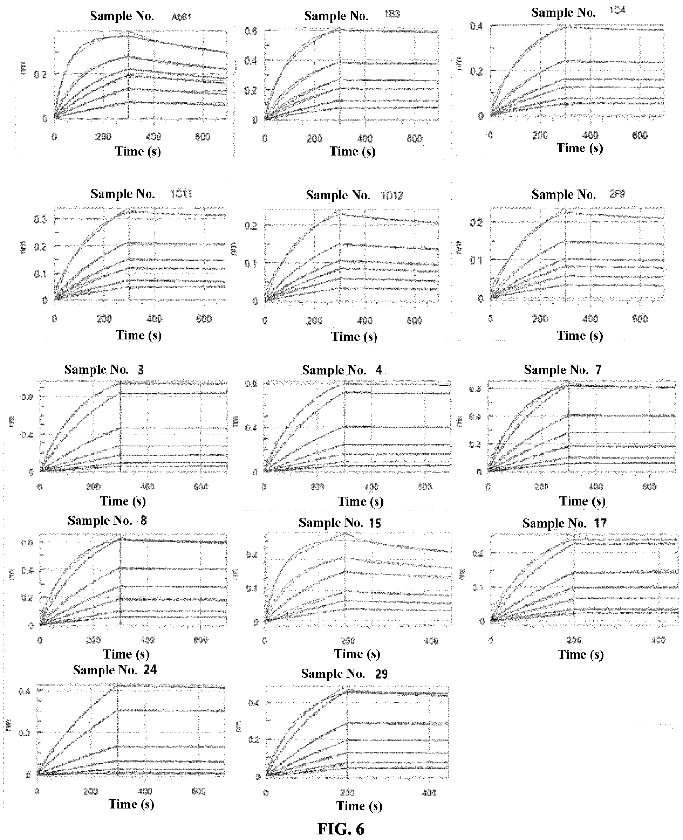

The ForteBio AHC sensor was first bound with 100 nM of the candidate VHH-Fc antibodies, and then bound with either the human AFP158-166/HLA-A02*01* or human TERT540/HLA-A02*01* complex, respectively, starting at 150 nM with 2-fold serial dilutions to obtain 7 concentration gradients. The association phase lasted 5 min and the dissociation phase 10 min. Affinity data were analyzed using Data Analysis 9.0, with the data results shown in Table 2, and the affinity results are shown in Fig. 6. Wherein, two antibodies, No.17 (a VHH-Fc antibody containing the nucleotide sequence of the VHH antibody No.17, with the nucleotide sequence of the antibody as set forth in SEQ ID NO: 11) and No.24 (a VHH-Fc antibody containing the nucleotide sequence of the VHH antibody No.24, with the nucleotide sequence of the antibody as set forth in SEQ ID NO: 12), hardly undergo dissociation. No.3 (a VHH-Fc antibody containing the nucleotide sequence of the VHH antibody No.3, with the nucleotide sequence of the antibody as set forth in SEQ ID NO: 6) demonstrates approximately 9-fold higher affinity compared to the positive control antibody Ab61 from Eureka Therapeutics (with the amino acid sequence as set forth in SEQ ID NO: 86, the nucleotide sequence as set forth in SEQ ID NO: 85, the Fc amino acid sequence as set forth in SEQ ID NO: 88, and the Fc nucleotide sequence as set forth in SEQ ID NO: 87). No.4 (a VHH-Fc antibody containing the nucleotide sequence of the VHH antibody No.4, with the nucleotide sequence of the antibody as set forth in SEQ ID NO: 7), No.29 (a VHH-Fc antibody containing the nucleotide sequence of the VHH antibody No.29, with the nucleotide sequence of the antibody as set forth in SEQ ID NO: 13), 1B3 (a VHH-Fc antibody containing the nucleotide sequence of the VHH antibody 1B3, with the nucleotide sequence of the antibody as set forth in SEQ ID NO: 1), and 1C4 (a VHH-Fc antibody containing the nucleotide sequence of the VHH antibody 1C4, with the nucleotide sequence of the antibody as set forth in SEQ ID NO: 2) also demonstrate better affinity than the positive control antibody Ab61 from Eureka Therapeutics.

**Table 2 Affinity Detection Results of Nanobodies**

| Sample No. | KD (M) | kon (1/Ms) | kdis (1/s) | Sample No. | KD (M) | kon (1/Ms) | kdis (1/s) |
|---|---|---|---|---|---|---|---|
| Ab61 (SEQ ID NO: 86) | 2.34E-09 | 2.38E+05 | 5.58E-04 | No.3 (SEQ ID NO: 77) | 2.59E-10 | 4.42E+04 | 1.15E-05 |
| 1 C4 (SEQ ID NO: 73) | 8.65E-10 | 1.01E+05 | 8.72E-05 | No.4 (SEQ ID NO: 78) | 7.83E-10 | 4.32E+04 | 3.38E-05 |
| 1B3 (SEQ ID NO: 72) | 5.04E-10 | 1.39E+05 | 7.01E-05 | No.7 (SEQ ID NO: 79) | 1.16E-09 | 5.34E+04 | 6.17E-05 |
| 1D12 (SEQ ID NO: 75) | 3.02E-09 | 9.07E+04 | 2.74E-04 | No.8 (SEQ ID NO: 80) | 1.43E-09 | 6.53E+04 | 9.33E-05 |
| 1C11 (SEQ ID NO: 74) | 1.21E-09 | 9.94E+04 | 1.20E-04 | No.17 (SEQ ID NO: 82) | <1.0E-12 | 6.75E+04 | <1.0E-07 |
| 2F9 (SEQ ID NO: 76) | 2.06E-09 | 8.75E+04 | 1.80E-04 | No.24 (SEQ ID NO: 83) | <1.0E-12 | 4.28E+04 | <1.0E-07 |
| | | | | No.29 (SEQ ID NO: 84) | 8.11E-10 | 7.59E+04 | 6.16E-05 |
| | | | | No.15 (SEQ ID NO: 81) | 2.00E-09 | 2.06E+05 | 4.12E-04 |

### 2.3 Identification of Non-Specific Binding Activity of Candidate VHH-Fc Antibodies to Different Human Endogenous Polypeptides

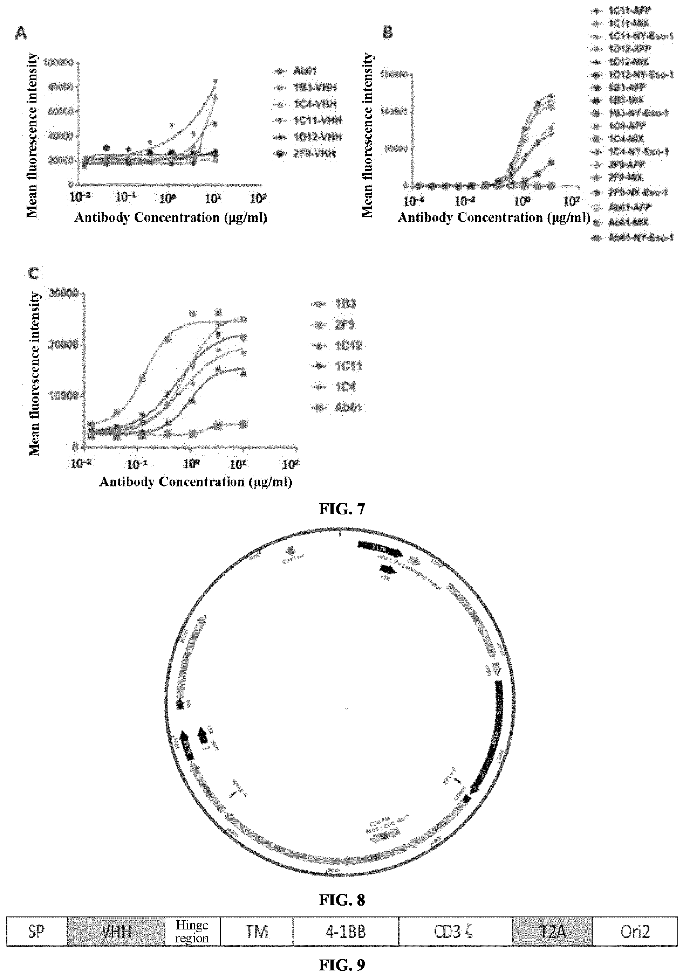

The binding activities of the antibodies were detected using the iQue Screener flow cytometer. Specifically, T2 cells, which were HLA-A02*01 * positive cells, were loaded respectively with 20 human endogenous polypeptides, such as the human AFP₁₅₈ polypeptide (with the amino acid sequence as set forth in SEQ ID NO: 90, and the nucleic acid sequence as set forth in SEQ ID NO: 89), the human NY-Eso-1 157 polypeptide (with the amino acid sequence as set forth in SEQ ID NO: 91), as well as IFI30 (with the amino acid sequence as set forth in any one of SEQ ID NO: 92-95), BTG2 (with the amino acid sequence as set forth in SEQ ID NO: 96), BCR (with the amino acid sequence as set forth in SEQ ID NO: 97), SSR1 (with the amino acid sequence as set forth in any one of SEQ ID NO: 98-99), PPP2R1B (with the amino acid sequence as set forth in SEQ ID NO: 100), DDX5 (with the amino acid sequence as set forth in SEQ ID NO: 101), CTSG (with the amino acid sequence as set forth in SEQ ID NO: 102), CD247 (with the amino acid sequence as set forth in SEQ ID NO: 103), DMTN (with the amino acid sequence as set forth in SEQ ID NO: 104), CALR (with the amino acid sequence as set forth in SEQ ID NO: 105), PIM1 (with the amino acid sequence as set forth in SEQ ID NO: 106), HLA-E (with the amino acid sequence as set forth in SEQ ID NO: 107), RPS6KB1 (with the amino acid sequence as set forth in SEQ ID NO: 108), CSF2RA (with the amino acid sequence as set forth in SEQ ID NO: 109), IL7 (with the amino acid sequence as set forth in SEQ ID NO: 110), and the human TERT540 polypeptide (with the amino acid sequence as set forth in SEQ ID NO: 111). These endogenous peptides are typically expressed in proteins of various types of nucleated human cells, such as hemoglobin α-chains, β-chains, nucleoprotein p68 and its analogs. Human AFP158/T2 was positively binding to cells, while human TERT540/T2, human NY-Eso-1 157/T2, and the other 19 human endogenous mixed polypeptides/T2 were non-specific control cells. Cells were formulated with a buffer solution to a concentration of 1×10⁶ cells/ml, and added to a 96-well V-bottom plate with 30 µl per well so as to achieve 3×10⁴ cells per well; detection antibodies were formulated with a buffer solution, starting at 10 µg/ml with 3-fold serial dilutions to set 7 concentration gradients, detected with a flow cytometer, so as to further identify the specific binding activity of the candidate VHH antibodies. The results are shown in Fig. 7A. 1D12 (a VHH-Fc antibody containing the nucleotide sequence of the VHH antibody 1D12), 2F9 (a VHH-Fc antibody containing the nucleotide sequence of the VHH antibody 2F9), and 1B3 (a VHH-Fc antibody containing the nucleotide sequence of the VHH antibody 1B3) exhibit no non-specific binding activity to human TERT540/T2 cells. While 1C11 (a VHH-Fc antibody containing the nucleotide sequence of the VHH antibody 1C11), 1C4 (a VHH-Fc antibody containing the nucleotide sequence of the VHH antibody 1C4), and the positive control antibody Ab61 from Eureka Therapeutics all exhibit non-specific binding activity to human TERT540/T2 cells at a high concentration of 10 µg/ml. As shown in Fig. 7B, 1C11, 1C4, 2F9, 1D12, and 1B3 can all bind with human AFP158/T2 cells, and all of the five candidate VHH-Fc antibodies exhibit no non-specific binding activity to the human NY-Eso-1 157/T2 control polypeptide or the human endogenous mixed polypeptides/T2.

### 2.4 Identification of cross-reactivity of candidate VHH-Fc antibodies with mouse AFP158 peptide (with amino acid sequence as set forth in SEQ ID NO: 126)

The cross-reactivity with the mouse AFP158 peptide/HLA-A*02:01 complex on the surface of live cells was further identified. The mouse AFP158 peptide differs from the human AFP158 peptide at two amino acid positions, namely position 4 and position 9. Antibodies that cross-react with both the human AFP158 peptide/MHC complex and the mouse AFP158 peptide/MHC complex are suitable for assessing antibody drug toxicity in HLA-A02*01* transgenic mice. The results are shown in Fig. 7C. All of the five candidate VHH-Fc antibodies (VHH-Fc antibodies containing the nucleotide sequences of VHH antibodies 1C11, 1C4, 2F9, 1B3, and 1D12) can bind with mouse AFP158/T2 cells, while the positive control antibody Ab61 from Eureka Therapeutics exhibits weaker binding activity.

### Example 3 Construction of CAR Core Plasmids Containing Nucleotide Sequences of Candidate VHH Antibodies, Lentivirus Packaging and Preparation of CAR-T cells

### 3.1 Construction of CAR Core Plasmids Containing Nucleotide Sequences of VHH Antibodies

The core plasmids were double-digested with the restriction endonucleases SphI and NotI, and mixed with the nucleotide sequences of the candidate VHH antibodies at a ratio of 1:3 (molar ratio), respectively for homologous recombination. The nucleotide sequence of the homologously recombined core plasmid was verified by sequencing. The amplified plasmid was stored for later use. The plasmid map is shown in Fig. 8, and the structure of the nucleotide sequence of the CAR core plasmid is shown in Fig. 9, with 41BB and CD3ζ being selected as the co-stimulatory domains.

### 3.2 Lentiviral Packaging and Titer Determination

The vector system for constructing the lentiviral plasmid of the present application belongs to the third generation of lentiviral vector system, which includes a total of three plasmids: 1: the packaging plasmid psPAX2 for encoding Gag-Pol protein and Rev protein; 2: the PMD2.G plasmid for encoding the envelope protein VSV-G; and 3: the CAR core plasmid constructed in 3.1 containing the nucleotide sequence encoding the VHH antibody. The expression of the CAR-encoding gene in the core plasmid of the plasmids based on the BBz platform was regulated by the elongation factor-1α (EF-1α) promoter. The lentivirus was packaged by the following process: 1×10⁶ 293T cells were suspended in 2 ml of DMEM medium containing 10% FBS, and plated in individual wells of a 6-well plate and incubated overnight. 144 µl of the medium (50 times the mass of the plasmid) was aspirated, and 144 µl of Opti-MEM medium containing 2.88 µg packaging plasmid (psPAX2:PMD2.G:core plasmid = 3:2:4) and 8.64 µl of FuGENEHD transfection reagent was added, and gently mixed, and then incubated in a CO₂ incubator at 37°C for 12 h. The medium containing the plasmid was removed, and after washing the plate once with PBS, the medium was replaced with 2 ml of DMEM medium containing 5% FBS and the plate was incubated for 48 h. 2.5 ml of viral supernatant was collected, which was centrifuged at 3000 rpm for 5 min, and then dispensed for freezing and storage at -80°C for later use, and tested for the titer.

### 3.3 Preparation of CAR-T Cells Containing VHH Antibodies

The CAR-T cells containing a VHH antibody were prepared as follows: Human peripheral blood mononuclear cells were obtained by density gradient centrifugation; the peripheral blood mononuclear cells were resuspended in a medium containing 200 U/ml interleukin 2 to a cell density of 2×10⁶/ml, and CD3/CD28 magnetic beads were added at a ratio of 1:3 (cells: magnetic beads) to activate T-cells. The activated peripheral blood mononuclear cells were incubated in a CO₂ incubator at 37°C for 24 h; the lentiviral supernatant obtained above was added at a ratio of the multiplicity of infection (MOI) of 3, and Polybrene was added to a final concentration of 5 µg/ml, and the cell suspension was placed in a well plate and centrifuged at 1200 rpm in a horizontal centrifuge for 1 h. The well plate was returned to the CO₂ incubator at 37°C and incubated for 24 h, then centrifuged at 300 g for 5 min. The supernatant was removed and the cells were resuspended with fresh X-VIVO medium containing 500 U/ml of interleukin 2 (Manufacturer: LONZA) to a cell density of 0.6× 10⁶/ml, and incubated in a CO₂ incubator at 37°C. The cells were counted every 2 days, and the medium was replenished with fresh X-VIVO medium containing 500 U/ml interleukin 2 (Manufacturer: LONZA) to adjust the cell density back to 0.6×10⁶/ml. The CAR-T cells that have been cultured for 9-14 days were tested for the positive rate of cells: the lentivirus used to infect the cells carried GFP, and the GFP positive rate was detected by flow cytometry after the lentivirus infected the cells to obtain the positive rate of CAR expression, and the cells with a positive rate of > 20% could be used for tumor killing assay.

### Example 4: Construction and Detection of Overexpression Cell Lines HepG2-MiniG Cells and SK-HEP-1-MiniG Cells

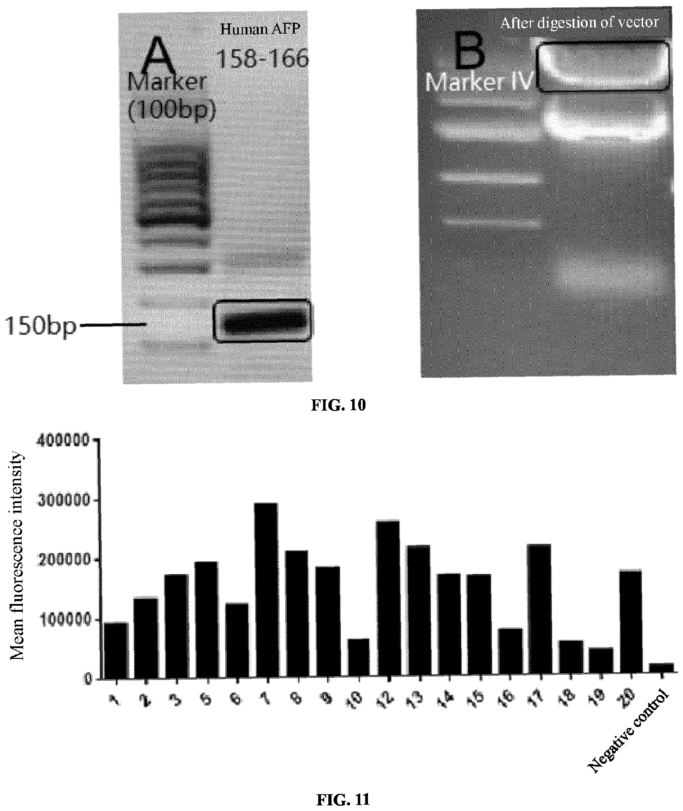

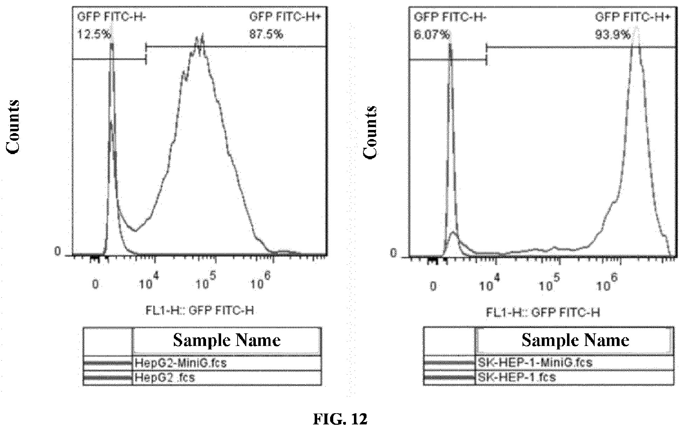

The HepG2-MiniG and SK-HEP-1-MiniG cells were prepared by introducing the human AFP₁₅₈₋₁₆₆ gene (SEQ ID NO: 89) into HepG2-MiniG and SK-HEP-1 cells, respectively, by means of lentiviral packaging and lentiviral infection. Taking the construction of SK-HEP-1-MiniG cells as an example, the recombinant plasmid pLV-C-GFPS-AFP-short (SEQ ID NO: ) was constructed by inserting the nucleotide sequence of human AFP158-166 into pLV-C-GFPS as a vector. The results of PCR and enzyme digestion are shown in FIG. 10. The PCR amplification results of the nucleotide sequence of human AFP158-166 are shown as follows: (A): from left to right successively, the bands of 100 bp Marker, and human AFP158-166 (approximately 150 bp); the results of vector digestion are shown as follows: (B): successively, the band of Marker IV and the band after digestion of the vector (about 7500 bp); after lentiviral infection, the recombinant plasmid pLV-C-GFPS-AFP-short was transformed into SK-HEP-1 cells, and single-clone SK-HEP-1-MiniG cells were selected for binding activity detection using flow cytometry, with the specific results shown in Fig. 11; the single-clone SK-HEP-1-MiniG-7 (single-clone No. 7 of SK-HEP-1-MiniG cells) exhibits the highest mean fluorescence intensity, and was expanded to be target cells, with the construction method for HepG2-MiniG cells the same as that for SK-HEP-1-MiniG cells. The positive rates of single clones of HepG2-MiniG cells and SK-HEP-1-MiniG cells after expanded culture were detected by flow cytometry, with the results shown in FIG. 12, showing that the positive rate of HepG2-MiniG cells was 87.5%, and the positive rate of SK-HEP-1-MiniG cells was 93.9%.

### Example 5: In Vitro Killing and Cytokine Detection of CAR-T Cells Containing Candidate VHH Antibodies

### 5.1 Repeated In Vitro Stimulation Experiment of CAR-T Cells Containing Candidate VHH Antibodies

CAR-T cells were prepared according to Example 3, and the positive rate was detected on days 9-12 of amplification. CAR-T cells were resuspended in a serum-free X-VIVO medium (Manufacturer: LONZA) to a density of 4×10⁵/ml, as effector cells; and HepG2-MiniG cells prepared in Example 4 were resuspended in the serum-free X-VIVO medium (Manufacturer: LONZA) to a density of 4×10⁵/ml, as target cells. The effector cells were mixed with the target cells at an effector-to-target ratio of 1:1 and left for culture in a 5% CO₂ incubator at 37°C. The color of the medium was observed every 2 days, and a one-fold volume of the medium was replenished when the medium changed from orange to yellow. The cells were counted on days 4 to 5 to calculate the amplification fold. After counting, 5×10⁵ CAR-T cells were collected to be subjected to the second, third and fourth rounds of amplification in the same way as described above. The total cumulative amplification fold was calculated by multiplying the amplification fold of the first round by that of the second, third and fourth rounds.

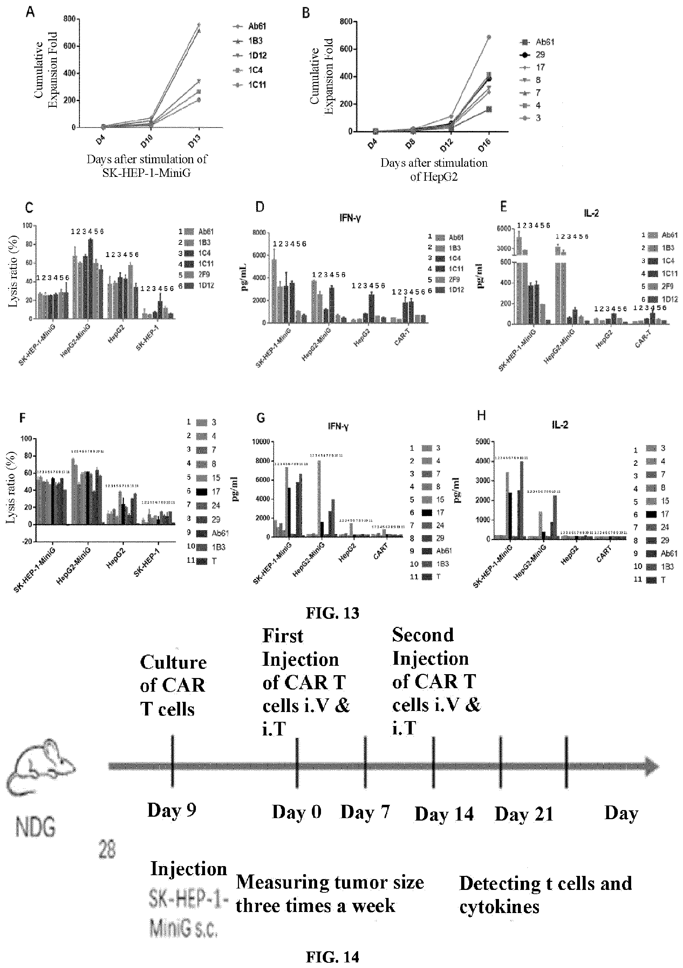

The repeated stimulation results are shown in FIG. 13A, showing that after three rounds of repeated stimulation of the CAR-T cell 1B3, the cumulative amplification fold is comparable to that of the CAR-T cell Ab61. As shown in Fig. 13B, after three rounds of repeated stimulation of all of the CAR-T cells No.3, No.4, No.7, No.8, No.17 and No.29 containing the candidate VHH antibodies, the cumulative amplification folds exceed that of the CAR-T cell Ab61.

### 5.2 In Vitro Killing Experiment of CAR-T Cells Containing Candidate VHH Antibodies

SK-HEP-1-MiniG cells prepared in Example 4 were used as target cells, CAR-T cells containing the candidate VHH antibodies prepared in Example 3 were used as effector cells, and T cells not infected with lentivirus could be used as control effector cells. The specific experiment process was as follows: the infection efficiency of the packaged CAR core plasmid-lentivirus prepared in Example 3 was detected, and the infection ratio was adjusted to the same level in each group with T cells not infected with lentivirus; effector cells and target cells were added into 200 µl of the X-VIVO medium according to effector cells: target cells (effector-target ratio) = 1:1, with the number of target cells of 1×10⁴/well, and this was used as an experiment group; the wells containing only an equal amount of effector cells as the experimental group were used as effector cell self-release background group; the wells containing only an equal amount of target cells as the experimental group were used as the target cell self-release background group; the obtained cells were cultured in a CO₂ incubator at 37°C for 18 h; 20 µl of 10× lysate was added to some of the wells containing only the target cells, and the reaction was carried out for 45 min as the maximum release of target cells. The resulting cell culture well plates were centrifuged at 300 g for 5 min, respectively. 50 µl of supernatant was collected to detect the release of lactate dehydrogenase (LDH) following the instructions of CytoTox96 nonradioactive cytotoxicity kit (Manufacturer: Promega). The released LDH was in the supernatant of the medium and can be detected by a coupled enzymatic reaction. The cell killing activity was calculated following the formula below: Killing toxicity% = 100 × (Experimental group - Self-release of effector cells - Self-release of target cells + Background value of culture medium) / (Maximum release of target cells - Self-release of target cells).

According to the cell killing formula, the killing effects of CAR-T cells containing the candidate VHH antibodies were analyzed, and the candidate VHH antibodies with obvious killing effects were selected for in vivo functional evaluation.

The killing results are shown in Fig. 13C. The results show that there is little difference in the killing effect of the five candidate VHH antibodies against HLA-A02*01 *+/AFP+ positive cells, i.e., SK-HEP-1-MiniG cells. In the cell killing results using HepG2-MiniG cells as target cells, CAR-T cells containing the candidate VHH antibody 1C11 exhibit a better killing effect against target cells than CAR-T cells containing the positive control antibody Ab61. While CAR-T cells containing the candidate VHH antibody 2F9 exhibit a better killing effect against HLA-A02*01*+/AFP+ positive cells HepG2 at a low-expressing level than CAR-T cells containing the positive control antibody Ab61. As shown in Fig. 13F, the results show that all of the CAR-T cells containing the candidate VHH antibodies 3, 4, 7, 8, 15, 17, 24, and 29 exhibit a comparable killing effect against HLA-A02*01*+/AFP+ positive cells, i.e., SK-HEP-1-MiniG cells, to CAR-T cells containing the positive control antibody Ab61. While in the cell killing results using HepG2-MiniG cells as target cells, CAR-T cells containing the candidate VHH antibodies 3 and 4 exhibit a significantly better killing effect than CAR-T cells containing the positive control antibody Ab61.

### 5.3 In Vitro Cytokine Secretion Experiment of CAR-T Cells Containing Candidate VHH Antibodies

HepG2-MiniG or SK-HEP-1-MiniG cells prepared in Example 4 were used as target cells, CAR-T cells containing the candidate VHH antibodies prepared in Example 3 were used as effector cells, and T cells not infected with lentivirus could be used as control effector cells. The specific experiment process was as follows: the infection efficiency of the packaged CAR core plasmid-lentivirus prepared in Example 3 was detected, and the ratio was adjusted to the same level in each group with T cells not infected with the virus; effector cells and target cells were added into 200 µl of the X-VIVO medium according to effector cells: target cells (effector-to-target ratio) = 1:1, with the number of target cells of 2×10⁴/well, and this was used as an experiment group; the wells containing only an equal amount of effector cells as an experiment group were used as a background group; the obtained cells were cultured in a CO₂ incubator at 37°C for 18 h; the resulting cell culture well plates were centrifuged at 300 g for 5 min, respectively, and 50 µl of the supernatant was collected to detect the expression level of IL2 and IFN-γ following the instructions of R&D DuoSet ELISA kit (Manufacturer: R&D SYSTEM).

The detection results are shown in Fig. 13D, showing that when the target cells are HepG2-MiniG or SK-HEP-1-MiniG, the CAR-T cells containing the candidate VHH antibodies 1B3, 1C4, and 1C11 secret a higher amount of the cytokine IFN-y; as shown in Fig. 13E, all of the CAR-T cells containing the candidate VHH antibodies exhibit a lower background level of the cytokine IL2 against HepG2-MiniG cells and T cells not infected with the virus. As shown in Fig. 13G, when the target cells are SK-HEP-1-MiniG, CAR-T cells containing the candidate VHH antibodies 3, 4, 7, 8, 15, 17, and 1B3 exhibit a better release of the cytokine IFN-γ. As shown in Fig. 13H, when the target cells are HepG2-MiniG cells or SK-HEP-1-MiniG cells, the CAR-T cells containing the candidate VHH antibodies 15, 17, and 1B3 exhibit a better secretion of the cytokine IL2, and all of the CAR-T cells containing the candidate VHH antibodies exhibit a lower background level of IL2 against HepG2-MiniG cells and T cells not infected with the virus.

### Example 6 In Vivo Efficacy Verification of CAR-T Cells Containing VHH Antibodies

CAR-T cells containing the candidate VHH antibody 17 and CAR-T cells containing the candidate VHH antibody 1B3 were randomly selected for in vivo efficacy evaluation. Six mice were each injected with 6×10⁶ K-HEP-1-MiniG cells to establish subcutaneous tumors at the middle of the dorsal region of the right upper limb. When the tumors reached 100 mm³, NDG mice were divided into 7 groups respectively, and each group was administered by intratumor injection or via tail vein: one group injected with T cells not infected with the virus (control group I.V), two groups injected with CAR-T cells containing the positive control antibody Ab61 (intratumor injection group Ab61-I.T and tail vein injection group Ab61-I.V), two groups injected with CAR-T cells containing the candidate VHH antibody 1B3 (intratumor injection group 1B3-I.T and tail vein injection group 1B3-I.V), and two groups injected with CAR-T cells containing the candidate VHH antibody 17 (intratumor injection group 17-I.T and tail vein injection group 17-I.V), with five mice per group, and 1×10⁷ respective CAR-T cells were reinfused, respectively. The respective CAR-T cells were reinfused every 2 weeks, and the body weight and tumor size changes of mice in each group after administration were recorded and their various biological responses were observed.

In the case of PG (D52), the tumor volumes and tumor inhibition ratios in each group are shown in Table 3, and the average tumor volume in the group of T cells not infected with the virus is 973.98±136.05 mm³; when compared with the group of T cells not infected with the virus (control group I.V), the average tumor volumes of mice in the intratumor injection group injected with CAR-T cells containing the positive control antibody Ab61 (Ab61-I.T), the intratumor injection group injected with CAR-T cells containing the candidate VHH antibody 1B3 (1B3-I.T), the intratumor injection group injected with CAR-T cells containing the candidate VHH antibody 17 (17-I.T), the tail vein injection group injected with CAR-T cells containing the positive control antibody Ab61 (Ab61-I.V), the tail vein injection group injected with CAR-T cells containing the candidate VHH antibody 1B3 (1B3-I.V), and the tail vein injection group injected with CAR-T cells containing the candidate VHH antibody 17 (17-I.V) are 2.73±1.71 mm³, 3.38±2.11 mm³, 9.58±1.12 mm³, 19.72±2.08 mm³, 24.8±1.08 mm³, and 38.32±1.33 mm³, respectively; compared with the group of T cells not infected with the virus (control group I.V), there are significant differences in all of these values (P < 0.0001); the tumor volume inhibition ratios are 109.68%, 109.57%, 108.88%, 107.75%, 107.19%, and 105.67%, respectively.

**Table 3 Statistics of Tumor Volume and Inhibition Ratio in Animal Experiments of PG (D52) Mouse**

| Group | Tumor volume (mm³) | Tumor inhibition ratio (TGI%) | Group | Tumor volume (mm³) | Tumor inhibition ratio (TGI%) |
|---|---|---|---|---|---|
| | | | Control group I.V | 973.98±136.05 | |
| Ab61-I.T | 2.73±1.71 | 109.68% | Ab61-I.V | 19.72±2.08 | 107.75% |
| 1B3-I.T | 3.38±2.11 | 109.57% | 1B3-I.V | 24.8±1.08 | 107.19% |
| 17-I.T | 9.58±1.12 | 108.88% | 17-I.V | 38.32±1.33 | 105.67% |

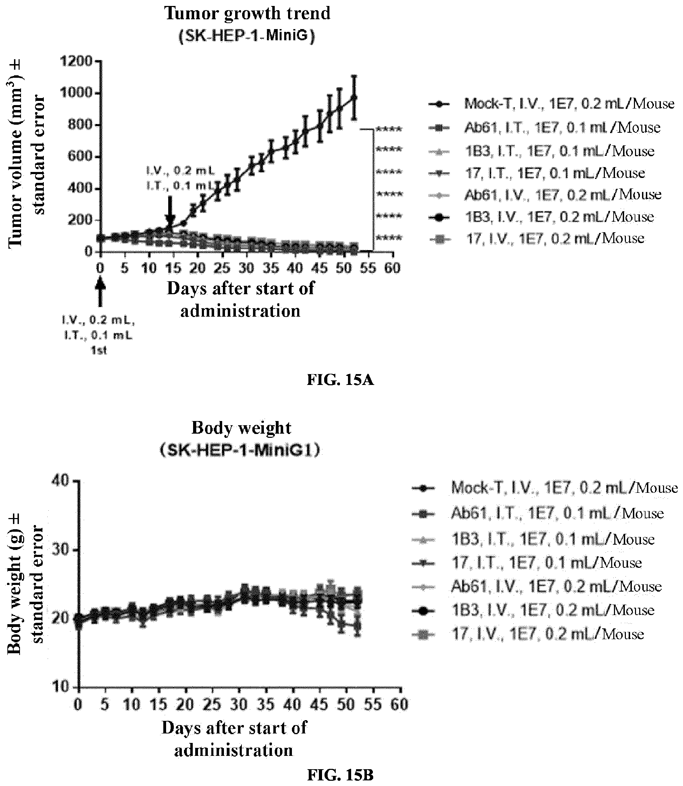

The animal experiment procedure is shown in Fig. 14. The results of the tumor growth curve in Fig. 15A show that the tumors in each group of mice begin to regress continuously from the 14th day after the first dose of respective CAR-T cells are reinfused. In each experiment group, there are significant differences (0.0001<P<0.001) in the average tumor volume of mice compared to the control group, and the tumor volume inhibition ratios (TGI) all exceed 100%. The tumor inhibition effect of the intratumor injection reinfusion group is significantly better than that of the tail vein injection reinfusion group, but with no statistical difference. Moreover, the tumor inhibition effects of the CAR-T cell groups containing the candidate VHH antibodies 1B3 and 17 are comparable to those of the positive control group, with no statistical difference. The animal body weight curve in Fig. 15B shows that, compared with the positive control group, one mouse each in the Ab61-I.T group and the 1B3-I.T group experiences a weight loss of over 20%. In accordance with animal welfare guidelines, these mice were euthanized and their vital organs were observed upon autopsy, with no abnormalities found. The body weights of mice in other experiment groups are all relatively stable. The experiment results indicate that the CAR-T cells of the present application are safe and effective for animal models with SK-HEP-1-MiniG cell-bearing tumors.

## Claims

1. An isolated antigen-binding protein, having one or more of the following properties:
1) specifically binding to a human AFP158-166/HLA-A02*01* complex with a KD value of about 3.1E-09M or less;
2) capable of binding to a mouse AFP 158//HLA-A02*01* complex.

2. The isolated antigen-binding protein of claim 1, comprising an HCDR3, wherein said HCDR3 comprises an amino acid sequence as set forth in any one of SEQ ID NO: 19, SEQ ID NO: 25, SEQ ID NO: 30, SEQ ID NO: 35, SEQ ID NO: 46, SEQ ID NO: 52, and SEQ ID NO: 56.

3. The isolated antigen-binding protein of any one of claims 1-2, comprising an HCDR2, wherein said HCDR2 comprises an amino acid sequence as set forth in any one of SEQ ID NO: 18, SEQ ID NO: 24, SEQ ID NO: 34, SEQ ID NO: 45, SEQ ID NO: 51, and SEQ ID NO: 55.

4. The isolated antigen-binding protein of any one of claims 1-3, comprising an HCDR1, wherein said HCDR1 comprises an amino acid sequence as set forth in any one of SEQ ID NO: 17, SEQ ID NO: 23, SEQ ID NO: 29, SEQ ID NO: 37, SEQ ID NO: 39, SEQ ID NO: 44, and SEQ ID NO: 50.

5. The isolated antigen-binding protein of any one of claims 1-4, comprising an HCDR1, an HCDR2, and an HCDR3 in a heavy chain variable region as set forth in any one of SEQ ID NO: 72 to SEQ ID NO: 84.

6. The isolated antigen-binding protein of any one of claims 1-5, comprising an HCDR1, an HCDR2, and an HCDR3, wherein said HCDR3 comprises an amino acid sequence as set forth in any one of SEQ ID NO: 19, SEQ ID NO: 25, SEQ ID NO: 30, SEQ ID NO: 35, SEQ ID NO: 46, SEQ ID NO: 52, and SEQ ID NO: 56; said HCDR2 comprises an amino acid sequence as set forth in any one of SEQ ID NO: 18, SEQ ID NO: 24, SEQ ID NO: 34, SEQ ID NO: 45, SEQ ID NO: 51, and SEQ ID NO: 55; and said HCDR1 comprises an amino acid sequence as set forth in any one of SEQ ID NO: 17, SEQ ID NO: 23, SEQ ID NO: 29, SEQ ID NO: 37, SEQ ID NO: 39, SEQ ID NO: 44, and SEQ ID NO: 50.

7. The isolated antigen-binding protein of claim 6, wherein said HCDR1, HCDR2, and HCDR3 comprise any one group of amino acid sequences selected from:
a) HCDR1: SEQ ID NO: 17, HCDR2: SEQ ID NO: 18, and HCDR3: SEQ ID NO: 19;
b) HCDR1: SEQ ID NO: 23, HCDR2: SEQ ID NO: 24, and HCDR3: SEQ ID NO: 25;
c) HCDR1: SEQ ID NO: 29, HCDR2: SEQ ID NO: 24, and HCDR3: SEQ ID NO: 30;
d) HCDR1: SEQ ID NO: 23, HCDR2: SEQ ID NO: 34, and HCDR3: SEQ ID NO: 35;
e) HCDR1: SEQ ID NO: 37, HCDR2: SEQ ID NO: 24, and HCDR3: SEQ ID NO: 25;
f) HCDR1: SEQ ID NO: 39, HCDR2: SEQ ID NO: 24, and HCDR3: SEQ ID NO: 25;
g) HCDR1: SEQ ID NO: 23, HCDR2: SEQ ID NO: 24, and HCDR3: SEQ ID NO: 30;
h) HCDR1: SEQ ID NO: 44, HCDR2: SEQ ID NO: 45, and HCDR3: SEQ ID NO: 46;
i) HCDR1: SEQ ID NO: 50, HCDR2: SEQ ID NO: 51, and HCDR3: SEQ ID NO: 52;
j) HCDR1: SEQ ID NO: 23, HCDR2: SEQ ID NO: 55, and HCDR3: SEQ ID NO: 56;
and
k) HCDR1: SEQ ID NO: 37, HCDR2: SEQ ID NO: 24, and HCDR3: SEQ ID NO: 30.

8. The isolated antigen-binding protein of any one of claims 1-7, comprising an H-FR1, wherein the C-terminal of said H-FR1 is linked to the N-terminal of said HCDR1 directly or indirectly, and said H-FR1 comprises an amino acid sequence as set forth in any one of SEQ ID NO: 58 to SEQ ID NO: 65.

9. The isolated antigen-binding protein of any one of claims 1-8, comprising an H-FR2, wherein said H-FR2 is located between said HCDR1 and said HCDR2, and said H-FR2 comprises an amino acid sequence as set forth in SEQ ID NO: 66.

10. The isolated antigen-binding protein of any one of claims 1-9, comprising an H-FR3, wherein said H-FR3 is located between said HCDR2 and said HCDR3, and said H-FR3 comprises an amino acid sequence as set forth in any one of SEQ ID NO: 67 to SEQ ID NO: 69.

11. The isolated antigen-binding protein of any one of claims 1-10, comprising an H-FR4, wherein the N-terminal of said H-FR4 is linked to the C-terminal of said HCDR3 directly or indirectly, and said H-FR4 comprises an amino acid sequence as set forth in any one of SEQ ID NO: 70 to SEQ ID NO: 71.

12. The isolated antigen-binding protein of any one of claims 1-11, comprising an H-FR1, an H-FR2, an H-FR3, and an H-FR4, wherein said H-FR1 comprises an amino acid sequence as set forth in any one of SEQ ID NO: 58 to SEQ ID NO: 65; said H-FR2 comprises an amino acid sequence as set forth in SEQ ID NO: 66; said H-FR3 comprises an amino acid sequence as set forth in any one of SEQ ID NO: 67 to SEQ ID NO: 69; and said H-FR4 comprises an amino acid sequence as set forth in any one of SEQ ID NO: 70 to SEQ ID NO: 71.

13. The isolated antigen-binding protein of claim 12, wherein said H-FR1, H-FR2, H-FR3, and H-FR4 comprise any one group of amino acid sequences selected from:
a) H-FR1: SEQ ID NO: 58, H-FR2: SEQ ID NO: 66, H-FR3: SEQ ID NO: 67, and H-FR4: SEQ ID NO: 70;
b) H-FR1: SEQ ID NO: 59, H-FR2: SEQ ID NO: 66, H-FR3: SEQ ID NO: 67, and H-FR4: SEQ ID NO: 70;
c) H-FR1: SEQ ID NO: 60, H-FR2: SEQ ID NO: 66, H-FR3: SEQ ID NO: 67, and H-FR4: SEQ ID NO: 70;
d) H-FR1: SEQ ID NO: 61, H-FR2: SEQ ID NO: 66, H-FR3: SEQ ID NO: 67, and H-FR4: SEQ ID NO: 70;
e) H-FR1: SEQ ID NO: 62, H-FR2: SEQ ID NO: 66, H-FR3: SEQ ID NO: 67, and H-FR4: SEQ ID NO: 70;
f) H-FR1: SEQ ID NO: 63, H-FR2: SEQ ID NO: 66, H-FR3: SEQ ID NO: 67, and H-FR4: SEQ ID NO: 70;
g) H-FR1: SEQ ID NO: 64, H-FR2: SEQ ID NO: 66, H-FR3: SEQ ID NO: 67, and H-FR4: SEQ ID NO: 70;
h) H-FR1: SEQ ID NO: 64, H-FR2: SEQ ID NO: 66, H-FR3: SEQ ID NO: 68, and H-FR4: SEQ ID NO: 71; and
i) H-FR1: SEQ ID NO: 65, H-FR2: SEQ ID NO: 66, H-FR3: SEQ ID NO: 69, and H-FR4: SEQ ID NO: 70.

14. The isolated antigen-binding protein of any one of claims 1-13, comprising a heavy chain variable region VH, wherein said VH comprises an amino acid sequence as set forth in any one of SEQ ID NO: 72 to SEQ ID NO: 84.

15. The isolated antigen-binding protein of any one of claims 1-14, comprising an antibody or an antigen binding fragment thereof.

16. The isolated antigen-binding protein of claim 15, wherein said antigen binding fragment is selected from a group consisting of: Fab, Fab', F(ab)2, an Fv fragment, F(ab')2, scFv, di-scFv, VHH, and dAb.

17. The isolated antigen-binding protein of any one of claims 1-16, comprising a VHH or an antigen binding fragment thereof.

18. The isolated antigen-binding protein of any one of claims 15-17, wherein said antibody is selected from a group consisting of: a monoclonal antibody, a humanized antibody, a chimeric antibody, a bispecific antibody, a multispecific antibody, and a fully human antibody.

19. The isolated antigen-binding protein of any one of claims 1-18, comprising an amino acid sequence as set forth in any one of SEQ ID NO: 72 to SEQ ID NO: 84.

20. A chimeric antigen receptor, comprising a targeting moiety, wherein said targeting moiety comprises an HCDR3, said HCDR3 comprises an amino acid sequence as set forth in any one of SEQ ID NO: 19, SEQ ID NO: 25, SEQ ID NO: 30, SEQ ID NO: 35, SEQ ID NO: 46, SEQ ID NO: 52, and SEQ ID NO: 56.

21. The chimeric antigen receptor of claim 20, wherein said targeting moiety comprises an HCDR2, said HCDR2 comprises an amino acid sequence as set forth in any one of SEQ ID NO: 18, SEQ ID NO: 24, SEQ ID NO: 34, SEQ ID NO: 45, SEQ ID NO: 51, and SEQ ID NO: 55.

22. The chimeric antigen receptor of any one of claims 20-21, wherein said targeting moiety comprises an HCDR1, said HCDR1 comprises an amino acid sequence as set forth in any one of SEQ ID NO: 17, SEQ ID NO: 23, SEQ ID NO: 29, SEQ ID NO: 37, SEQ ID NO: 39, SEQ ID NO: 44, and SEQ ID NO: 50.

23. The chimeric antigen receptor of any one of claims 20-22, wherein said targeting moiety comprises an HCDR1, an HCDR2, and an HCDR3 in a heavy chain variable region as set forth in any one of SEQ ID NO: 72 to SEQ ID NO: 84.

24. The chimeric antigen receptor of any one of claims 20-23, wherein said targeting moiety comprises an HCDR1, an HCDR2, and an HCDR3, said HCDR3 comprises an amino acid sequence as set forth in any one of SEQ ID NO: 19, SEQ ID NO: 25, SEQ ID NO: 30, SEQ ID NO: 35, SEQ ID NO: 46, SEQ ID NO: 52, and SEQ ID NO: 56; said HCDR2 comprises an amino acid sequence as set forth in any one of SEQ ID NO: 18, SEQ ID NO: 24, SEQ ID NO: 34, SEQ ID NO: 45, SEQ ID NO: 51, and SEQ ID NO: 55; and said HCDR1 comprises an amino acid sequence as set forth in any one of SEQ ID NO: 17, SEQ ID NO: 23, SEQ ID NO: 29, SEQ ID NO: 37, SEQ ID NO: 39, SEQ ID NO: 44, and SEQ ID NO: 50.

25. The chimeric antigen receptor of claim 24, wherein said HCDR1, HCDR2, and HCDR3 comprise any one group of amino acid sequences selected from:
a) HCDR1: SEQ ID NO: 17, HCDR2: SEQ ID NO: 18, and HCDR3: SEQ ID NO: 19;
b) HCDR1: SEQ ID NO: 23, HCDR2: SEQ ID NO: 24, and HCDR3: SEQ ID NO: 25;
c) HCDR1: SEQ ID NO: 29, HCDR2: SEQ ID NO: 24, and HCDR3: SEQ ID NO: 30;
d) HCDR1: SEQ ID NO: 23, HCDR2: SEQ ID NO: 34, and HCDR3: SEQ ID NO: 35;
e) HCDR1: SEQ ID NO: 37, HCDR2: SEQ ID NO: 24, and HCDR3: SEQ ID NO: 25;
f) HCDR1: SEQ ID NO: 39, HCDR2: SEQ ID NO: 24, and HCDR3: SEQ ID NO: 25;
g) HCDR1: SEQ ID NO: 23, HCDR2: SEQ ID NO: 24, and HCDR3: SEQ ID NO: 30;
h) HCDR1: SEQ ID NO: 44, HCDR2: SEQ ID NO: 45, and HCDR3: SEQ ID NO: 46;
i) HCDR1: SEQ ID NO: 50, HCDR2: SEQ ID NO: 51, and HCDR3: SEQ ID NO: 52;
j) HCDR1: SEQ ID NO: 23, HCDR2: SEQ ID NO: 55, and HCDR3: SEQ ID NO: 56;
and
k) HCDR1: SEQ ID NO: 37, HCDR2: SEQ ID NO: 24, and HCDR3: SEQ ID NO: 30.

26. The chimeric antigen receptor of any one of claims 20-25, wherein said targeting moiety comprises an H-FR1, the C-terminal of said H-FR1 is linked to the N-terminal of said HCDR1 directly or indirectly, and said H-FR1 comprises an amino acid sequence as set forth in any one of SEQ ID NO: 58 to SEQ ID NO: 65.

27. The chimeric antigen receptor of any one of claims 20-26, wherein said targeting moiety comprises an H-FR2, said H-FR2 is located between said HCDR1 and said HCDR2, and said H-FR2 comprises an amino acid sequence as set forth in SEQ ID NO: 66.

28. The chimeric antigen receptor of any one of claims 20-27, wherein said targeting moiety comprises an H-FR3, said H-FR3 is located between said HCDR2 and said HCDR3, and said H-FR3 comprises an amino acid sequence as set forth in any one of SEQ ID NO: 67 to SEQ ID NO: 69.

29. The chimeric antigen receptor of any one of claims 20-28, wherein said targeting moiety comprises an H-FR4, the N-terminal of said H-FR4 is linked to the C-terminal of said HCDR3 directly or indirectly, and said H-FR4 comprises an amino acid sequence as set forth in any one of SEQ ID NO: 70 to SEQ ID NO: 71.

30. The chimeric antigen receptor of any one of claims 20-29, wherein said targeting moiety comprises an H-FR1, an H-FR2, an H-FR3, and an H-FR4, said H-FR1 comprises an amino acid sequence as set forth in any one of SEQ ID NO: 58 to SEQ ID NO: 65; said H-FR2 comprises an amino acid sequence as set forth in SEQ ID NO: 66; said H-FR3 comprises an amino acid sequence as set forth in any one of SEQ ID NO: 67 to SEQ ID NO: 69; and said H-FR4 comprises an amino acid sequence as set forth in any one of SEQ ID NO: 70 to SEQ ID NO: 71.

31. The chimeric antigen receptor of claim 30, wherein said H-FR1, H-FR2, H-FR3, and H-FR4 comprise any one group of amino acid sequences selected from:
a) H-FR1: SEQ ID NO: 58, H-FR2: SEQ ID NO: 66, H-FR3: SEQ ID NO: 67, and H-FR4: SEQ ID NO: 70;
b) H-FR1: SEQ ID NO: 59, H-FR2: SEQ ID NO: 66, H-FR3: SEQ ID NO: 67, and H-FR4: SEQ ID NO: 70;
c) H-FR1: SEQ ID NO: 60, H-FR2: SEQ ID NO: 66, H-FR3: SEQ ID NO: 67, and H-FR4: SEQ ID NO: 70;
d) H-FR1: SEQ ID NO: 61, H-FR2: SEQ ID NO: 66, H-FR3: SEQ ID NO: 67, and H-FR4: SEQ ID NO: 70;
e) H-FR1: SEQ ID NO: 62, H-FR2: SEQ ID NO: 66, H-FR3: SEQ ID NO: 67, and H-FR4: SEQ ID NO: 70;
f) H-FR1: SEQ ID NO: 63, H-FR2: SEQ ID NO: 66, H-FR3: SEQ ID NO: 67, and H-FR4: SEQ ID NO: 70;
g) H-FR1: SEQ ID NO: 64, H-FR2: SEQ ID NO: 66, H-FR3: SEQ ID NO: 67, and H-FR4: SEQ ID NO: 70;
h) H-FR1: SEQ ID NO: 64, H-FR2: SEQ ID NO: 66, H-FR3: SEQ ID NO: 68, and H-FR4: SEQ ID NO: 71; and
i) H-FR1: SEQ ID NO: 65, H-FR2: SEQ ID NO: 66, H-FR3: SEQ ID NO: 69, and H-FR4: SEQ ID NO: 70.

32. The chimeric antigen receptor of any one of claims 20-31, wherein said targeting moiety comprises an antibody or an antigen binding fragment.

33. The chimeric antigen receptor of claim 32, wherein said antigen binding fragment is selected from a group consisting of: Fab, Fab', F(ab)2, an Fv fragment, F(ab')2, scFv, di-scFv, VHH and/or dAb.

34. The chimeric antigen receptor of any one of claims 20-33, wherein said targeting moiety comprises a VHH.

35. The chimeric antigen receptor of claim 34, wherein said VHH targets a human AFP158-166/HLA-A02*01* complex.

36. The chimeric antigen receptor of any one of claims 20-35, wherein said targeting moiety comprises an amino acid sequence as set forth in any one of SEQ ID NO: 72 to SEQ ID NO: 84.

37. The chimeric antigen receptor of any one of claims 20-36, comprising a hinge region.

38. The chimeric antigen receptor of claim 37, wherein said hinge region comprises a hinge region derived from a protein selected from a group consisting of: IgG4, IgG1, and CD8.

39. The chimeric antigen receptor of any one of claims 37-38, wherein said hinge region comprises an amino acid sequence as set forth in SEQ ID NO: 147.

40. The chimeric antigen receptor of any one of claims 20-39, comprising a transmembrane domain.

41. The chimeric antigen receptor of claim 40, wherein said transmembrane domain comprises a transmembrane domain derived from a protein selected from a group consisting of: CD8, CD28, CD24, 4-1BB, CD4, CD27, CD7, PD-1, TRAC, TRBC, CD3ε, CD5, ICOS, OX40, NKG2D, 2B4, CD244, FcεRIγ, BTLA, CD30, GITR, HVEM, DAP10, CD2, NKG2C, LIGHT, DAP12, CD40L, TIM1, CD226, DR3, CD45, CD80, CD86, CD9, CD16, CD22, CD33, CD37, CD64, CD134, CD154, and SLAM, or a combination thereof.

42. The chimeric antigen receptor of any one of claims 40-41, wherein said transmembrane domain comprises a transmembrane domain derived from CD8.

43. The chimeric antigen receptor of any one of claims 40-42, wherein said transmembrane domain comprises an amino acid sequence as set forth in SEQ ID NO: 149.

44. The chimeric antigen receptor of any one of claims 40-43, wherein the N-terminal of said transmembrane domain is linked to the C-terminal of said hinge region.

45. The chimeric antigen receptor of any one of claims 20-44, comprising a costimulatory signaling domain.

46. The chimeric antigen receptor of claim 45, wherein said costimulatory signaling domain comprises a costimulatory signaling domain derived from a protein selected from a group consisting of: CD28, 4-1BB, CD27, CD2, CD7, CD8, OX40, CD226, DR3, SLAM, CDS, ICAM-1, NKG2D, NKG2C, B7-H3, 2B4, FcεRIγ, BTLA, GITR, HVEM, DAP10, DAP12, CD30, CD40, CD40L, TIM1, PD-1, LFA-1, LIGHT, JAML, CD244, CD100, ICOS, a ligand of CD83, CD40, and MyD88, or a combination thereof.

47. The chimeric antigen receptor of any one of claims 45-46, wherein said costimulatory signaling domain comprises a costimulatory signaling domain derived from 4-1BB.

48. The chimeric antigen receptor of any one of claims 45-47, wherein said costimulatory signaling domain comprises a costimulatory signaling domain derived from CD28.

49. The chimeric antigen receptor of any one of claims 45-48, wherein said costimulatory signaling domain comprises an amino acid sequence as set forth in SEQ ID NO: 132 or SEQ ID NO: 130.

50. The chimeric antigen receptor of any one of claims 45-49, wherein the N-terminal of said costimulatory signaling domain is linked to the C-terminal of said transmembrane domain.

51. The chimeric antigen receptor of any one of claims 20-50, comprising an intracellular signaling domain.

52. The chimeric antigen receptor of claim 51, wherein said intracellular signaling domain comprises an intracellular signaling domain derived from a protein selected from a group consisting of: CD3ζ, CD3δ, CD3γ, CD3ε, CD79a, CD79b, FceRIγ, FceRIβ, FcyRIIa, bovine leukemia virus gp30, Epstein-Barr virus (EBV) LMP2A, simian immunodeficiency virus PBj14 Nef, Kaposi's sarcoma-associated herpesvirus (HSKV), DAP10, and DAP-12, or a combination thereof.

53. The chimeric antigen receptor of any one of claims 51-52, wherein said intracellular signaling domain comprises an intracellular signaling domain derived from CD3ζ.

54. The chimeric antigen receptor of any one of claims 51-53, wherein said intracellular signaling domain comprises an amino acid sequence as set forth in SEQ ID NO: 134.

55. The chimeric antigen receptor of any one of claims 51-54, wherein the N-terminal of said intracellular signaling domain is linked to the C-terminal of said costimulatory signaling domain.

56. The chimeric antigen receptor of any one of claims 20-55, comprising an amino acid sequence as set forth in any one of SEQ ID NO: 112 to SEQ ID NO: 124.

57. A polypeptide, comprising the isolated antigen-binding protein of any one of claims 1-19 and/or the chimeric antigen receptor of any one of claims 20-56.

58. An immunoconjugate, comprising the isolated antigen-binding protein of any one of claims 1-19.

59. An isolated nucleic acid molecule, encoding the isolated antigen-binding protein of any one of claims 1-19, the chimeric antigen receptor of any one of claims 20-56, or the polypeptide of claim 57.

60. A vector, comprising the isolated nucleic acid molecule of claim 59.

61. A modified cell, comprising the isolated antigen-binding protein of any one of claims 1-19, the chimeric antigen receptor of any one of claims 20-56, the polypeptide of claim 57, the immunoconjugate of claim 58, the isolated nucleic acid molecule of claim 59 and/or the vector of claim 60.

62. The modified cell of claim 61, wherein said modification comprises upregulating the expression level of said low-density lipoprotein receptor-related protein or the fragment thereof in said cell.

63. The modified cell of claim 62, wherein said low-density lipoprotein receptor-related protein or the fragment thereof comprises one or more selected from a group consisting of: low-density lipoprotein receptor-related proteins 1-12, and functional fragments thereof.

64. The modified cell of any one of claims 62-63, wherein said low-density lipoprotein receptor-related protein or the fragment thereof is derived from human.

65. The modified cell of any one of claims 63-64, wherein said functional fragment comprises a fragment or a truncated body of said low-density lipoprotein receptor-related protein having the activity of said low-density lipoprotein receptor-related protein.

66. The modified cell of any one of claims 62-65, wherein said low-density lipoprotein receptor-related protein comprises a low-density lipoprotein receptor-related protein 6 and a truncated body thereof, and/or a low-density lipoprotein receptor-related protein 5 and a truncated body thereof.

67. The modified cell of any one of claims 62-66, wherein the truncated body of said low-density lipoprotein receptor-related protein 6 comprises an intracellular region of said low-density lipoprotein receptor-related protein 6; and/or, the truncated body of said low-density lipoprotein receptor-related protein 5 comprises an intracellular region of said low-density lipoprotein receptor-related protein 5.

68. The modified cell of any one of claims 62-67, wherein the truncated body of said low-density lipoprotein receptor-related protein 6 comprises a transmembrane region of said low-density lipoprotein receptor-related protein 6 and an LDLR region of said low-density lipoprotein receptor-related protein 6; and/or, the truncated body of said low-density lipoprotein receptor-related protein 5 comprises a transmembrane region of said low-density lipoprotein receptor-related protein 5 and an LDLR region of said low-density lipoprotein receptor-related protein 5.

69. The modified cell of any one of claims 62-68, wherein said low-density lipoprotein receptor-related protein or the fragment thereof comprises an amino acid sequence as set forth in any one of SEQ ID NO: 138, SEQ ID NO: 140, SEQ ID NO: 142, and SEQ ID NO: 144.

70. The modified cell of any one of claims 61-69, wherein said modification comprises introducing into said modified cell a vector that upregulates the expression level of said low-density lipoprotein receptor-related protein or the fragment thereof.

71. The modified cell of any one of claims 61-70, comprising an immune cell.

72. The modified cell of claim 71, wherein said immune cell is selected from a group consisting of: T cells, B cells, natural killer cells (NK cells), macrophages, NKT cells, monocytes, dendritic cells, granulocytes, lymphocytes, leukocytes and/or peripheral blood mononuclear cells.

73. The modified cell of any one of claims 61-72, wherein said cell comprises a T cell.

74. A method for preparing the isolated antigen-binding protein of any one of claims 1-19, the chimeric antigen receptor of any one of claims 20-56, and/or the polypeptide of claim 57, the method comprising culturing the modified cell of any one of claims 61-73
under a condition enabling the expression of said isolated antigen-binding protein and/or said polypeptide.

75. A pharmaceutical composition, comprising the isolated antigen-binding protein of any one of claims 1-19, the chimeric antigen receptor of any one of claims 20-56, the polypeptide of claim 57, the immunoconjugate of claim 58, the isolated nucleic acid molecule of claim 59, the vector of claim 60, the modified cell of any one of claims 61-73, and/or a pharmaceutically acceptable adjuvant and/or excipient.

76. A method for detecting AFP protein, comprising:
administering the isolated antigen-binding protein of any one of claims 1-19, the polypeptide of claim 57 or the immunoconjugate of claim 58.

77. A kit for detecting AFP protein, comprising the isolated antigen-binding protein of any one of claims 1-19, the polypeptide of claim 57 or the immunoconjugate of claim 58.

78. Use of the isolated antigen-binding protein of any one of claims 1-19, the polypeptide of claim 57 or the immunoconjugate of claim 58 in the preparation of a kit for detecting the presence and/or content of AFP protein.

79. Use of the isolated antigen-binding protein of any one of claims 1-19, the chimeric antigen receptor of any one of claims 20-56, the polypeptide of claim 57, the immunoconjugate of claim 58, the isolated nucleic acid molecule of claim 59, the vector of claim 60, and/or the modified cell of any one of claims 61-73 in the preparation of a drug for preventing and/or treating a tumor.

80. The use of claim 79, wherein said tumor comprises a solid tumor.

81. The use of any one of claims 79-80, wherein said tumor comprises a non-solid tumor.

82. The use of any one of claims 79-81, wherein said tumor comprises an AFP expression-associated tumor.

83. The use of any one of claims 79-82, wherein said tumor comprises liver cancer.

84. Use of the isolated antigen-binding protein of any one of claims 1-19, the chimeric antigen receptor of any one of claims 20-56, the polypeptide of claim 57, the immunoconjugate of claim 58, the isolated nucleic acid molecule of claim 59, the vector of claim 60, and/or the modified cell of any one of claims 61-73 for preventing and/or treating a tumor.

85. The use of claim 84, wherein said tumor comprises a solid tumor.

86. The use of any one of claims 84-85, wherein said tumor comprises a non-solid tumor.

87. The use of any one of claims 84-86, wherein said tumor comprises an AFP expression-associated tumor.

88. The use of any one of claims 84-87, wherein said tumor comprises liver cancer.

89. A method for preventing and/or treating a disease or disorder, comprising administering to a subject in need thereof an effective amount of the isolated antigen-binding protein of any one of claims 1-19, the chimeric antigen receptor of any one of claims 20-56, the polypeptide of claim 57, the immunoconjugate of claim 58, the isolated nucleic acid molecule of claim 59, the vector of claim 60, and/or the modified cell of any one of claims 61-73.

90. The method of claim 89, wherein said tumor comprises a solid tumor.

91. The method of any one of claims 89-90, wherein said tumor comprises a non-solid tumor.

92. The method of any one of claims 89-91, wherein said tumor comprises an AFP expression-associated tumor.

93. The method of any one of claims 89-92, wherein said tumor comprises liver cancer.
